# EUROPEAN PATENT SPECIFICATION

(11) **EP 2 260 862 B1**
(45) Date of publication and mention of the grant of the patent: **27.06.2012**
(21) Application number: 10008550.5
(22) Date of filing: 27.09.2006
(51) Int. Cl.: A61K 39/00

(54) **Compositions and methods for treating tumors presenting survivin antigens**
Zusammensetzungen und Verfahren zur Behandlung von Tumoren, die Survivin-Antigene aufweisen
Compositions et procédés pour traiter les tumeurs présentant des antigènes de survivine

(30) Priority: 27.09.2005 US 721199 P
(43) Date of publication of application: 15.12.2010
(62) Divisional of application: 06805883.3
(73) Proprietor: Merck Patent GmbH, 64293 Darmstadt (DE)
(72) Inventor: Gillies, Stephen D., Dr., Carlisle, MA 01741-1044 (US); Hettmann, Thore A.O., Dr., 81243 München (DE); Stein, Pascal André, Boston, MA 02120 (US); Klinz, Stephan G., Dr., Norwood, MA 02062 (US)

(56) References cited:
- WO-A-00/03693
- WO-A-02/02622
- WO-A-98/22589
- WO-A2-2004/067023
- SCHMITZ M ET AL: "Generation of survivin-specific CD8+ T effector cells by dendritic cells pulsed with protein or selected peptides" CANCER RESEARCH, AMERICAN ASSOCIATION FOR CANCER RESEARCH, BALTIMORE, MD, US, vol. 60, no. 17, 1 September 2000 (2000-09-01), pages 4845-4849, XP002283855 ISSN: 0008-5472
- GENTSCHEV IVAYLO ET AL: "Use of a recombinant Salmonella enterica serovar Typhimurium strain expressing C-Raf for protection against C-Raf induced lung adenoma in mice" BMC CANCER, BIOMED CENTRAL, LONDON, GB, vol. 5, no. 1, 9 February 2005 (2005-02-09), page 15, XP021004758 ISSN: 1471-2407
- JOHNSON A L ET AL: "Survivin as a cell cycle-related and antiapoptotic protein in granulosa cells" ENDOCRINOLOGY, vol. 143, no. 9, September 2002 (2002-09), pages 3405-3413, XP002429333 ISSN: 0013-7227

### SEQUENCE LISTING

<110> Merck Patent GmbH
<120> Compositions and Methods for Treating Tumors Presenting Survivin Antigens
<130> LEX-037
<150> EP 06 805 883.3
   <151> 2006-09-27
<160> 84
<170> PatentIn version 3.3
<210> 1
   <211> 9
   <212> PRT
   <213> Homo sapiens
<400> 1
   Leu Thr Leu Gly Glu Phe Leu Lys Leu
   1               5
<210> 2
   <211> 9
   <212> PRT
   <213> Homo sapiens
<400> 2
   Cys Pro Thr Glu Asn Glu Pro Asp Leu
   1               5
<210> 3
   <211> 9
   <212> PRT
   <213> Homo sapiens
<400> 3
   Glu Pro Asp Leu Ala Gln Cys Phe Phe
   1 5
<210> 4
   <211> 7
   <212> PRT
   <213> Homo sapiens
<400> 4
   Glu Pro Lys Ser Cys Asp Lys
   1 5
<210> 5
   <211> 7
   <212> PRT
   <213> Artificial sequence
<220>
   <223> fragment of IgGl hinge region with serine in place of cysteine
<400> 5
   Glu Pro Lys Ser Ser Asp Lys
   1 5
<210> 6
   <211> 9
   <212> PRT
   <213> Artificial sequence
<220>
   <223> modified fragment of mammalian survivin protein
<400> 6
   Leu Met Leu Gly Glu Phe Leu Lys Leu
   1 5
<210> 7
   <211> 9
   <212> PRT
   <213> Gallus gallus
<400> 7
   Ser Thr Arg Ala Ala Thr Phe Arg Asn
   1 5
<210> 8
   <211> 142
   <212> PRT
   <213> Homo sapiens
<400> 8
   Met Gly Ala Pro Thr Leu Pro Pro Ala Trp Gln Pro Phe Leu Lys Asp
   1               5                   10                  15
   His Arg Ile Ser Thr Phe Lys Asn Trp Pro Phe Leu Glu Gly Cys Ala
               20                  25                  30
   Cys Thr Pro Glu Arg Met Ala Glu Ala Gly Phe Ile His Cys Pro Thr
           35                  40                  45
   Glu Asn Glu Pro Asp Leu Ala Gln Cys Phe Phe Cys Phe Lys Glu Leu
       50                  55                  60
   Glu Gly Trp Glu Pro Asp Asp Asp Pro Ile Glu Glu His Lys Lys His
   65                  70                  75                  80
   Ser Ser Gly Cys Ala Phe Leu Ser Val Lys Lys Gln Phe Glu Glu Leu
                   85                  90                  95
   Thr Leu Gly Glu Phe Leu Lys Leu Asp Arg Glu Arg Ala Lys Asn Lys
               100                 105                 110
   Ile Ala Lys Glu Thr Asn Asn Lys Lys Lys Glu Phe Glu Glu Thr Ala
           115                 120                 125
   Lys Lys Val Arg Arg Ala Ile Glu Gln Leu Ala Ala Met Asp
       130                 135                 140
<210> 9
   <211> 142
   <212> PRT
   <213> Canis familiaris
<400> 9
<210> 10
   <211> 140
   <212> PRT
   <213> Mus musculus
<400> 10
   Met Gly Ala Pro Ala Leu Pro Gln Ile Trp Gln Leu Tyr Leu Lys Asn
   1 5 10 15
   Tyr Arg Ile Ala Thr Phe Lys Asn Trp Pro Phe Leu Glu Asp Cys Ala
   20 25 30
   Cys Thr Pro Glu Arg Met Ala Glu Ala Gly Phe Ile His Cys Pro Thr
   35 40 45
   Glu Asn Glu Pro Asp Leu Ala Gln Cys Phe Phe Cys Phe Lys Glu Leu
   50 55 60
   Glu Gly Trp Glu Pro Asp Asp Asn Pro Ile Glu Glu His Arg Lys His
   65 70 75 80
   Ser Pro Gly Cys Ala Phe Leu Thr Val Lys Lys Gln Met Glu Glu Leu
   85 90 95
   Thr Val Ser Glu Phe Leu Lys Leu Asp Arg Gln Arg Ala Lys Asn Lys
   100 105 110
   Ile Ala Lys Glu Thr Asn Asn Lys Gln Lys Glu Phe Glu Glu Thr Ala
   115 120 125
   Lys Thr Thr Arg Gln Ser Ile Glu Gln Leu Ala Ala
   130 135 140
<210> 11
   <211> 142
   <212> PRT
   <213> Gallus gallus
<400> 11
   Met Ala Ala Tyr Ala Glu Met Leu Pro Lys Glu Trp Leu Val Tyr Leu
   1 5 10 15
   Val Ser Thr Arg Ala Ala Thr Phe Arg Asn Trp Pro Phe Thr Glu Gly
   20 25 30
   Cys Ala Cys Thr Pro Glu Arg Met Ala Ala Ala Gly Phe Val His Cys
   35 40 45
   Pro Ser Glu Asn Ser Pro Asp Val Val Gln Cys Phe Phe Cys Leu Lys
   50 55 60
   Glu Leu Glu Gly Trp Glu Pro Asp Asp Asp Pro Leu Glu Glu His Lys
   65 70 75 80
   Lys His Ser Ala Gly Cys Ala Phe Ala Ala Leu Gln Lys Asp Pro Ser
   85 90 95
   Asn Leu Thr Val Gln Glu Phe Leu Lys Leu Asp Lys Lys Arg Thr Lys
   100 105 110
   Asn Val Ile Lys Lys Ala Ile Ser Gln Lys Glu Thr Asp Ile Glu Asp
   115 120 125
   Val Ala Lys Gly Val Arg His Ala Ile Glu Asn Met Gly Pro
   130 135 140
<210> 12
   <211> 142
   <212> PRT
   <213> Artificial sequence
<220>
   <223> chickensurvivin(N97E, T99M, V100L, Q101G)
<400> 12
   Met Ala Ala Tyr Ala Glu Met Leu Pro Lys Glu Trp Leu Val Tyr Leu
   1 5 10 15
   Val Ser Thr Arg Ala Ala Thr Phe Arg Asn Trp Pro Phe Thr Glu Gly
   20 25 30
   Cys Ala Cys Thr Pro Glu Arg Met Ala Ala Ala Gly Phe Val His Cys
   35 40 45
   Pro Ser Glu Asn Ser Pro Asp Val Val Gln Cys Phe Phe Cys Leu Lys
   50 55 60
   Glu Leu Glu Gly Trp Glu Pro Asp Asp Asp Pro Leu Glu Glu His Lys
   65 70 75 80
   Lys His Ser Ala Gly Cys Ala Phe Ala Ala Leu Gln Lys Asp Pro Ser
   85 90 95
   Glu Leu Met Leu Gly Glu Phe Leu Lys Leu Asp Lys Lys Arg Thr Lys
   100 105 110
   Asn Val Ile Lys Lys Ala Ile Ser Gln Lys Glu Thr Asp Ile Glu Asp
   115 120 125
   Val Ala Lys Gly Val Arg His Ala Ile Glu Asn Met Gly Pro
   130 135 140
<210> 13
   <211> 157
   <212> PRT
   <213> Xenopus laevis
<400> 13
   Met Leu Ser Ile Ser Pro Ile Val Ser Leu Arg Arg Cys Asp Asn Glu
   1               5                   10                  15
   Pro Ser Met Pro Asp Glu Trp Arg Leu Tyr Asn Leu Ala Thr Arg Leu
               20                  25                  30
   Arg Thr Phe Ser Asn Trp Pro Phe Thr Glu Asp Cys Ala Cys Thr Pro
           35                  40                  45
   Glu Arg Met Ala Glu Ala Gly Phe Val His Cys Pro Thr Asp Asn Ser
       50                  55                  60
   Pro Asp Val Val Lys Cys Phe Phe Cys Leu Lys Glu Leu Glu Gly Trp
   65                  70                  75                  80
   Gln Pro Glu Asp Asp Pro Met Asp Glu His Lys Lys His Ser Pro Ser
                   85                  90                  95
   Cys Leu Phe Ile Ala Leu Lys Lys Lys Ala Glu Glu Leu Thr Leu Ser
               100                 105                 110
   Glu Phe Leu Lys Leu Asp Leu Glu His Thr Lys Ile Lys Met Gln Lys
           115                 120                 125
   Gln Met Asn Leu His Ile Glu Arg Phe Gln Ala Lys Ala Asn Glu Val
       130                 135                 140
   Arg Gly His Leu Glu Lys Leu Asp Ala Asp Glu Thr Gln
   145                 150                 155
<210> 14
   <211> 9
   <212> PRT
   <213> Homo sapiens
<400> 14
   Val Val His Phe Phe Lys Asn Ile Val
   1               5
<210> 15
   <211> 9
   <212> PRT
   <213> Homo sapiens
<400> 15
   Glu Pro Asp Leu Ala Gln Cys Phe Tyr
   1               5
<210> 16
   <211> 9
   <212> PRT
   <213> Homo sapiens
<400> 16
   Cys Pro Thr Glu Asn Glu Pro Asp Tyr
   1               5
<210> 17
   <211> 19
   <212> DNA
   <213> Artificial sequence
<220>
   <223> synthetic: outside sense primer
<400> 17
   gaaaaatggc ggcctatgc 19
<210> 18
   <211> 21
   <212> DNA
   <213> Artificial sequence
<220>
   <223> synthetic: outside antisense primer
<400> 18
   caccgtagac ccagaggaac c 21
<210> 19
   <211> 23
   <212> DNA
   <213> Artificial sequence
<220>
   <223> synthetic: inside sense primer incorporating an Xba I restriction site
<400> 19
   ctctagaatg gcggcctatg ctg 23
<210> 20
   <211> 27
   <212> DNA
   <213> Artificial sequence
<220>
   <223> synthetic: inside antisense primer incorporating an Xho I restriction site
<400> 20
   cctcgagacc taagggccca tgttctc 27
<210> 21
   <211> 429
   <212> DNA
   <213> Gallus gallus
<400> 21
   atggcggcct atgctgaaat gctgcccaag gaatggctgg tctacctcgt ctccacccgc 60
   gccgccacct tccgcaactg gcccttcacc gagggctgcg cctgcacgcc cgagcggatg 120
   gcggcggcgg gcttcgtgca ctgccccagc gagaacagcc ccgacgtggt gcagtgcttc 180
   ttctgcctca aggagctgga gggctgggag cccgacgacg acccgctgga ggaacacaaa 240
   aagcactccg cgggctgcgc ttttgccgct cttcagaaag atccctctaa cctgacggtg 300
   caggaattct tgaagctgga taaaaagcgg accaaaaacg taattaaaaa agcaatttct 360
   cagaaggaaa ctgatatcga agatgtagcc aagggcgtgc ggcacgcgat agagaacatg 420
   ggcccttag 429
<210> 22
   <211> 36
   <212> DNA
   <213> Artificial Sequence
<220>
   <223> synthetic: duplex adapter molecule sense oligo
<400> 22
   gggtgcagcg gcctatgctg aaatgctgcc caagga 36
<210> 23
   <211> 33
   <212> DNA
   <213> Artificial Sequence
<220>
   <223> synthetic: duplex adapter molecule antisense oligo
<400> 23
   ttgggcagca tttcagcata ggccgctgca ccc 33
<210> 24
   <211> 1337
   <212> DNA
   <213> Artificial sequence
<220>
   <223> sequence encoding huFc-chickenSurvivin fusion protein
<400> 24
   gagcccaaat cttctgacaa aactcacaca tgcccaccgt gcccaggtaa gccagcccag 60
   gcctcgccct ccagctcaag gcgggacagg tgccctagag tagcctgcat ccagggacag 120
   gccccagccg ggtgctgaca cgtccacctc catctcttcc tcagcacctg aactcctggg 180
   gggaccgtca gtcttcctct tccccccaaa acccaaggac accctcatga tctcccggac 240
   ccctgaggtc acatgcgtgg tggtggacgt gagccacgaa gaccctgagg tcaagttcaa 300
   ctggtacgtg gacggcgtgg aggtgcataa tgccaagaca aagccgcggg aggagcagta 360
   caacagcacg taccgtgtgg tcagcgtcct caccgtcctg caccaggact ggctgaatgg 420
   caaggagtac aagtgcaagg tctccaacaa agccctccca gcccccatcg agaaaaccat 480
   ctccaaagcc aaaggtggga cccgtggggt gcgagggcca catggacaga ggccggctcg 540
   gcccaccctc tgccctgaga gtgaccgctg taccaacctc tgtccctaca gggcagcccc 600
   gagaaccaca ggtgtacacc ctgcccccat cacgggagga gatgaccaag aaccaggtca 660
   gcctgacctg cctggtcaaa ggcttctatc ccagcgacat cgccgtggag tgggagagca 720
   atgggcagcc ggagaacaac tacaagacca cgcctcccgt gctggactcc gacggctcct 780
   tcttcctcta tagcaagctc accgtggaca agagcaggtg gcagcagggg aacgtcttct 840
   catgctccgt gatgcatgag gctctgcaca accactacac gcagaagagc ctctccctgt 900
   ccccgggtgc agcggcctat gctgaaatgc tgcccaagga atggctggtc tacctcgtct 960
   ccacccgcgc cgccaccttc cgcaactggc ccttcaccga gggctgcgcc tgcacgcccg 1020
   agcggatggc ggcggcgggc ttcgtgcact gccccagcga gaacagcccc gacgtggtgc 1080
   agtgcttctt ctgcctcaag gagctggagg gctgggagcc cgacgacgac ccgctggagg 1140
   aacacaaaaa gcactccgcg ggctgcgctt ttgccgctct tcagaaagat ccctctaacc 1200
   tgacggtgca ggaattcttg aagctggata aaaagcggac caaaaacgta attaaaaaag 1260
   caatttctca gaaggaaact gatatcgaag atgtagccaa gggcgtgcgg cacgcgatag 1320
   agaacatggg cccttag 1337
<210> 25
   <211> 373
   <212> PRT
   <213> Artificial sequence
<220>
   <223> huFc-chickenSurvivin fusion protein
<400> 25
   Glu Pro Lys Ser Ser Asp Lys Thr His Thr Cys Pro Pro Cys Pro Ala
   1               5                   10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Glu Glu Met Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Ala Ala Ala Tyr Ala Glu Met Leu Pro
   225                 230                 235                 240
   Lys Glu Trp Leu Val Tyr Leu Val Ser Thr Arg Ala Ala Thr Phe Arg
                   245                 250                 255
   Asn Trp Pro Phe Thr Glu Gly Cys Ala Cys Thr Pro Glu Arg Met Ala
               260                 265                 270
   Ala Ala Gly Phe Val His Cys Pro Ser Glu Asn Ser Pro Asp Val Val
           275                 280                 285
   Gln Cys Phe Phe Cys Leu Lys Glu Leu Glu Gly Trp Glu Pro Asp Asp
       290                 295                 300
   Asp Pro Leu Glu Glu His Lys Lys His Ser Ala Gly Cys Ala Phe Ala
   305                 310                 315                 320
   Ala Leu Gln Lys Asp Pro Ser Asn Leu Thr Val Gln Glu Phe Leu Lys
                   325                 330                 335
   Leu Asp Lys Lys Arg Thr Lys Asn Val Ile Lys Lys Ala Ile Ser Gln
               340                 345                 350
   Lys Glu Thr Asp Ile Glu Asp Val Ala Lys Gly Val Arg His Ala Ile
           355                 360                 365
   Glu Asn Met Gly Pro
       370
<210> 26
   <211> 1125
   <212> DNA
   <213> Artificial Sequence
<220>
   <223> sequence encoding muFc-Chickensurvivin fusion protein
<400> 26
   gagcccagag ggcccacaat caagccctgt cctccatgca aatgcccagc acctaacctc 60
   ttgggtggac catccgtctt catcttccct ccaaagatca aggatgtact catgatctcc 120
   ctgagcccca tagtcacatg tgtggtggtg gatgtgagcg aggatgaccc agatgtccag 180
   atcagctggt ttgtgaacaa cgtggaagta cacacagctc agacacaaac ccatagagag 240
   gattacaaca gtactctccg ggtggtcagt gccctcccca tccagcacca ggactggatg 300
   agtggcaagg agttcaaatg caaggtcaac aacaaagacc tcccagcgcc catcgagaga 360
   accatctcaa aacccaaagg gtcagtaaga gctccacagg tatatgtctt gcctccacca 420
   gaagaagaga tgactaagaa acaggtcact ctgacctgca tggtcacaga cttcatgcct 480
   gaagacattt acgtggagtg gaccaacaac gggaaaacag agctaaacta caagaacact 540
   gaaccagtcc tggactctga tggttcttac ttcatgtaca gcaagctgag agtggaaaag 600
   aagaactggg tggaaagaaa tagctactcc tgttcagtgg tccacgaggg tctgcacaat 660
   caccacacga ctaagagctt ctcccggacc ccgggtgcag cggcctatgc tgaaatgctg 720
   cccaaggaat ggctggtcta cctcgtctcc acccgcgccg ccaccttccg caactggccc 780
   ttcaccgagg gctgcgcctg cacgcccgag cggatggcgg cggcgggctt cgtgcactgc 840
   cccagcgaga acagccccga cgtggtgcag tgcttcttct gcctcaagga gctggagggc 900
   tgggagcccg acgacgaccc gctggaggaa cacaaaaagc actccgcggg ctgcgctttt 960
   gccgctcttc agaaagatcc ctctaacctg acggtgcagg aattcttgaa gctggataaa 1020
   aagcggacca aaaacgtaat taaaaaagca atttctcaga aggaaactga tatcgaagat 1080
   gtagccaagg gcgtgcggca cgcgatagag aacatgggcc cttag 1125
<210> 27
   <211> 374
   <212> PRT
   <213> Artificial Sequence
<220>
   <223> muFc-Chickensurvivin fusion protein
<400> 27
   Glu Pro Arg Gly Pro Thr Ile Lys Pro Cys Pro Pro Cys Lys Cys Pro
   1               5                   10                  15
   Ala Pro Asn Leu Leu Gly Gly Pro Ser Val Phe Ile Phe Pro Pro Lys
               20                  25                  30
   Ile Lys Asp Val Leu Met Ile Ser Leu Ser Pro Ile Val Thr Cys Val
           35                  40                  45
   Val Val Asp Val Ser Glu Asp Asp Pro Asp Val Gln Ile Ser Trp Phe
       50                  55                  60
   Val Asn Asn Val Glu Val His Thr Ala Gln Thr Gln Thr His Arg Glu
   65                  70                  75                  80
   Asp Tyr Asn Ser Thr Leu Arg Val Val Ser Ala Leu Pro Ile Gln His
                   85                  90                  95
   Gln Asp Trp Met Ser Gly Lys Glu Phe Lys Cys Lys Val Asn Asn Lys
               100                 105                 110
   Asp Leu Pro Ala Pro Ile Glu Arg Thr Ile Ser Lys Pro Lys Gly Ser
           115                 120                 125
   Val Arg Ala Pro Gln Val Tyr Val Leu Pro Pro Pro Glu Glu Glu Met
   130 135 140
   Thr Lys Lys Gln Val Thr Leu Thr Cys Met Val Thr Asp Phe Met Pro
   145 150 155 160
   Glu Asp Ile Tyr Val Glu Trp Thr Asn Asn Gly Lys Thr Glu Leu Asn
   165 170 175
   Tyr Lys Asn Thr Glu Pro Val Leu Asp Ser Asp Gly Ser Tyr Phe Met
   180 185 190
   Tyr Ser Lys Leu Arg Val Glu Lys Lys Asn Trp Val Glu Arg Asn Ser
   195 200 205
   Tyr Ser Cys Ser Val Val His Glu Gly Leu His Asn His His Thr Thr
   210 215 220
   Lys Ser Phe Ser Arg Thr Pro Gly Ala Ala Ala Tyr Ala Glu Met Leu
   225 230 235 240
   Pro Lys Glu Trp Leu Val Tyr Leu Val Ser Thr Arg Ala Ala Thr Phe
   245 250 255
   Arg Asn Trp Pro Phe Thr Glu Gly Cys Ala Cys Thr Pro Glu Arg Met
   260 265 270
   Ala Ala Ala Gly Phe Val His Cys Pro Ser Glu Asn Ser Pro Asp Val
   275 280 285
   Val Gln Cys Phe Phe Cys Leu Lys Glu Leu Glu Gly Trp Glu Pro Asp
   290 295 300
   Asp Asp Pro Leu Glu Glu His Lys Lys His Ser Ala Gly Cys Ala Phe
   305 310 315 320
   Ala Ala Leu Gln Lys Asp Pro Ser Asn Leu Thr Val Gln Glu Phe Leu
   325 330 335
   Lys Leu Asp Lys Lys Arg Thr Lys Asn Val Ile Lys Lys Ala Ile Ser
   340 345 350
   Gln Lys Glu Thr Asp Ile Glu Asp Val Ala Lys Gly Val Arg His Ala
   355 360 365
   Ile Glu Asn Met Gly Pro
   370
<210> 28
   <211> 38
   <212> DNA
   <213> Artificial Sequence
<220>
   <223> synthetic: mutagenic sense primer Mut1s
<400> 28
   cctctgaact gatgttgggg gagttcttga agctggat 38
<210> 29
   <211> 35
   <212> DNA
   <213> Artificial Sequence
<220>
   <223> synthetic: mutagenic antisense primer Mut1a
<400> 29
   aactccccca acatcagttc agagggatct ttctg 35
<210> 30
   <211> 41
   <212> DNA
   <213> Artificial Sequence
<220>
   <223> synthetic: flanking primer Pr1s
<400> 30
   ccgcggccgc ccccttcacc atggcggcct atgctgaaat g 41
<210> 31
   <211> 34
   <212> DNA
   <213> Artificial sequence
<220>
   <223> synthetic: flanking primer Pr1a
<400> 31
   agatctggat ccctaagggc ccatgttctc tatc 34
<210> 32
   <211> 30
   <212> PRT
   <213> Artificial Sequence
<220>
   <223> chicken Survivin derived peptide
<400> 32
   Gly Cys Ala Phe Ala Ala Leu Gln Lys Asp Pro Ser Glu Leu Met Leu
   1               5                   10                  15
   Gly Glu Phe Leu Lys Leu Asp Arg Glu Arg Ala Lys Asn Val
               20                  25                  30
<210> 33
   <211> 10
   <212> PRT
   <213> Artificial sequence
<220>
   <223> modified Human survivin protein fragment
<400> 33
   Glu Pro Asp Asp Asp Pro Ile Glu Glu Ala
   1 5 10
<210> 34
   <211> 10
   <212> PRT
   <213> Homo sapiens
<400> 34
   Glu Pro Asp Asp Asp Pro Ile Glu Glu His
   1 5 10
<210> 35
   <211> 9
   <212> PRT
   <213> Artificial Sequence
<220>
   <223> modified Human Survivin protein fragment
<400> 35
   Cys Leu Thr Glu Asn Glu Pro Asp Leu
   1 5
<210> 36
   <211> 10
   <212> PRT
   <213> Artificial Sequence
<220>
   <223> modified Human Survivin protein fragment
<400> 36
   Ala Leu Cys Phe Phe Cys Phe Lys Glu Leu
   1 5 10
<210> 37
   <211> 10
   <212> PRT
   <213> Homo sapiens
<400> 37
   Ala Gln Cys Phe Phe Cys Phe Lys Glu Leu
   1 5 10
<210> 38
   <211> 10
   <212> PRT
   <213> Homo sapiens
<400> 38
   Glu Leu Thr Leu Gly Glu Phe Leu Lys Leu
   1 5 10
<210> 39
   <211> 142
   <212> PRT
   <213> Sus scrofa
<400> 39
   Met Ser Ala Pro Ser Leu Pro Pro Ala Trp Gln Leu Tyr Leu Lys Asp
   1 5 10 15
   His Arg Ile Ser Thr Phe Lys Asn Trp Pro Phe Leu Glu Gly Cys Ala
   20 25 30
   Cys Thr Pro Glu Arg Met Ala Ala Ala Gly Phe Ile His Cys Pro Thr
   35 40 45
   Glu Asn Glu Pro Asp Leu Ala Gln Cys Phe Phe Cys Phe Lys Glu Leu
   50 55 60
   Glu Gly Trp Glu Pro Asp Asp Asp Pro Ile Glu Glu His Lys Lys His
   65 70 75 80
   Ser Ser Gly Cys Ala Phe Leu Ser Val Lys Lys Gln Phe Glu Glu Leu
   85 90 95
   Thr Leu Ser Glu Phe Leu Lys Leu Asp Lys Glu Arg Ala Lys Asn Lys
   100 105 110
   Ile Ala Lys Glu Thr Asn Asn Lys Gln Lys Glu Phe Glu Glu Thr Ala
   115 120 125
   Lys Lys Val Arg Cys Ala Ile Glu Gln Leu Ala Ala Ser Glu
   130 135 140
<210> 40
   <211> 142
   <212> PRT
   <213> Bos taurus
<400> 40
   Met Ser Ala Pro Ser Leu Pro Pro Ala Trp Gln Leu Tyr Leu Lys Asp
   1 5 10 15
   His Arg Ile Ser Thr Phe Lys Asn Trp Pro Phe Leu Glu Gly Cys Ala
   20 25 30
   Cys Thr Pro Glu Arg Met Ala Glu Ala Gly Phe Ile His Cys Pro Thr
   35 40 45
   Glu Asn Glu Pro Asp Leu Ala Gln Cys Phe Leu Cys Phe Lys Glu Leu
   50 55 60
   Glu Gly Trp Glu Pro Asp Asp Asp Pro Ile Glu Glu His Lys Lys His
   65 70 75 80
   Ser Ser Gly Cys Ala Phe Leu Ser Val Lys Lys Gln Phe Glu Glu Leu
                   85                  90                  95
   Thr Leu Ser Glu Phe Leu Lys Leu Asp Lys Glu Arg Ala Lys Asn Lys
               100                 105                 110
   Ile Ala Lys Glu Thr Asn Asn Lys Gln Lys Glu Phe Glu Glu Thr Ala
           115                 120                 125
   Lys Lys Val Arg Cys Ala Ile Glu Gln Leu Val Ala Met Asp
       130                 135                 140
<210> 41
   <211> 142
   <212> PRT
   <213> Felis catus
<400> 41
   Met Gly Ala Ser Ser Leu Pro Pro Ala Trp Gln Leu Tyr Leu Lys Asp
   1               5                   10                  15
   His Arg Ile Ser Thr Phe Lys Asn Trp Pro Phe Leu Glu Gly Cys Ala
               20                  25                  30
   Cys Thr Pro Glu Arg Met Ala Glu Ala Gly Phe Ile His Cys Pro Thr
           35                  40                  45
   Glu Asn Glu Pro Asp Leu Ala Gln Cys Phe Phe Cys Phe Lys Glu Leu
       50                  55                  60
   Glu Gly Trp Glu Pro Asp Asp Asp Pro Ile Glu Glu His Lys Lys His
   65                  70                  75                  80
   Ser Ser Gly Cys Ala Phe Leu Ser Val Lys Lys Gln Phe Glu Glu Leu
                   85                  90                  95
   Thr Leu Ser Glu Phe Leu Lys Leu Asp Lys Glu Arg Ala Lys Asn Lys
               100                 105                 110
   Ile Ala Lys Glu Thr Asn His Lys Gln Lys Glu Phe Glu Glu Thr Ala
           115                 120                 125
   Lys Arg Val Arg Cys Ala Ile Glu Gln Leu Ala Ala Leu Glu
       130                 135                 140
<210> 42
   <211> 142
   <212> PRT
   <213> Rattus norvegicus
<400> 42
   Met Gly Ala Thr Ala Leu Pro Pro Ile Trp Gln Met Tyr Leu Lys Asp
   1 5 10 15
   His Arg Ile Tyr Thr Phe Lys Asn Trp Pro Phe Leu Glu Asp Cys Ser
   20 25 30
   Cys Thr Pro Glu Arg Met Ala Glu Ala Gly Phe Ile His Cys Pro Thr
   35 40 45
   Glu Asn Glu Pro Asp Leu Ala Gln Cys Phe Phe Cys Phe Lys Glu Leu
   50 55 60
   Glu Gly Trp Glu Pro Asp Asp Asn Pro Ile Glu Glu His Arg Lys His
   65 70 75 80
   Ser Pro Gly Cys Ala Phe Leu Thr Val Lys Lys Gln Val Glu Glu Leu
   85 90 95
   Thr Val Ser Glu Phe Leu Lys Leu Asp Lys Gln Arg Ala Lys Asn Lys
   100 105 110
   Ile Ala Lys Glu Thr Asn Asn Lys Gln Lys Glu Phe Glu Glu Thr Arg
   115 120 125
   Arg Thr Val Arg Gln Ser Ile Glu Gln Leu Ala Ala Leu Arg
   130 135 140
<210> 43
   <211> 142
   <212> PRT
   <213> Pongo pygmaeus
<400> 43
   Met Gly Ala Pro Thr Leu Pro Pro Ala Trp Gln Pro Phe Leu Lys Asp
   1 5 10 15
   His Arg Ile Ser Thr Phe Lys Asn Trp Pro Phe Leu Glu Gly Cys Ala
   20 25 30
   Cys Thr Pro Glu Arg Met Ala Glu Ala Gly Phe Ile His Cys Pro Thr
   35 40 45
   Glu Asn Glu Pro Asp Leu Ala Gln Cys Phe Phe Cys Phe Lys Glu Leu
   50 55 60
   Glu Gly Trp Glu Pro Asp Asp Asp Pro Ile Glu Glu His Lys Lys His
   65 70 75 80
   Ser Ser Gly Cys Ala Phe Leu Ser Val Lys Lys Gln Phe Glu Glu Leu
   85 90 95
   Thr Leu Gly Glu Phe Leu Lys Leu Asp Arg Glu Arg Ala Lys Asn Lys
   100 105 110
   Ile Ala Lys Glu Thr Asn Asn Lys Lys Lys Glu Phe Glu Glu Thr Ala
   115 120 125
   Lys Lys Val Arg Arg Ala Ile Glu Gln Leu Ala Ala Met Asp
   130 135 140
<210> 44
   <211> 135
   <212> PRT
   <213> Gallus gallus
<400> 44
   Met Ala Ala Tyr Ala Glu Met Leu Pro Lys Glu Trp Leu Val Tyr Leu
   1               5                   10                  15
   Val Ser Thr Arg Ala Ala Thr Phe Arg Asn Trp Pro Phe Thr Glu Gly
               20                  25                  30
   Cys Ala Cys Thr Pro Glu Arg Met Ala Ala Ala Gly Phe Val His Cys
           35                  40                  45
   Pro Ser Glu Asn Ser Pro Asp Val Val Gln Cys Phe Phe Cys Leu Lys
       50                  55                  60
   Glu Leu Glu Gly Trp Glu Pro Asp Asp Asp Pro Leu Glu Glu His Lys
   65                  70                  75                  80
   Lys His Ser Ala Gly Cys Ala Phe Ala Ala Leu Gln Lys Asp Pro Ser
                   85                  90                  95
   Asn Leu Thr Val Gln Glu Phe Leu Lys Leu Asp Lys Lys Arg Thr Lys
               100                 105                 110
   Asn Val Ile Val Arg Thr Pro Arg Gln Arg Gly Gly Ala Pro Cys Pro
           115                 120                 125
   Ala Leu Leu Pro Ala Leu Ala
       130                 135
<210> 45
   <211> 59
   <212> PRT
   <213> Gallus gallus
<400> 45
   Met Ala Ala Tyr Ala Glu Met Leu Pro Lys Glu Trp Leu Val Tyr Leu
   1               5                   10                  15
   Val Ser Thr Arg Ala Ala Thr Phe Arg Asn Trp Pro Phe Thr Glu Gly
               20                  25                  30
   Cys Ala Cys Thr Pro Glu Arg Gly Gly Thr Gln Lys Ala Leu Arg Gly
           35                  40                  45
   Leu Arg Phe Cys Arg Ser Ser Glu Arg Ser Leu
       50                  55
<210> 46
   <211> 160
   <212> PRT
   <213> Xenopus laevis
<400> 46
   Met Tyr Ser Ala Lys Asn Arg Phe Val Gln Ala Val Gln Arg Leu Gln
   1 5 10 15
   Asp Phe Lys Asn Met Tyr Asp Tyr Asp Ala Arg Leu Ala Thr Phe Ala
   20 25 30
   Asp Trp Pro Phe Thr Glu Asn Cys Lys Cys Thr Pro Glu Ser Met Ala
   35 40 45
   Lys Ala Gly Phe Val His Cys Pro Thr Glu Asn Glu Pro Asp Val Ala
   50 55 60
   Cys Cys Phe Phe Cys Leu Lys Glu Leu Glu Gly Trp Glu Pro Asp Asp
   65 70 75 80
   Asp Pro Trp Thr Glu His Ser Lys Arg Ser Ala Asn Cys Gly Phe Leu
   85 90 95
   Ser Leu Thr Lys Cys Val Asn Asp Leu Thr Met Glu Gly Phe Leu Arg
   100 105 110
   Leu Glu Gly Asp Arg Ile Lys Ser Phe Tyr Arg Lys Phe Ser Thr Val
   115 120 125
   Val Leu Gln Tyr Val Glu Glu Glu Met Thr Ala Ala Thr Lys Arg Leu
   130 135 140
   Leu Glu Tyr Phe Ser Asn Gln His Gln Cys Ser Ile Asp Leu Asp His
   145 150 155 160
<210> 47
   <211> 156
   <212> PRT
   <213> Xenopus laevis
<400> 47
   Met Ser Ser Cys Ser Ile Val Ser Leu Leu Pro Gly Asp Lys Glu Pro
   1 5 10 15
   Ser Met Pro Asp Glu Trp Arg Leu Tyr Lys Leu Ala Thr Arg Leu Arg
   20 25 30
   Thr Phe Ser Asn Trp Pro Phe Thr Glu Asp Cys Ala Cys Thr Pro Glu
   35 40 45
   Arg Met Ala Glu Ala Gly Phe Val His Cys Pro Ser Asp Asn Ser Pro
   50 55 60
   Asp Val Val Lys Cys Phe Phe Cys Leu Lys Glu Leu Glu Gly Trp Gln
   65 70 75 80
   Pro Glu Asp Asp Pro Met Asp Glu His Lys Lys His Ser Pro Ser Cys
   85 90 95
   Leu Phe Ile Ala Leu Lys Lys Lys Ala Glu Glu Leu Thr Leu Ser Glu
   100 105 110
   Phe Leu Lys Leu Asp Leu Glu Arg Thr Lys Ile Lys Met Gln Lys Gln
   115 120 125
   Met Asn Leu Arg Ile Glu Cys Phe Gln Ala Lys Ala Asn Glu Val Arg
   130 135 140
   Gly His Leu Glu Gln Leu Asp Ala Asp Glu Thr Gln
   145 150 155
<210> 48
   <211> 142
   <212> PRT
   <213> Danio rerio
<400> 48
   Met Asp Leu Ala Ser Asp Asp Gln Thr Lys Met Tyr Phe Tyr Glu Asn
   1 5 10 15
   Arg Leu Gln Thr Phe Val Gly Trp Pro Phe Glu Glu Gly Cys Val Cys
   20 25 30
   Thr Pro Glu Asn Met Ala Lys Ala Gly Phe Ile His Thr Pro Ser Glu
   35 40 45
   Asn Ser Pro Asp Ile Ala Gln Cys Phe Phe Cys Leu Lys Glu Leu Glu
   50 55 60
   Gly Trp Glu Pro Glu Asp Asp Pro Glu Lys Glu His Lys Ala His Ser
   65 70 75 80
   Pro Ser Cys Asp Phe Ile Leu Leu Lys Lys Thr Val Asp Ser Leu Thr
   85 90 95
   Val Glu Glu Phe Leu Lys Leu Gln Lys Glu Arg Gln Lys Phe Ile Ile
   100 105 110
   Lys Lys Ser Cys Gly His Ala Ile Asp Lys Phe Glu Asp Ala Val Lys
   115 120 125
   Leu Lys Arg Asn Gln Ile Ile Gln Thr Ala Met Gly Glu Glu
   130 135 140
<210> 49
   <211> 128
   <212> PRT
   <213> Danio rerio
<400> 49
   Met Tyr Ser Tyr Glu Lys Arg Leu Gln Thr Phe Ser Glu Trp Pro Phe
   1 5 10 15
   Arg Glu Asp Cys Gln Cys Thr Pro Glu Leu Met Ala Lys Ala Gly Phe
   20 25 30
   Val His Cys Pro Ser Glu Asn Glu Pro Asp Val Ala Cys Cys Phe Tyr
   35 40 45
   Cys Leu Arg Glu Leu Glu Gly Trp Glu Pro Asp Asp Asn Pro Trp Ser
   50 55 60
   Glu His Ala Lys Arg Ser Pro Asn Cys Ala Phe Leu His Met Ser Lys
   65 70 75 80
   Thr Phe Asp Glu Leu Thr Ala Ile Glu Tyr Phe His Leu Glu Gln Glu
   85 90 95
   Arg Leu Arg Ile Tyr Ile Lys Lys Met Gly His Arg Lys Ile Ala Tyr
   100 105 110
   Phe Arg Asp Glu Val Glu Ala Thr Ser Lys Asn Leu Arg Ala Leu Ile
   115 120 125
<210> 50
   <211> 110
   <212> PRT
   <213> Tetraodon nigroviridis
<400> 50
   Met Asn Pro Tyr Asn Asp Gln His Leu Lys Met Tyr Phe Tyr Glu Asn
   1 5 10 15
   Arg Leu Lys Thr Phe Glu Gly Trp Pro Phe Glu Glu Asp Cys Leu Cys
   20 25 30
   Thr Pro Glu Asn Met Ala Lys Ala Gly Phe Val His Thr Pro Ser Glu
   35 40 45
   Asn Ser Pro Asp Thr Ala Met Cys Phe Phe Cys Leu Lys Glu Leu Glu
   50 55 60
   Gly Trp Glu Pro Asp Asp Glu Pro Lys Lys Glu His Lys Ser His Ser
   65 70 75 80
   Pro Ser Cys His Phe Ile Ala Leu Lys Lys Lys Val Glu Glu Leu Ser
   85 90 95
   Val Glu Glu Phe Val Lys Leu Gln Met Glu Arg Gln Lys Phe
   100 105 110
<210> 51
   <211> 116
   <212> PRT
   <213> Tetraodon nigroviridis
<400> 51
   Met Ala Ser Ile Asp Val Leu Lys Glu Arg Phe Ser Ser Tyr Asp Arg
   1 5 10 15
   Met Tyr Arg Gln Glu Leu Arg Glu Arg Thr Phe Val Asp Trp Pro Phe
   20 25 30
   Arg Glu Asp Cys Asn Cys Thr Pro Glu Lys Met Ala Lys Ala Gly Phe
   35 40 45
   Val His Cys Pro Ser Glu Asn Glu Pro Asp Val Ala Cys Cys Phe Phe
   50 55 60
   Cys Leu Ile Glu Leu Glu Gly Trp Glu Pro Asp Asp Asp Pro Trp Ser
   65 70 75 80
   Glu His Val Lys Arg Tyr Pro Asn Cys Gly Phe Leu Thr Met Lys Lys
   85 90 95
   Asp Phe Thr Glu Leu Thr Val Gly Glu Tyr Phe Leu Met Glu Gln Glu
   100 105 110
   Arg Leu Lys Val
   115
<210> 52
   <211> 140
   <212> PRT
   <213> Ictalurus punctatus
<400> 52
   Met Glu Leu Met Asn Glu Asp Leu Lys Met Tyr Phe Tyr Glu Asn Arg
   1 5 10 15
   Leu Ala Ser Tyr Ser Gly Trp Pro Phe Asp Glu Asp Cys Ala Cys Thr
   20 25 30
   Ser Asp Asn Met Ala Lys Ala Gly Phe Ile His Lys Pro Thr Glu Asn
   35 40 45
   Ser Pro Asp Val Ala Gln Cys Phe Phe Cys Phe Lys Glu Leu Glu Gly
   50 55 60
   Trp Glu Pro Glu Asp Asp Pro Leu Lys Glu His Lys Ser His Ser Pro
   65 70 75 80
   Thr Cys Asn Phe Ile Leu Leu Lys Lys Pro Val Glu Glu Leu Thr Val
   85 90 95
   Glu Glu Ile Leu Lys Leu Gln Lys Glu Arg Gln Lys Phe His Ile Lys
   100 105 110
   Met Ala Cys Arg Gln Val Ile Glu Lys Phe Glu Glu Ala Ala Lys Ser
   115 120 125
   Arg Arg Gly Asp Ile Ile Lys Leu Ala Met Glu Gly
   130 135 140
<210> 53
   <211> 72
   <212> PRT
   <213> Drosophila melanogaster
<400> 53
   Gln His Arg Val Glu Ser Tyr Lys Ser Trp Pro Phe Pro Glu Thr Ala
   1 5 10 15
   Ser Cys Ser Ile Ser Lys Met Ala Glu Ala Gly Phe Tyr Trp Thr Gly
   20 25 30
   Thr Lys Arg Glu Asn Asp Thr Ala Thr Cys Phe Val Cys Gly Lys Thr
   35 40 45
   Leu Asp Gly Trp Glu Pro Glu Asp Asp Pro Trp Lys Glu His Val Lys
   50 55 60
   His Ala Pro Gln Cys Glu Phe Ala
   65 70
<210> 54
   <211> 68
   <212> PRT
   <213> Caenorhabditis elegans
<400> 54
   Asp Arg Leu Met Thr Phe Lys Asn Phe Glu Tyr Asp Arg Asp Pro Asp
   1 5 10 15
   Ala Lys Cys Thr Ser Gln Ala Val Ala Gln Ala Gly Phe Tyr Cys Thr
   20 25 30
   Gly Pro Gln Ser Gly Lys Cys Ala Phe Cys Asn Lys Glu Leu Asp Phe
   35 40 45
   Asp Pro Glu Asp Asp Pro Trp Tyr Glu His Thr Lys Arg Asp Glu Pro
   50 55 60
   Cys Glu Phe Val
   65
<210> 55
   <211> 157
   <212> PRT
   <213> Artificial Sequence
<220>
   <223> Xenopus laevis SIX Survivin(T110M, S112G)
<400> 55
   Met Leu Ser Ile Ser Pro Ile Val Ser Leu Arg Arg Cys Asp Asn Glu
   1               5                   10                  15
   Pro Ser Met Pro Asp Glu Trp Arg Leu Tyr Asn Leu Ala Thr Arg Leu
               20                  25                  30
   Arg Thr Phe Ser Asn Trp Pro Phe Thr Glu Asp Cys Ala Cys Thr Pro
           35                  40                  45
   Glu Arg Met Ala Glu Ala Gly Phe Val His Cys Pro Thr Asp Asn Ser
       50                  55                  60
   Pro Asp Val Val Lys Cys Phe Phe Cys Leu Lys Glu Leu Glu Gly Trp
   65                  70                  75                  80
   Gln Pro Glu Asp Asp Pro Met Asp Glu His Lys Lys His Ser Pro Ser
                   85                  90                  95
   Cys Leu Phe Ile Ala Leu Lys Lys Lys Ala Glu Glu Leu Met Leu Gly
               100                 105                 110
   Glu Phe Leu Lys Leu Asp Leu Glu His Thr Lys Ile Lys Met Gln Lys
           115                 120                 125
   Gln Met Asn Leu His Ile Glu Arg Phe Gln Ala Lys Ala Asn Glu Val
       130                 135                 140
   Arg Gly His Leu Glu Lys Leu Asp Ala Asp Glu Thr Gln
   145                 150                 155
<210> 56
   <211> 142
   <212> PRT
   <213> Artificial Sequence
<220>
   <223> HumanSurvivin(R18E, P47L, Q56L, H77A, C84A, A128P, I135P)
<400> 56
   Met Gly Ala Pro Thr Leu Pro Pro Ala Trp Gln Pro Phe Leu Lys Asp
   1 5 10 15
   His Glu Ile Ser Thr Phe Lys Asn Trp Pro Phe Leu Glu Gly Cys Ala
   20 25 30
   Cys Thr Pro Glu Arg Met Ala Glu Ala Gly Phe Ile His Cys Leu Thr
   35 40 45
   Glu Asn Glu Pro Asp Leu Ala Leu Cys Phe Phe Cys Phe Lys Glu Leu
   50 55 60
   Glu Gly Trp Glu Pro Asp Asp Asp Pro Ile Glu Glu Ala Lys Lys His
   65 70 75 80
   Ser Ser Gly Ala Ala Phe Leu Ser Val Lys Lys Gln Phe Glu Glu Leu
   85 90 95
   Thr Leu Gly Glu Phe Leu Lys Leu Asp Arg Glu Arg Ala Lys Asn Lys
   100 105 110
   Ile Ala Lys Glu Thr Asn Asn Lys Lys Lys Glu Phe Glu Glu Thr Pro
   115 120 125
   Lys Lys Val Arg Arg Ala Pro Glu Gln Leu Ala Ala Met Asp
   130 135 140
<210> 57
   <211> 429
   <212> DNA
   <213> Homo sapiens
<400> 57
   atgggtgccc cgacgttgcc ccctgcctgg cagccctttc tcaaggacca ccgcatctct 60
   acattcaaga actggccctt cttggagggc tgcgcctgca ccccggagcg gatggccgag 120
   gctggcttca tccactgccc cactgagaac gagccagact tggcccagtg tttcttctgc 180
   ttcaaggagc tggaaggctg ggagccagat gacgacccca tagaggaaca taaaaagcat 240
   tcgtccggtt gcgctttcct ttctgtcaag aagcagtttg aagaattaac ccttggtgaa 300
   tttttgaaac tggacagaga aagagccaag aacaaaattg caaaggaaac caacaataag 360
   aagaaagaat ttgaggaaac tgcggagaaa gtgcgccgtg ccatcgagca gctggctgcc 420
   atggattga 429
<210> 58
   <211> 474
   <212> DNA
   <213> Xenopus laevis
<400> 58
   atgttgagta tttctccgat tgtttctcta cgtcgctgtg ataatgagcc gtctatgccc      60
   gacgaatggc gcctgtataa cttggctacg cgcctcagga ccttctctaa ctggcccttt     120
   actgaagact gcgcttgtac cccagagcgg atggcagagg ctggatttgt tcactgcccc     180
   actgacaaca gtccagatgt ggttaagtgt ttcttctgtc tcaaagaact agaaggttgg     240
   cagcctgagg atgaccctat ggatgaacac aagaaacatt caccaagctg cttattcatc     300
   gcattgaaaa agaaagcaga ggaactgaca ctaagcgagt tcctgaaact ggacttggag     360
   catacgaaaa tcaagatgca aaagcagatg aacctgcaca ttgaaaggtt ccaggcaaaa     420
   gcaaatgagg tgcggggtca cttggagaaa cttgatgctg acgaaacaca gtga           474
<210> 59
   <211> 483
   <212> DNA
   <213> Xenopus laevis
<400> 59
   atgtactctg ccaagaacag gtttgtgcaa gcagtgcaac gcctgcagga ttttaagaat      60
   atgtatgatt atgacgcccg ccttgcaaca ttcgcagact ggccctttac tgaaaactgc     120
   aagtgcaccc cagaaagtat ggctaaagct ggctttgttc actgccccac tgagaatgaa     180
   cccgatgttg cttgctgttt tttctgcttg aaggagctgg agggctggga gccagatgat     240
   gacccttgga ctgaacattc caagcgttct gccaactgtg gtttcctctc cctgacaaag     300
   tgtgtcaatg acctcacaat ggaggggttt ctgcggctgg aaggtgatcg gatcaagagt     360
   ttctatcgca agttttctac tgtcgttctc cagtacgtcg aggaagaaat gactgcagct     420
   acaaagcgcc tgctggaata cttctccaac caacaccatt gctccataga tcttgaccac     480
   tga                                                                   483
<210> 60
   <211> 429
   <212> DNA
   <213> Danio rerio
<400> 60
   atggatcttg caagtgatga tcagaccaaa atgtactttt acgagaatag acttcaaaca      60
   ttcgtcggtt ggccgtttga agagggctgc gtttgcactc cagaaaacat ggctaaagct     120
   ggatttattc acaccccgtc ggagaacagt ccagatatag cgcagtgttt cttctgtctg     180
   aaagagctgg aaggatggga accggaggat gaccctgaga aagagcataa agcgcattct     240
   cccagctgtg attttatctt gctgaagaag acagtggaca gtctgactgt ggaggaattt     300
   ctaaaacttc agaaagagag acagaagttt attattaaaa aatcatgtgg ccacgcgatt     360
   gataaatttg aagacgcagt gaagctcaag agaaaccaga tcatccagac tgcgatggga     420
   gaggaatga                                                             429
<210> 61
   <211> 423
   <212> DNA
   <213> Ictalurus punctatus
<400> 61
   atggagctta tgaacgaaga tttaaaaatg tacttttacg agaacagatt agcttcttat 60
   tcagggtggc cgtttgatga ggactgcgcc tgcacgtcgg ataatatggc caaagctggt 120
   ttcatccaca aacccactga aaacagtccg gatgtggccc agtgcttctt ctgctttaaa 180
   gagctggagg gttgggagcc tgaggatgat cctctcaagg aacacaagtc tcactctcca 240
   acttgcaatt tcattctgct gaaaaagcca gtggaggagc tcacagtgga ggagattctg 300
   aagctgcaga aagagaggca aaagttccac attaaaatgg cctgcagaca agtaattgag 360
   aagtttgagg aagcagcaaa atcgagaaga ggtgacatta tcaaactagc catggaaggc 420
   tga 423
<210> 62
   <211> 429
   <212> DNA
   <213> Artificial Sequence
<220>
   <223> sequence encoding Chickensurvivin(N97E, T99M, V100L, Q101G)
<400> 62
   atggcggcct atgctgaaat gctgcccaag gaatggctgg tctacctcgt ctccacccgc 60
   gccgccacct tccgcaactg gcccttcacc gagggctgcg cctgcacgcc cgagcggatg 120
   gcggcggcgg gcttcgtgca ctgccccagc gagaacagcc ccgacgtggt gcagtgcttc 180
   ttctgcctca aggagctgga gggctgggag cccgacgacg acccgctgga ggaacacaaa 240
   aagcactccg cgggctgcgc ttttgccgct cttcagaaag atccctctga actgatgttg 300
   ggggagttct tgaagctgga taaaaagcgg accaaaaacg taattaaaaa agcaatttct 360
   cagaaggaaa ctgatatcga agatgtagcc aagggcgtgc ggcacgcgat agagaacatg 420
   ggcccttag 429
<210> 63
   <211> 72
   <212> PRT
   <213> Homo sapiens
<400> 63
   Asp His Arg Ile Ser Thr Phe Lys Asn Trp Pro Phe Leu Glu Gly Cys
   1 5 10 15
   Ala Cys Thr Pro Glu Arg Met Ala Glu Ala Gly Phe Ile His Cys Pro
   20 25 30
   Thr Glu Asn Glu Pro Asp Leu Ala Gln Cys Phe Phe Cys Phe Lys Glu
   35 40 45
   Leu Glu Gly Trp Glu Pro Asp Asp Asp Pro Ile Glu Glu His Lys Lys
   50 55 60
   His Ser Ser Gly Cys Ala Phe Leu
   65 70
<210> 64
   <211> 142
   <212> PRT
   <213> Homo sapiens
<220>
   <221> misc_feature
   <223> GenBank accession number NM_001168.2
<400> 64
   Met Gly Ala Pro Thr Leu Pro Pro Ala Trp Gln Pro Phe Leu Lys Asp
   1               5                   10                  15
   His Arg Ile Ser Thr Phe Lys Asn Trp Pro Phe Leu Glu Gly Cys Ala
               20                  25                  30
   Cys Thr Pro Glu Arg Met Ala Glu Ala Gly Phe Ile His Cys Pro Thr
           35                  40                  45
   Glu Asn Glu Pro Asp Leu Ala Gln Cys Phe Phe Cys Phe Lys Glu Leu
       50                  55                  60
   Glu Gly Trp Glu Pro Asp Asp Asp Pro Ile Glu Glu His Lys Lys His
   65                  70                  75                  80
   Ser Ser Gly Cys Ala Phe Leu Ser Val Lys Lys Gln Phe Glu Glu Leu
                   85                  90                  95
   Thr Leu Gly Glu Phe Leu Lys Leu Asp Arg Glu Arg Ala Lys Asn Lys
               100                 105                 110
   Ile Ala Lys Glu Thr Asn Asn Lys Lys Lys Glu Phe Glu Glu Thr Ala
           115                 120                 125
   Glu Lys Val Arg Arg Ala Ile Glu Gln Leu Ala Ala Met Asp
       130                 135                 140
<210> 65
   <211> 142
   <212> PRT
   <213> Homo sapiens
<220>
   <221> misc_feature
   <223> GenBank accession number 015392
<400> 65
   Met Gly Ala Pro Thr Leu Pro Pro Ala Trp Gln Pro Phe Leu Lys Asp
   1               5                   10                  15
   His Arg Ile Ser Thr Phe Lys Asn Trp Pro Phe Leu Glu Gly Cys Ala
               20                  25                  30
   Cys Thr Pro Glu Arg Met Ala Glu Ala Gly Phe Ile His Cys Pro Thr
           35                  40                  45
   Glu Asn Glu Pro Asp Leu Ala Gln Cys Phe Phe Cys Phe Lys Glu Leu
       50                  55                  60
   Glu Gly Trp Glu Pro Asp Asp Asp Pro Ile Glu Glu His Lys Lys His
   65                  70                  75                  80
   Ser Ser Gly Cys Ala Phe Leu Ser Val Lys Lys Gln Phe Glu Glu Leu
                   85                  90                  95
   Thr Leu Gly Glu Phe Leu Lys Leu Asp Arg Glu Arg Ala Lys Asn Lys
               100                 105                 110
   Ile Ala Lys Glu Thr Asn Asn Lys Lys Lys Glu Phe Glu Glu Thr Ala
           115                 120                 125
   Lys Lys Val Arg Arg Ala Ile Glu Gln Leu Ala Ala Met Asp
       130                 135                 140
<210> 66
   <211> 142
   <212> PRT
   <213> Canis familiaris
<220>
   <221> misc_feature
   <223> GenBank accession number NP_001003019.1
<400> 66
<210> 67
   <211> 142
   <212> PRT
   <213> Sus scrofa
<220>
   <221> misc_feature
   <223> GenBank accession number NP_999306.1
<400> 67
   Met Ser Ala Pro Ser Leu Pro Pro Ala Trp Gln Leu Tyr Leu Lys Asp
   1 5 10 15
   His Arg Ile Ser Thr Phe Lys Asn Trp Pro Phe Leu Glu Gly Cys Ala
   20 25 30
   Cys Thr Pro Glu Arg Met Ala Ala Ala Gly Phe Ile His Cys Pro Thr
   35 40 45
   Glu Asn Glu Pro Asp Leu Ala Gln Cys Phe Phe Cys Phe Lys Glu Leu
   50 55 60
   Glu Gly Trp Glu Pro Asp Asp Asp Pro Ile Glu Glu His Lys Lys His
   65 70 75 80
   Ser Ser Gly Cys Ala Phe Leu Ser Val Lys Lys Gln Phe Glu Glu Leu
   85 90 95
   Thr Leu Ser Glu Phe Leu Lys Leu Asp Lys Glu Arg Ala Lys Asn Lys
   100 105 110
   Ile Ala Lys Glu Thr Asn Asn Lys Gln Lys Glu Phe Glu Glu Thr Ala
   115 120 125
   Lys Lys Val Arg Cys Ala Ile Glu Gln Leu Ala Ala Ser Glu
   130 135 140
<210> 68
   <211> 142
   <212> PRT
   <213> Bos taurus
<220>
   <221> mist feature
   <223> GenBank accession number NP_001001855.2
<400> 68
   Met Gly Ala Gln Ser Leu Pro Pro Ala Trp Gln Leu Tyr Leu Lys Asp
   1               5                   10                  15
   His Arg Val Ser Thr Phe Lys Asn Trp Pro Phe Leu Glu Gly Cys Ala
               20                  25                  30
   Cys Thr Pro Glu Arg Met Ala Ala Ala Gly Phe Ile His Cys Pro Thr
           35                  40                  45
   Glu Asn Glu Pro Asp Leu Ala Gln Cys Phe Phe Cys Phe Lys Glu Leu
       50                  55                  60
   Glu Gly Trp Glu Pro Asp Asp Asp Pro Ile Glu Glu His Lys Lys His
   65                  70                  75                  80
   Ser Ser Gly Cys Ala Phe Leu Ser Val Lys Lys Gln Phe Glu Glu Leu
                   85                  90                  95
   Thr Leu Ser Glu Phe Leu Lys Leu Asp Lys Glu Arg Thr Lys Asn Lys
               100                 105                 110
   Ile Ala Lys Glu Thr Asn Asn Lys Gln Lys Glu Phe Glu Glu Thr Ala
           115                 120                 125
   Lys Lys Val Arg Cys Ala Ile Glu Gln Leu Ala Ala Leu Glu
       130                 135                 140
<210> 69
   <211> 142
   <212> PRT
   <213> Felis catus
<220>
   <221> misc_feature
   <223> GenBank accession number NP_001009280.1
<400> 69
   Met Gly Ala Ser Ser Leu Pro Pro Ala Trp Gln Leu Tyr Leu Lys Asp
   1               5                   10                  15
   His Arg Ile Ser Thr Phe Lys Asn Trp Pro Phe Leu Glu Gly Cys Ala
               20                  25                  30
   Cys Thr Pro Glu Arg Met Ala Glu Ala Gly Phe Ile His Cys Pro Thr
           35                  40                  45
   Glu Asn Glu Pro Asp Leu Ala Gln Cys Phe Phe Cys Phe Lys Glu Leu
       50                  55                  60
   Glu Gly Trp Glu Pro Asp Asp Asp Pro Ile Glu Glu His Lys Lys His
   65                  70                  75                  80
   Ser Ser Gly Cys Ala Phe Leu Ser Val Lys Lys Gln Phe Glu Glu Leu
                   85                  90                  95
   Thr Leu Ser Glu Phe Leu Lys Leu Asp Lys Glu Arg Ala Lys Asn Lys
               100                 105                 110
   Ile Ala Lys Glu Thr Asn His Lys Gln Lys Glu Phe Glu Glu Thr Ala
           115                 120                 125
   Lys Arg Val Arg Cys Ala Ile Glu Gln Leu Ala Ala Leu Glu
       130                 135                 140
<210> 70
   <211> 140
   <212> PRT
   <213> Mus musculus
<220>
   <221> misc_feature
   <223> GenBank accession number NP_033819.1
<400> 70
   Met Gly Ala Pro Ala Leu Pro Gln Ile Trp Gln Leu Tyr Leu Lys Asn
   1               5                   10                  15
   Tyr Arg Ile Ala Thr Phe Lys Asn Trp Pro Phe Leu Glu Asp Cys Ala
               20                  25                  30
   Cys Thr Pro Glu Arg Met Ala Glu Ala Gly Phe Ile His Cys Pro Thr
           35                  40                  45
   Glu Asn Glu Pro Asp Leu Ala Gln Cys Phe Phe Cys Phe Lys Glu Leu
       50                  55                  60
   Glu Gly Trp Glu Pro Asp Asp Asn Pro Ile Glu Glu His Arg Lys His
   65                  70                  75                  80
   Ser Pro Gly Cys Ala Phe Leu Thr Val Lys Lys Gln Met Glu Glu Leu
                   85                  90                  95
   Thr Val Ser Glu Phe Leu Lys Leu Asp Arg Gln Arg Ala Lys Asn Lys
               100                 105                 110
   Ile Ala Lys Glu Thr Asn Asn Lys Gln Lys Glu Phe Glu Glu Thr Ala
           115                 120                 125
   Lys Thr Thr Arg Gln Ser Ile Glu Gln Leu Ala Ala
       130                 135                 140
<210> 71
   <211> 142
   <212> PRT
   <213> Rattus norvegicus
<220>
   <221> misc_feature
   <223> GenBank accession number AAF82586.1
<400> 71
   Met Gly Ala Thr Ala Leu Pro Pro Ile Trp Gln Met Tyr Leu Lys Asp
   1 5 10 15
   His Arg Ile Tyr Thr Phe Lys Asn Trp Pro Phe Leu Glu Asp Cys Ser
   20 25 30
   Cys Thr Pro Glu Arg Met Ala Glu Ala Gly Phe Ile His Cys Pro Thr
   35 40 45
   Glu Asn Glu Pro Asp Leu Ala Gln Cys Phe Phe Cys Phe Lys Glu Leu
   50 55 60
   Glu Gly Trp Glu Pro Asp Asp Asn Pro Ile Glu Glu His Arg Lys His
   65 70 75 80
   Ser Pro Gly Cys Ala Phe Leu Thr Val Lys Lys Gln Val Glu Glu Leu
   85 90 95
   Thr Val Ser Glu Phe Leu Lys Leu Asp Lys Gln Arg Ala Lys Asn Lys
   100 105 110
   Ile Ala Lys Glu Thr Asn Asn Lys Gln Lys Glu Phe Glu Glu Thr Arg
   115 120 125
   Arg Thr Val Arg Gln Ser Ile Glu Gln Leu Ala Ala Leu Arg
   130 135 140
<210> 72
   <211> 142
   <212> PRT
   <213> Pongo pygmaeus
<220>
   <221> misc_feature
   <223> GenBank accession number CAH91231.1
<400> 72
   Met Gly Ala Pro Thr Leu Pro Pro Ala Trp Gln Pro Phe Leu Lys Asp
   1 5 10 15
   His Arg Ile Ser Thr Phe Lys Asn Trp Pro Phe Leu Glu Gly Cys Ala
   20 25 30
   Cys Thr Pro Glu Arg Met Ala Glu Ala Gly Phe Ile His Cys Pro Thr
   35 40 45
   Glu Asn Glu Pro Asp Leu Ala Gln Cys Phe Phe Cys Phe Lys Glu Leu
   50 55 60
   Glu Gly Trp Glu Pro Asp Asp Asp Pro Ile Glu Glu His Lys Lys His
   65 70 75 80
   Ser Ser Gly Cys Ala Phe Leu Ser Val Lys Lys Gln Phe Glu Glu Leu
   85 90 95
   Thr Leu Gly Glu Phe Leu Lys Leu Asp Arg Glu Arg Ala Lys Asn Lys
   100 105 110
   Ile Ala Lys Glu Thr Asn Asn Lys Lys Lys Glu Phe Glu Glu Thr Ala
   115 120 125
   Lys Lys Val Arg Arg Ala Ile Glu Gln Leu Ala Ala Met Asp
   130 135 140
<210> 73
   <211> 142
   <212> PRT
   <213> Gallus gallus
<220>
   <221> misc_feature
   <223> GenBank accession number NP_001012318.1
<400> 73
   Met Ala Ala Tyr Ala Glu Met Leu Pro Lys Glu Trp Leu Val Tyr Leu
   1 5 10 15
   Val Ser Thr Arg Ala Ala Thr Phe Arg Asn Trp Pro Phe Thr Glu Gly
   20 25 30
   Cys Ala Cys Thr Pro Glu Arg Met Ala Ala Ala Gly Phe Val His Cys
   35 40 45
   Pro Ser Glu Asn Ser Pro Asp Val Val Gln Cys Phe Phe Cys Leu Lys
   50 55 60
   Glu Leu Glu Gly Trp Glu Pro Asp Asp Asp Pro Leu Glu Glu His Lys
   65 70 75 80
   Lys His Ser Ala Gly Cys Ala Phe Ala Ala Leu Gln Lys Asp Pro Ser
   85 90 95
   Asn Leu Thr Val Gln Glu Phe Leu Lys Leu Asp Lys Lys Arg Thr Lys
   100 105 110
   Asn Val Ile Lys Lys Ala Ile Ser Gln Lys Glu Thr Asp Ile Glu Asp
   115 120 125
   Val Ala Lys Gly Val Arg His Ala Ile Glu Asn Met Gly Pro
   130 135 140
<210> 74
   <211> 157
   <212> PRT
   <213> Xenopus laevis
<220>
   <221> misc_feature
   <223> GenBank accession number Aha020085.1
<400> 74
   Met Leu Ser Ile Ser Pro Ile Val Ser Leu Arg Arg Cys Asp Asn Glu
   1               5                   10                  15
   Pro Ser Met Pro Asp Glu Trp Arg Leu Tyr Asn Leu Ala Thr Arg Leu
               20                  25                  30
   Arg Thr Phe Ser Asn Trp Pro Phe Thr Glu Asp Cys Ala Cys Thr Pro
           35                  40                  45
   Glu Arg Met Ala Glu Ala Gly Phe Val His Cys Pro Thr Asp Asn Ser
       50                  55                  60
   Pro Asp Val Val Lys Cys Phe Phe Cys Leu Lys Glu Leu Glu Gly Trp
   65                  70                  75                  80
   Gln Pro Glu Asp Asp Pro Met Asp Glu His Lys Lys His Ser Pro Ser
                   85                  90                  95
   Cys Leu Phe Ile Ala Leu Lys Lys Lys Ala Glu Glu Leu Thr Leu Ser
               100                 105                 110
   Glu Phe Leu Lys Leu Asp Leu Glu His Thr Lys Ile Lys Met Gln Lys
           115                 120                 125
   Gln Met Asn Leu His Ile Glu Arg Phe Gln Ala Lys Ala Asn Glu Val
       130                 135                 140
   Arg Gly His Leu Glu Lys Leu Asp Ala Asp Glu Thr Gln
   145                 150                 155
<210> 75
   <211> 160
   <212> PRT
   <213> Xenopus laevis
<220>
   <221> misc_feature
   <223> GenBank accession number AAM76714.1
<400> 75
   Met Tyr Ser Ala Lys Asn Arg Phe Val Gln Ala Val Gln Arg Leu Gln
   1 5 10 15
   Asp Phe Lys Asn Met Tyr Asp Tyr Asp Ala Arg Leu Ala Thr Phe Ala
   20 25 30
   Asp Trp Pro Phe Thr Glu Asn Cys Lys Cys Thr Pro Glu Ser Met Ala
   35 40 45
   Lys Ala Gly Phe Val His Cys Pro Thr Glu Asn Glu Pro Asp Val Ala
   50 55 60
   Cys Cys Phe Phe Cys Leu Lys Glu Leu Glu Gly Trp Glu Pro Asp Asp
   65 70 75 80
   Asp Pro Trp Thr Glu His Ser Lys Arg Ser Ala Asn Cys Gly Phe Leu
   85 90 95
   Ser Leu Thr Lys Cys Val Asn Asp Leu Thr Met Glu Gly Phe Leu Arg
   100 105 110
   Leu Glu Gly Asp Arg Ile Lys Ser Phe Tyr Arg Lys Phe Ser Thr Val
   115 120 125
   Val Leu Gln Tyr Val Glu Glu Glu Met Thr Ala Ala Thr Lys Arg Leu
   130 135 140
   Leu Glu Tyr Phe Ser Asn Gln His Gln Cys Ser Ile Asp Leu Asp His
   145 150 155 160
<210> 76
   <211> 156
   <212> PRT
   <213> Xenopus laevis
<220>
   <221> misc_feature
   <223> GenBank accession number AAH89271.1
<400> 76
   Met Ser Ser Cys Ser Ile Val Ser Leu Leu Pro Gly Asp Lys Glu Pro
   1 5 10 15
   Ser Met Pro Asp Glu Trp Arg Leu Tyr Lys Leu Ala Thr Arg Leu Arg
   20 25 30
   Thr Phe Ser Asn Trp Pro Phe Thr Glu Asp Cys Ala Cys Thr Pro Glu
   35 40 45
   Arg Met Ala Glu Ala Gly Phe Val His Cys Pro Ser Asp Asn Ser Pro
   50 55 60
   Asp Val Val Lys Cys Phe Phe Cys Leu Lys Glu Leu Glu Gly Trp Gln
   65 70 75 80
   Pro Glu Asp Asp Pro Met Asp Glu His Lys Lys His Ser Pro Ser Cys
   85 90 95
   Leu Phe Ile Ala Leu Lys Lys Lys Ala Glu Glu Leu Thr Leu Ser Glu
   100 105 110
   Phe Leu Lys Leu Asp Leu Glu Arg Thr Lys Ile Lys Met Gln Lys Gln
   115 120 125
   Met Asn Leu Arg Ile Glu Cys Phe Gln Ala Lys Ala Asn Glu Val Arg
   130 135 140
   Gly His Leu Glu Gln Leu Asp Ala Asp Glu Thr Gln
   145 150 155
<210> 77
   <211> 942
   <212> DNA
   <213> Ictalurus punctatus
<220>
   <221> misc_feature
   <223> GenBank accession number CK419466.1
<220>
   <221> mise feature
   <222> (832)..(834)
   <223> n is a, c, g, or t
<400> 77
   ccacgcgtcc ggaacttctg aactaattag cgcctttttt gtgctttttt caaaaacatg 60
   gagcttatga acgaagattt aaaaatgtac ttttacgaga acagattagc ttcttattca 120
   gggtggccgt ttgatgagga ctgcgcctgc acgtcggata atatggccaa agctggtttc 180
   atccacaaac ccactgaaaa cagtccggat gtggcccagt gcttcttctg ctttaaagag 240
   ctggagggtt gggagcctga ggatgatcct ctcaaggaac acaagtctca ctctccaact 300
   tgcaatttca ttctgctgaa aaagccagtg gaggagctca cagtggagga gattctgaag 360
   ctgcagaaag agaggcaaaa gttccacatt aaaatggcct gcagacaagt aattgagaag 420
   tttgaggaag cagcaaaatc gagaagaggt gacattatca aactagccat ggaaggctga 480
   atggagattt ctgtcttttc ctcctatgat tgctcttttg ttatcggtca tggacatggt 540
   cccaagctgc actaccatgc ttgtgtttac taaaaaaaga atttattaag agattgacat     600
   tattcttcac aacctcttta atggagtttt atatcaactc cccatgatta cagaggtgga     660
   ttcatacagc ctggctaagt tccttctctg tattcatgat gttgcatgta taaaacagct     720
   agatactgta attggaactg aacagtccct taagtttaag tttttgtcca tgttttccta     780
   gtctgtaatg acatgaaagc tattatgact tggcatatcc tccttattta tnnnatatat     840
   tattattata attattattt taatttttat tttttttttt ttttacaaag tgtcttcaaa     900
   agttcacatt gtctatagag cactcagaca tttgacaatt tt                        942
<210> 78
   <211> 142
   <212> PRT
   <213> Danio rerio
<220>
   <221> misc_feature
   <223> GenBank accession number NP_919378.1
<400> 78
   Met Asp Leu Ala Ser Asp Asp Gln Thr Lys Met Tyr Phe Tyr Glu Asn
   1               5                   10                  15
   Arg Leu Gln Thr Phe Val Gly Trp Pro Phe Glu Glu Gly Cys Val Cys
               20                  25                  30
   Thr Pro Glu Asn Met Ala Lys Ala Gly Phe Ile His Thr Pro Ser Glu
           35                  40                  45
   Asn Ser Pro Asp Ile Ala Gln Cys Phe Phe Cys Leu Lys Glu Leu Glu
       50                  55                  60
   Gly Trp Glu Pro Glu Asp Asp Pro Glu Lys Glu His Lys Ala His Ser
   65                  70                  75                  80
   Pro Ser Cys Asp Phe Ile Leu Leu Lys Lys Thr Val Asp Ser Leu Thr
                   85                  90                  95
   Val Glu Glu Phe Leu Lys Leu Gln Lys Glu Arg Gln Lys Phe Ile Ile
               100                 105                 110
   Lys Lys Ser Cys Gly His Ala Ile Asp Lys Phe Glu Asp Ala Val Lys
           115                 120                 125
   Leu Lys Arg Asn Gln Ile Ile Gln Thr Ala Met Gly Glu Glu
       130                 135                 140
<210> 79
   <211> 128
   <212> PRT
   <213> Danio rerio
<220>
   <221> misc_feature
   <223> GenBank accession number NP_660196.1
<400> 79
   Met Tyr Ser Tyr Glu Lys Arg Leu Gln Thr Phe Ser Glu Trp Pro Phe
   1               5                   10                  15
   Arg Glu Asp Cys Gln Cys Thr Pro Glu Leu Met Ala Lys Ala Gly Phe
               20                  25                  30
   Val His Cys Pro Ser Glu Asn Glu Pro Asp Val Ala Cys Cys Phe Tyr
           35                  40                  45
   Cys Leu Arg Glu Leu Glu Gly Trp Glu Pro Asp Asp Asn Pro Trp Ser
       50                  55                  60
   Glu His Ala Lys Arg Ser Pro Asn Cys Ala Phe Leu His Met Ser Lys
   65                  70                  75                  80
   Thr Phe Asp Glu Leu Thr Ala Ile Glu Tyr Phe His Leu Glu Gln Glu
                   85                  90                  95
   Arg Leu Arg Ile Tyr Ile Lys Lys Met Gly His Arg Lys Ile Ala Tyr
               100                 105                 110
   Phe Arg Asp Glu Val Glu Ala Thr Ser Lys Asn Leu Arg Ala Leu Ile
           115                 120                 125
<210> 80
   <211> 110
   <212> PRT
   <213> Tetraodon nigroviridis
<220>
   <221> misc_feature
   <223> GenBank accession number CAG04432.1
<400> 80
   Met Asn Pro Tyr Asn Asp Gln His Leu Lys Met Tyr Phe Tyr Glu Asn
   1 5 10 15
   Arg Leu Lys Thr Phe Glu Gly Trp Pro Phe Glu Glu Asp Cys Leu Cys
   20 25 30
   Thr Pro Glu Asn Met Ala Lys Ala Gly Phe Val His Thr Pro Ser Glu
   35 40 45
   Asn Ser Pro Asp Thr Ala Met Cys Phe Phe Cys Leu Lys Glu Leu Glu
   50 55 60
   Gly Trp Glu Pro Asp Asp Glu Pro Lys Lys Glu His Lys Ser His Ser
   65 70 75 80
   Pro Ser Cys His Phe Ile Ala Leu Lys Lys Lys Val Glu Glu Leu Ser
   85 90 95
   Val Glu Glu Phe Val Lys Leu Gln Met Glu Arg Gln Lys Phe
   100 105 110
<210> 81
   <211> 116
   <212> PRT
   <213> Tetraodon nigroviridis
<220>
   <221> misc_feature
   <223> GenBank accession number CAG07433.1
<400> 81
   Met Ala Ser Ile Asp Val Leu Lys Glu Arg Phe Ser Ser Tyr Asp Arg
   1               5                   10                  15
   Met Tyr Arg Gln Glu Leu Arg Glu Arg Thr Phe Val Asp Trp Pro Phe
               20                  25                  30
   Arg Glu Asp Cys Asn Cys Thr Pro Glu Lys Met Ala Lys Ala Gly Phe
           35                  40                  45
   Val His Cys Pro Ser Glu Asn Glu Pro Asp Val Ala Cys Cys Phe Phe
       50                  55                  60
   Cys Leu Ile Glu Leu Glu Gly Trp Glu Pro Asp Asp Asp Pro Trp Ser
   65                  70                  75                  80
   Glu His Val Lys Arg Tyr Pro Asn Cys Gly Phe Leu Thr Met Lys Lys
                   85                  90                  95
   Asp Phe Thr Glu Leu Thr Val Gly Glu Tyr Phe Leu Met Glu Gln Glu
               100                 105                 110
   Arg Leu Lys Val
           115
<210> 82
   <211> 153
   <212> PRT
   <213> Drosophila melanogaster
<220>
   <221> misc_feature
   <223> GenBank accession number AAF55399.1
<400> 82
   Met Glu Ser Pro Val Val Asn Glu Val Ala Ala Ser Leu Gly Gly Glu
   1 5 10 15
   Lys Leu Glu Val Phe Arg Lys Leu Asn Leu Leu Glu Gln His Arg Val
   20 25 30
   Glu Ser Tyr Lys Ser Trp Pro Phe Pro Glu Thr Ala Ser Cys Ser Ile
   35 40 45
   Ser Lys Met Ala Glu Ala Gly Phe Tyr Trp Thr Gly Thr Lys Arg Glu
   50 55 60
   Asn Asp Thr Ala Thr Cys Phe Val Cys Gly Lys Thr Leu Asp Gly Trp
   65 70 75 80
   Glu Pro Glu Asp Asp Pro Trp Lys Glu His Val Lys His Ala Pro Gln
   85 90 95
   Cys Glu Phe Ala Lys Leu Ser Cys Pro Glu Arg Asn Leu Thr Val Ser
   100 105 110
   Gln Phe Leu Glu Ile Leu Gly Thr Val Val Lys Gly Ser Ile Glu Lys
   115 120 125
   Thr Cys Lys Ala Phe Lys Ser Ser Phe Val Arg Glu Asn Glu Lys Arg
   130 135 140
   Leu Asp Glu Phe Thr Arg Asn Gln Lys
   145 150
<210> 83
   <211> 155
   <212> PRT
   <213> Caenorhabditis elegans
<220>
   <221> misc_feature
   <223> GenBank accession number NP_505949.1
<400> 83
   Met Ala Pro Gly Thr Lys Lys Lys Ser Asp Met Ala Lys Phe Thr Phe
   1 5 10 15
   Tyr Lys Asp Arg Leu Met Thr Phe Lys Asn Phe Glu Tyr Asp Arg Asp
   20 25 30
   Pro Asp Ala Lys Cys Thr Ser Gln Ala Val Ala Gln Ala Gly Phe Tyr
   35 40 45
   Cys Thr Gly Pro Gln Ser Gly Lys Cys Ala Phe Cys Asn Lys Glu Leu
   50 55 60
   Asp Phe Asp Pro Glu Asp Asp Pro Trp Tyr Glu His Thr Lys Arg Asp
   65 70 75 80
   Glu Pro Cys Glu Phe Val Arg Ile Gly Lys Leu Asp Asp Ser Glu Leu
   85 90 95
   Thr Ile Asn Asp Thr Val Arg Leu Ser Gln Thr Ala Met Ile Met Thr
   100 105 110
   Lys Leu Phe Glu His Glu Met Met Ile Asn Asn Leu Ser Asn His Ser
   115 120 125
   Ser Ser Asp Ala Leu Phe Asp Gln Leu Lys Lys Val Pro Asn Thr Ala
   130 135 140
   Ser Thr Thr Lys Ser Asn Ser Arg Arg Gly Lys
   145 150 155
<210> 84
   <211> 142
   <212> PRT
   <213> Artificial Sequence
<220>
   <223> HumanSurv-ivin(R18E, H77A, c84A, A128P)
<400> 84
   Met Gly Ala Pro Thr Leu Pro Pro Ala Trp Gln Pro Phe Leu Lys Asp
   1 5 10 15
   His Glu Ile Ser Thr Phe Lys Asn Trp Pro Phe Leu Glu Gly Cys Ala
   20 25 30
   Cys Thr Pro Glu Arg Met Ala Glu Ala Gly Phe Ile His Cys Pro Thr
   35 40 45
   Glu Asn Glu Pro Asp Leu Ala Gln Cys Phe Phe Cys Phe Lys Glu Leu
   50 55 60
   Glu Gly Trp Glu Pro Asp Asp Asp Pro Ile Glu Glu Ala Lys Lys His
   65 70 75 80
   Ser Ser Gly Ala Ala Phe Leu Ser Val Lys Lys Gln Phe Glu Glu Leu
   85 90 95
   Thr Leu Gly Glu Phe Leu Lys Leu Asp Arg Glu Arg Ala Lys Asn Lys
   100 105 110
   Ile Ala Lys Glu Thr Asn Asn Lys Lys Lys Glu Phe Glu Glu Thr Pro
   115 120 125
   Lys Lys Val Arg Arg Ala Ile Glu Gln Leu Ala Ala Met Asp
   130 135 140

## Description

### FIELD OF THE INVENTION

This invention relates to vaccination strategies for tumor treatment. Specifically, this invention relates to vaccine compositions stimulating in a human an immune response to human survivin, thus attacking tumor diseased cells overexpressing survivin. In particular, the present invention relates to vaccine compositions comprising peptides derived from non-mammalian survivin as well as from modified mammalian, especially human, survivin.
This application is a divisional application of EP 06 805 883.3 filed on 27.09.2006

### BACKGROUND

The immune system carries out its surveillance function in part by monitoring the protein compositions of cells. In a process referred to as T cell antigen presentation, proteins inside a cell are processed into peptides. A subset of the processed peptides, the antigens, are exported to the cell surface, in a specific association with a member of a class of receptors known as the major histocompatibility complex (MHC) receptors. These MHC-peptide complexes are sampled by specialized immune cells, T cells, via an interaction with T cell surface proteins known as T Cell Receptors (TCRs). Each given T cell carries a unique TCR on its surface. Upon a productive interaction of the T cell's TCR with the MHC-peptide complex, an immune response is activated which leads to the elimination of the cell presenting that particular MHC-peptide complex. Generally the interaction is non-productive if the presented antigens are derived from the host, so-called "self" antigens. Commonly, immune responses are activated by the interaction with an antigen derived from a foreign protein, for example, with an antigen derived from a constituent protein of an infecting virus.

The aim of treating tumors by active immunization is to induce the immune system to recognize and to eliminate tumor cells. Just as in any other cell, peptide antigens on tumor cells are derived from the repertoire of expressed proteins. Tumor-enriched antigens derived from differentially expressed proteins in tumors are in principle a target of immune surveillance just as viral antigens are in infected cells. Accordingly, it would be useful to harness branches of the immune system that have evolved to counter an intracellular infection for the purpose of eliminating a tumor.

One such tumor-enriched protein is survivin. Tumor cells often overexpress the survivin protein, which is believed to block apoptosis and thus interfere with mechanisms that prevent the abnormal growth of tumor cells. Normal cells, on the other hand, express very little survivin. (see, *e.g.*, Ambrosini et al., (1997) Nat. Med., 3:917-921). Therefore, survivin is an ideal tumor-specific or tumor-enriched antigen. It would be useful to deliver an antigenic form of the survivin protein to antigen-presenting cells, so that an immune response to survivin may be stimulated. However, in contrast to virally infected cells, survivin, like many other differentially expressed proteins in tumors, are host proteins and do not activate the immune system. Therefore, there remains a need in the art to develop effective compositions and methods that elicit an effective immune response to tumor cells, particularly those characterized with the overexpression of survivin.

### SUMMARY OF THE INVENTION

The present invention provides effective compositions and methods that elicit an effective immune response to tumor cells. Specifically, the present invention provides vaccination strategies using antigenic forms of tumor-enriched proteins as T cell antigens. In particular, the present invention provides an antigenic form of a tumor-enriched protein, survivin, preferably human survivin.

The present invention relates to a vaccine including a nucleic acid encoding a modified human survivin peptide. Alternatively, the present invention relates to a vaccine including a modified human survivin peptide. The modified human survivin peptide is biologically inert but immunologically substantially similar to human survivin. As used herein, by "biologically inert" is meant a survivin peptide that is not capable of substantially exerting or affecting an activity usually associated with normal survivin. For example, a "biologically inert" survivin peptide does not have a substantial anti-apoptotic activity, nor is it capable of substantially interfering with an anti-apoptotic activity usually associated with an endogenous survivin protein. A biologically inert survivin would not typically have a substantial dominant-negative effect. As used herein, by "immunologically substantially similar to human survivin" is meant a modified survivin peptide capable of eliciting at least one immune response to the same target sequence as is the corresponding non-modified human survivin peptide. An immune response may be measured, for example, by the CD8+ T cell population or the IFNγ release by the CD8+ T cells in response to a target sequence.

In one preferred embodiment, the vaccine of the invention includes a nucleic acid encoding a modified human survivin peptide. The nucleic acid may include a mammalian promoter. In one embodiment, the nucleic acid is a naked DNA. In another embodiment, the nucleic acid is formulated with a reagent that enhances transfection of mammalian cells. The vaccine of the present invention may also include an adjuvant.

In one embodiment, the nucleic acid encoding a modified human survivin peptide is part of a viral particle, for example, an adenoviral particle. Adenoviral particles suitable for the invention include those derived from adenoviruses capable of replication, or those derived from conditionally replicating adenoviruses ("CRADs") that replicate in only certain cells or only under certain conditions, or those derived from adenoviruses that are replication-incompetent. Other suitable viral particles include, but are not limited to, viral particles derived from lentiviruses or other RNA viruses, adeno-associated viruses, rotaviruses, or vaccinia viruses, to name but a few.

In one embodiment, the vaccine includes a bacterium containing the nucleic acid encoding a modified human survivin peptide. Preferably, the bacterium is an enteric bacterium, such as a Salmonella, Escherichia, or Klebsiella. Other bacteria, such as Listeria species, may also be used to host such nucleic acids. The bacterium may be a wild-type bacterium or may contain mutations that, for example, attenuate its pathogenicity. Generally, it is preferred to use a mutant form of a bacterium, such as an auxotroph.

In another preferred embodiment, the vaccine of the invention includes a modified human survivin peptide. Preferably, the modified human survivin peptide includes a modified BIR domain. The modified BIR domain may include at least one amino acid substitution at a position corresponding to Arg18, Cys57, Cys60, His77 and Cys84 of human survivin. Alternatively, or in addition the modified human survivin peptide includes a modified helical domain. The modified helical domain may include at least one amino acid substitution at a position corresponding to Lys 122, Alga128, and Ile 135 of human survivin. For example, the modified helical domain may contain a Pro at a position corresponding to Ala 128 of human survivin. The modified helical domain may also include a Pro at a position corresponding to IIe135. The modified human survivin peptide preferably contains one or more MHC Class I T cell epitopes. For example, the modified human survivin peptide may include the amino acid sequence LTLGEFLKL (SEQ ID NO: 1) or LMLGEFLKL (SEQ ID NO: 6).

In some embodiments, the modified human survivin peptide is fused to an Fc moiety. A preferred Fc moiety is derived from a mammalian Fc region, more preferably from a human Fc region. The Fc moiety may contain mutations that improve assembly, for example, substitution of the cysteine in the sequence EPKSCDK (SEQ ID NO: 4) of the IgG1 hinge with a serine. As a result, the Fc moiety may contain a modified sequence EPKSSDK (SEQ ID NO: 5). The Fc moiety may also contain mutations that reduce immunogenicity of regions for which an immune response is not desired, for example, the junction between the fusion protein moieties.

In certain embodiments, the modified human survivin peptide functions in concert with an effector molecule that contributes to the immune response. For example, such an effector molecule can be a cytokine moiety including, but not limited to, IL-2, IL-7, IL-12, IL-18, IL-21, IL-23, GM-CSF, or any other cytokine, particularly one capable of activating a Th1 immune response. Such an effector molecule can also be an inhibitor of a cytokine that represses the immune system, for example, a STAT3 inhibitor (e.g., a dominant negative STAT3 protein such as STAT3β). Cytokines or other effector molecules may also contain mutations. For example, a cytokine may contain a Ser substitution at a position corresponding to Cys125 of IL-2.

Thus, in preferred embodiments, the vaccine of the invention includes a nucleic acid encoding a modified human survivin peptide and a second peptide including an effector molecule described above that contributes to the immune response. The regions encoding the modified human survivin peptide and the effector molecule peptide can be in a single transcription unit, or in two separate transcription units. Alternatively, the vaccine of the invention includes a nucleic acid encoding a modified human survivin peptide and a separate nucleic acid encoding a second peptide including an effector molecule described above that contributes to the immune response.

In other embodiments, the vaccine of the invention includes a modified human survivin peptide and a second peptide including an effector molecule described above that contributes to the immune response. In one embodiment, the modified human survivin peptide is fused or conjugated to the peptide including an effector molecule. The modified human survivin peptide and effector molecule fusion protein may be further fused or conjugated to an Fc moiety as described above.

In another aspect, the present invention relates to a nucleic acid capable of eliciting an immune response against cells expressing human survivin. The nucleic acid encodes a peptide including an amino acid sequence with more than 50% identity but less than 80% identity with the human survivin BIR domain and the nucleic acid contains a promoter capable of expressing the peptide in a mammalian cell.

The present invention also relates to a method of treatment comprising administering a vaccine or a nucleic acid as described above. In particular, the invention provides methods of treatment for cancer and other diseases involving unwanted cell proliferation. The methods of treatment of the invention may optionally include other steps, for example, a step first testing a patient's tumor for expression of high levels of survivin. Another optional step is pre-treatment of the patient with a mildly immunosuppressive agent, such as a low-dose of cyclophosphamide, before administering a vaccine or a nucleic acid of the invention. Without wishing to be bound by theory, one effect of such a pre-treatment is to reduce numbers of T-regulatory cells. Therefore, other pre-treatments that have similar effect are encompassed in the invention.

In addition to survivin, the present invention can be applied to a wide variety of proteins that can be used as antigens, such as tumor specific antigens, viral antigens, and other antigens. Among tumor-specific antigens, in addition to survivin, the invention may be applied to epithelial cell adhesion molecules, oncogenes such as Ras, carcinoembryonic antigen, and other tumor specific or tumor-enriched proteins. Among viral antigens, the invention may be applied to p24 of HIV, influenza hemmagglutinin, and other viral proteins.

In summary the invention is related to the following items:
Although this patent application is specifically focused to mammalian preferably human modified survivin, in the following, the Figures, Detailed Description and Examples contain data and results from non-human survivin in addition to human survivin.
This additional information is provided for illustrative and comparative purposes.

### BRIEF DESCRIPTION OF THE FIGURES

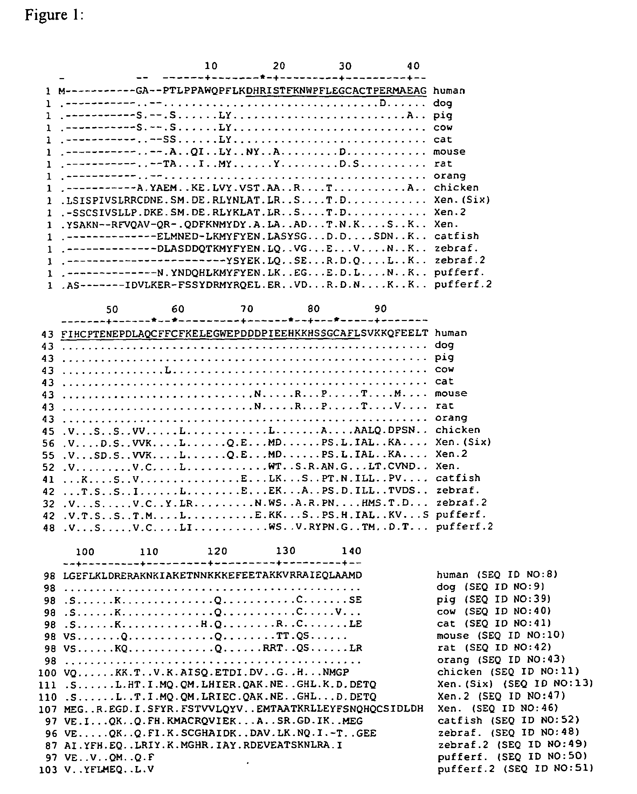

Figure 1 is an alignment of protein sequences of survivin homologs. Alignments were performed by the Clustal W method (see Thompson et al. (1994) Nucl. Acid Res. 22:4673-4680). Sequences were obtained from the NCBI database, using the following Accession numbers: human survivin (Accession #: NM_001168, SEQ ID NO:8), dog survivin (NP_001003019, SEQ ID NO:9), pig survivin (NP_999306, SEQ ID NO:39), cow survivin (NP_001001855, SEQ ID NO:40), cat survivin (NP_001009280, SEQ ID NO:41), mouse survivin (NP_033819, SEQ ID NO:10), rat survivin (AAF82586, SEQ ID NO:42), orangutan survivin (CAH91231, SEQ ID NO:43), chicken survivin (NP_001012318, SEQ ID NO:11), Xenopus laevis SIX (AAO20085, SEQ ID NO:13), Xenopus laevis survivin homolog (AAM76714, SEQ ID NO:46), Xenopus laevis survivin homolog 2 (AAH89271, SEQ ID NO:47), catfish survivin homolog (CK419466, SEQ ID NO:52), zebrafish survivin homolog (NP_919378, SEQ ID NO:48), zebrafish survivin homolog 2 (NP_660196, SEQ ID NO:49), pufferfish survivin-like homolog (CAG04432, SEQ ID NO:50), and pufferfish survivin-like homolog 2 (CAG07433, SEQ ID NO:51). The BIR domain is underlined; asterisks indicate signature residues involved in Zn coordination; and dashes indicate gaps in the alignment. In the non-human sequences, residues that are identical to the human residues are represented by periods; other residues are represented in single-letter amino acid code.

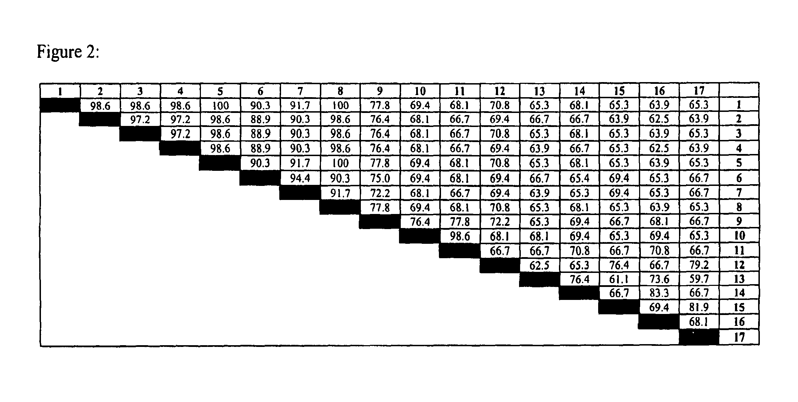

Figure 2 is a table of amino acid percentage identities for comparisons of BIR domains from various survivin homologs. Sequence 1 is the human sequence; sequence 2 is from dog; sequence 3 is from pig; sequence 4 is from cow; sequence 5 is from cat; sequence 6 is from mouse; sequence 7 is from rat; sequence 8 is from orangutan; sequence 9 is from chicken; sequence 10 is from the frog Xenopus laevis SIX protein; sequence 11 is from a frog Xenopus laevis homolog; sequence 12 is from frog Xenopus laevis homolog 2; sequence 13 is from a catfish homolog; sequence 14 is from a zebrafish homolog; sequence 15 is from zebrafish homolog 2; sequence 16 is from pufferfish survivin-like protein 1; and sequence 17 is from pufferfish survivin-like protein 2.

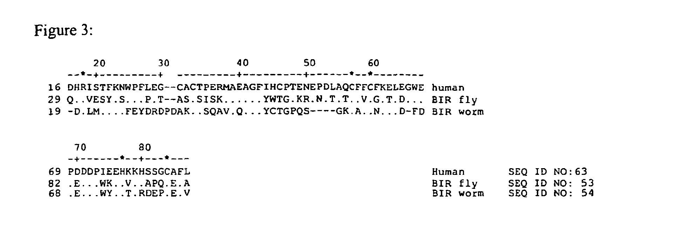

Figure 3 is an alignment of BIR domain sequences from invertebrates with human survivin. The human survivin BIR domain sequence is shown in SEQ ID NO:63. Alignments were performed by the ClustalW method (see Thompson et al. (1994) Nucl. Acid Res. 22:4673-4680). Sequences were obtained from the NCBI database, using the following accession numbers: human survivin (NM_001168, BIR domain sequence shown in SEQ ID NO:63), *D. melanogaster* (AAF55399, BIR domain homolog shown in SEQ ID NO:53) and *C. elegans* (NP_505949, BIR domain homolog shown in SEQ ID NO:54). The BIR domain is underlined; asterisks indicate signature residues involved in Zn coordination; and dashes indicate gaps in the alignment. In the non-human sequences, residues that are identical to the human residues are represented by periods; other residues are represented in single-letter amino acid code.

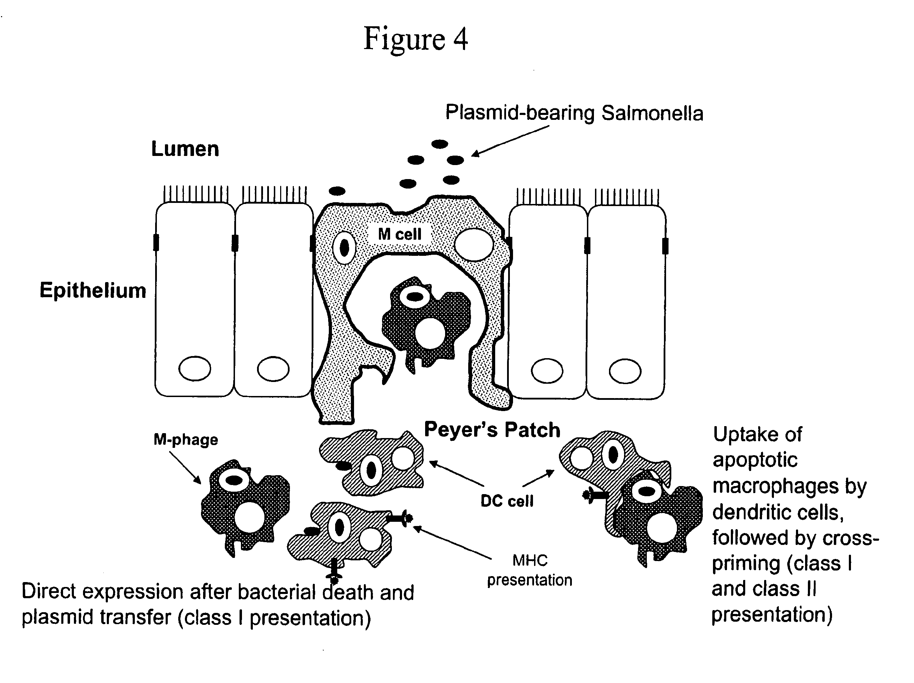

Figure 4 is a schematic depiction of modes of DNA vaccination by Salmonella bearing plasmids encoding a survivin polypeptide. Salmonella in the gut lumen invade the gut epithelium through M cells of Peyer's patches. Once across the epithelium, Salmonella invade cells such as macrophages and dendritic cells. Bacterial death and plasmid transfer permit expression of the plasmid by the macrophages, leading to presentation of peptides from the encoded survivin polypeptide in MHC class I molecules. In addition, uptake by dendritic cells of apoptotic macrophages expressing the survivin polypeptide leads to cross-priming resulting from presentation of the peptides in MHC class I and MHC class II molecules.

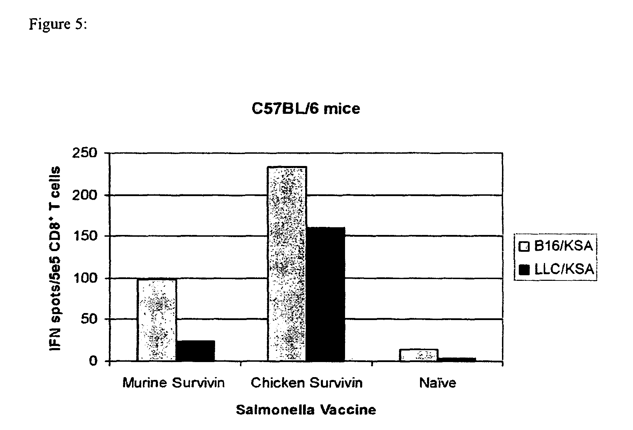

Figure 5 is a bar graph depicting IFNγ release from T-cells isolated from survivin-vaccinated mice upon exposure to cancer cells. IFNγ release was measured in an ELISpot assay. C57B1/6 mice were vaccinated with Salmonella carrying either a murine or chicken survivin expression plasmid, or were not vaccinated (x-axis), and the number of IFNγ spots per 5 x 10⁵ plated T-cells was measured (y-axis) after T cells were exposed to either B16/KSA (striped bars) or LLC/KSA (black bars) cancer cell lines.

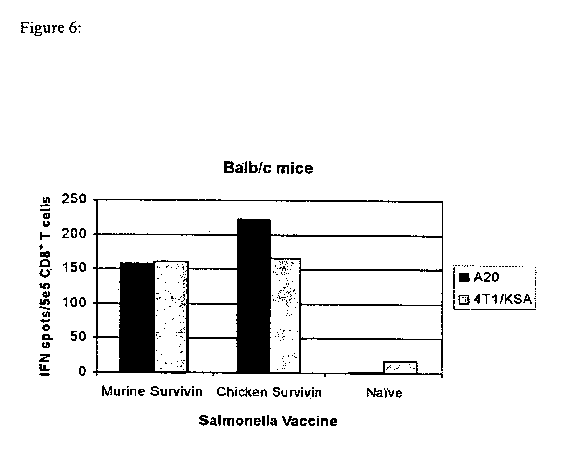

Figure 6 is a bar graph depicting IFNγ release from T-cells isolated from survivin-vaccinated mice upon exposure to cancer cells. IFNγ release was measured in an ELISpot assay. Balb/c mice were vaccinated with Salmonella carrying either a murine or chicken survivin expression plasmid, or were not vaccinated (x-axis), and the number of IFNγ spots per 5 x 10⁵ plated T-cells was measured (y-axis) after T cells were exposed to either 4T1/KSA (striped bars) or A20 (black bars) cancer cell lines.

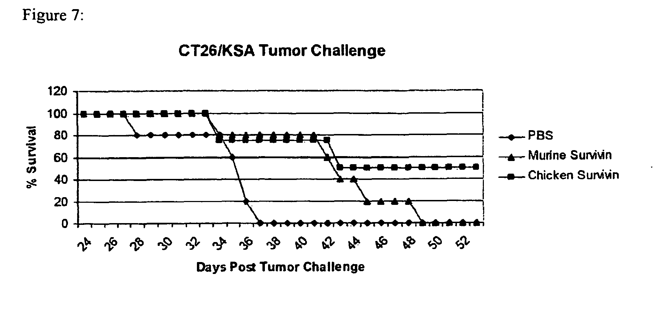

Figure 7 is a survival plot of mice vaccinated with Salmonella SL7207 chicken survivin or murine survivin. The percentage of surviving Balb/c mice (y-axis) is plotted against number of days post CT26/KSA challenge (x-axis) for mice vaccinated with either Salmonella carrying an expression plasmid for murine survivin (filled triangles) or a chicken survivin (filled squares), or PBS (filled diamonds).

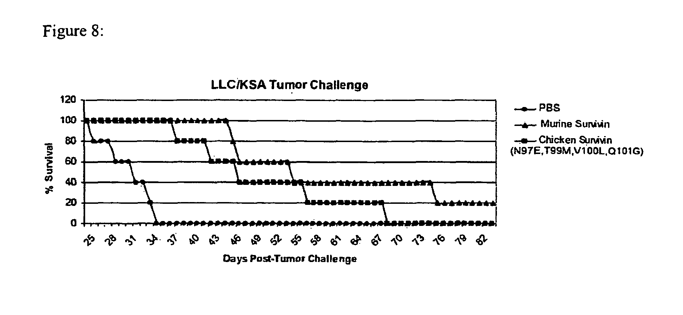

Figure 8 is a survival plot of mice vaccinated with Salmonella SL7207 chicken survivin or murine survivin. % of surviving C57B1/6 mice (y-axis) is plotted against number of days post LLC/KSA challenge (x-axis) for mice vaccinated with either Salmonella carrying an expression plasmid for murine survivin (filled triangles) or a chicken survivin mutant, ChickenSurvivin(N97E, T99M, V100L, Q101G) (filled squares), or PBS (filled diamonds).

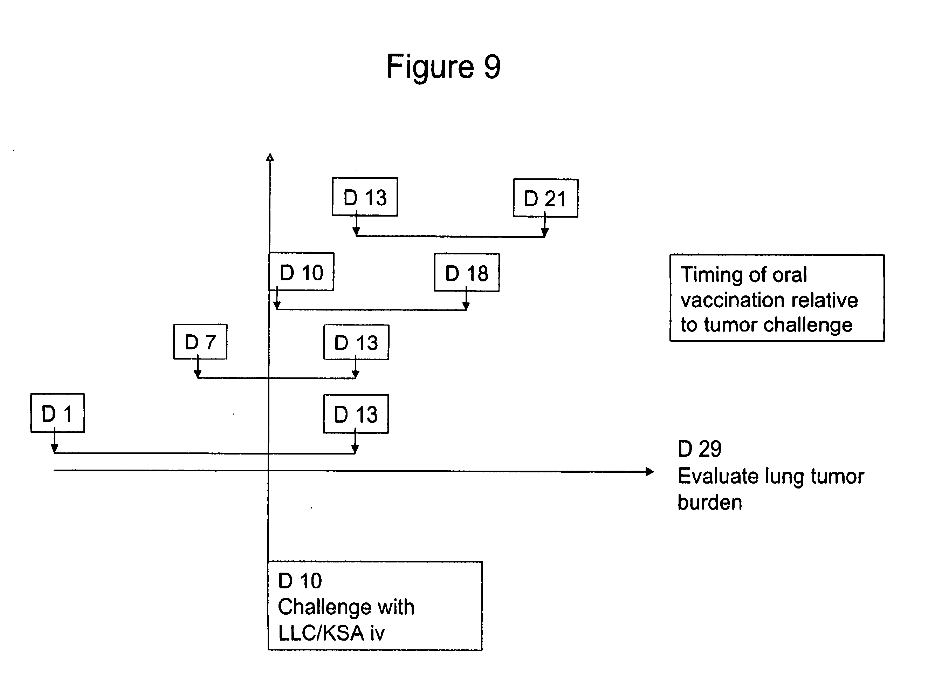

Figure 9 is a schematic depiction a dosing schedule to evaluate the effect of vaccination timing relative to a tumor challenge on day 10. "D" is an abbreviation for "day." Thus, for example, some mice received oral vaccination on day 1, a tumor challenge on day 10, and oral vaccination on day 13; other mice received a tumor challenge and an oral vaccination on day 10, and a second oral vaccination on day 18. Lung tumor burdens were evaluated on day 29.

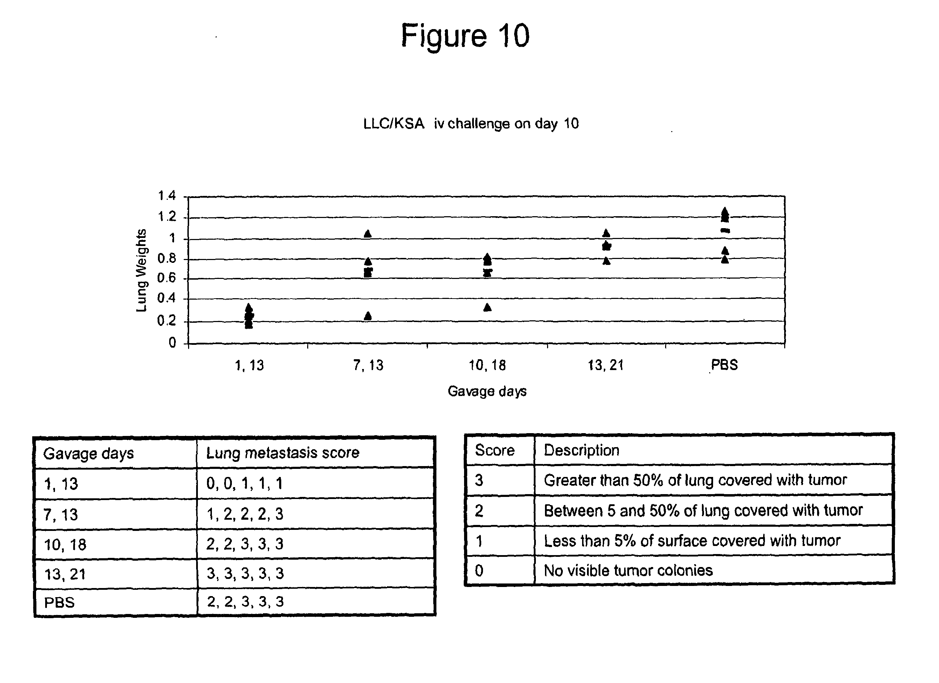

Figure 10 is a chart depicting lung weights for mice receiving vaccinations on days 1 and 13; 7 and 13; 10 and 18; or 13 and 21; or receiving PBS. All mice received tumor challenges of LLC/KSA cells intravenously on day 10. Lung metastasis scores for each of the mice are also provided.

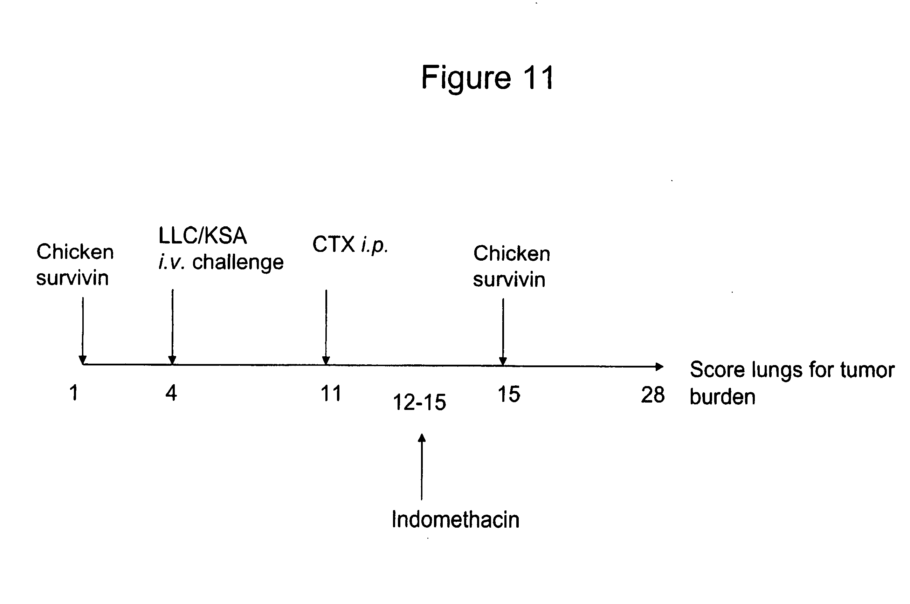

Figure 11 is a schematic depiction of a dosing schedule, including Salmonella-mediated vaccinations with chicken survivin on days 1 and 15; intravenous tumor challenge with LLC/KSA cells on day 4; and intraperitoneal cyclophosphamide (CTX) on day 11 and indomethacin treatment on days 12-15. Lungs were scored for tumor burden on day 28.

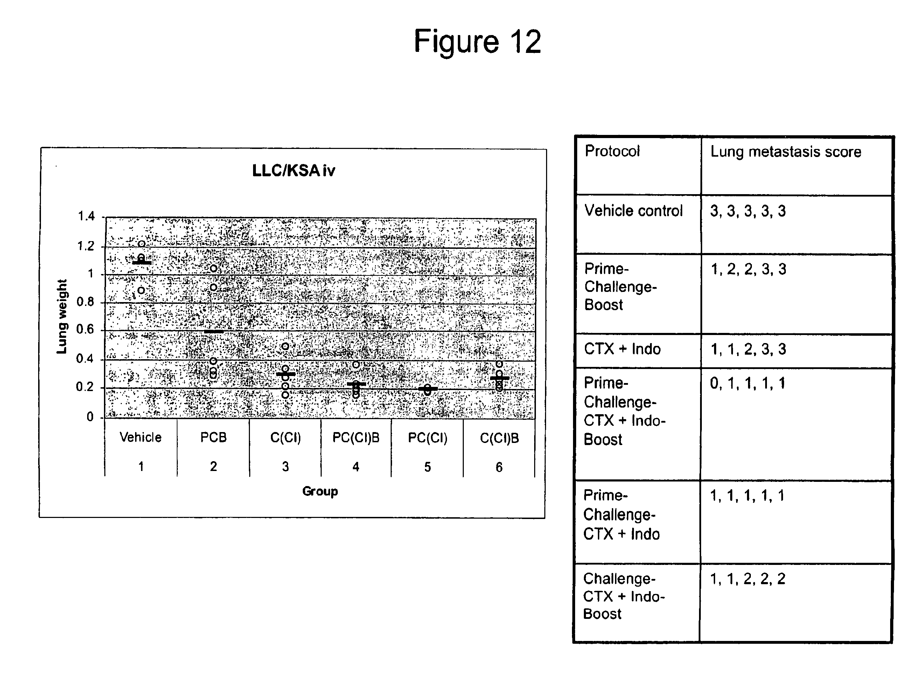

Figure 12 is a chart depicting lung weights for mice receiving tumor challenge only (vehicle); vaccination with chicken survivin on days 1 (prime) and 15 (boost) and tumor challenge on day 4 (PCB); CTX and indomethacin and tumor challenge C(CI); prime, challenge, CTX, indomethacin, and boost (PC(CI)B); prime, challenge, CTX, and indomethacin (PC(CI)), or challenge, CTX, indomethacin and boost C(CI)B. Lung metastasis scores for each of the mice are also provided.

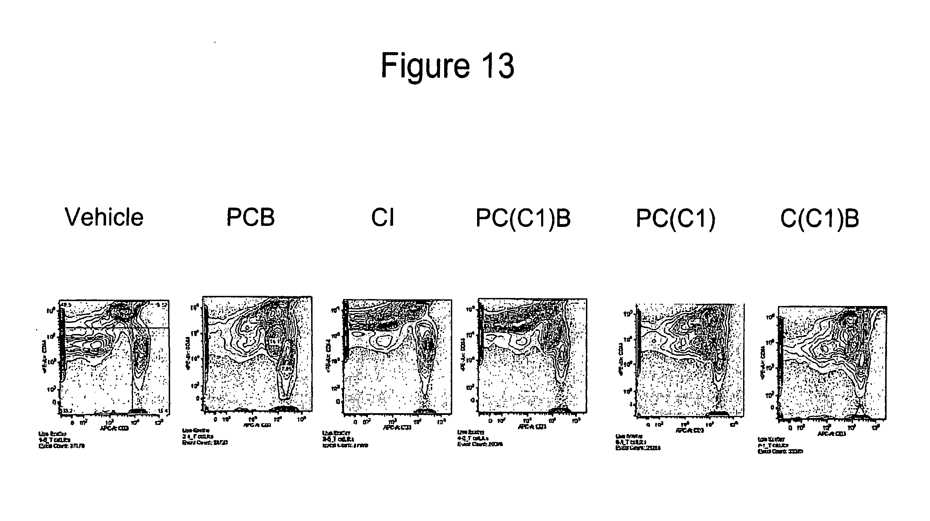

Figure 13 provides depictions of flow cytometry data from peripheral blood cells demonstrating the appearance of a novel population of CD44^{bright}, CD3^{low} cells in mice after vaccination with Salmonella carrying a plasmid encoding non-mammalian survivin. The x-axis represents the CD3 cells and the y-axis represents the CD44 cells.

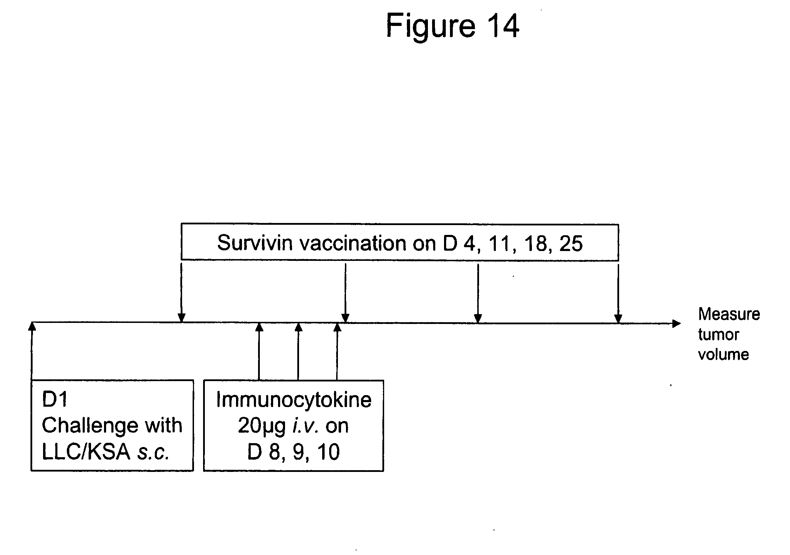

Figure 14 is a schematic depiction of a dosing schedule, including subcutaneous tumor challenge on day 1; Salmonella-mediated vaccinations with non-mammalian survivin on days 4, 11, 18, and 25; and intravenous immunocytokine treatment on days 8, 9 and 10.

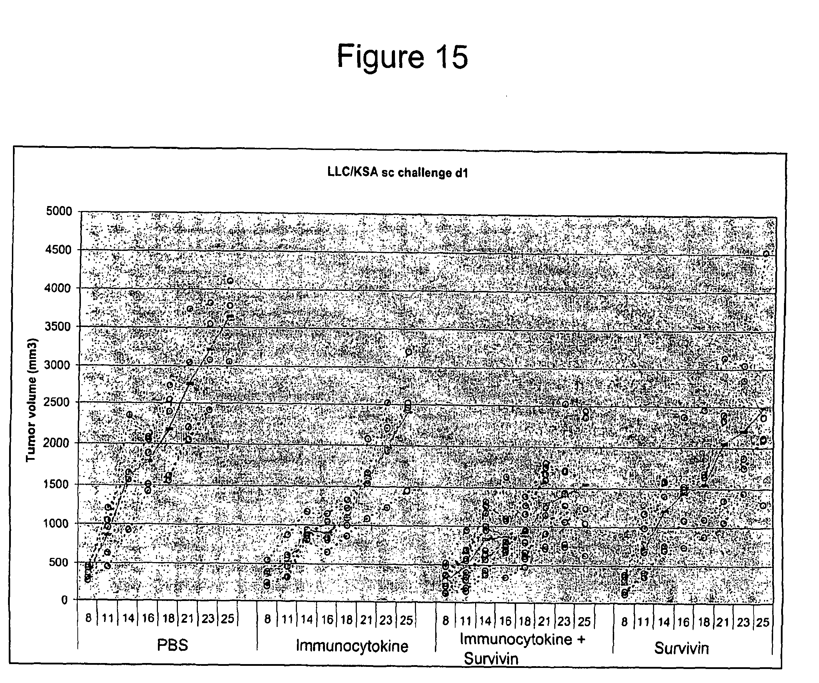

Figure 15 is a chart depicting tumor volumes over time for mice treated with PBS; immunocytokine; non-mammalian survivin; or immunocytokine and non-mammalian survivin.

### DETAILED DESCRIPTION OF THE INVENTION

The present invention improves treatment of cancer or other diseases by providing compositions and methods that effectively elicit immune response against diseased cells. Cancer is the primary target disease, although the invention applies to other diseases and conditions such as unwanted proliferation of normal cells, such as fibroid tissue. The invention also contemplates compositions and methods for treatment of viral infection, such as HIV infections, influenza virus infections, and other viral infections.

In particular, for the treatment of cancer or other tumors, the present invention provides compositions and methods for the presentation of a peptide related to a tumor-specific or tumor-enriched protein on antigen presenting cells to elicit an adaptive immune response in a mammal against cancer or tumor cells. One preferred tumor-specific or tumor-enriched antigen for this invention is survivin.

### Overview of T-cell antigen presentation

Antigen presentation is a cellular process in which proteins in cells are processed into peptides and then trafficked along the secretory pathway to the cell surface. The processed peptides are trafficked as a stable complex with specialized peptide-presenting membrane proteins, known as MHCs. This process allows components of the immune system, e.g., the helper T-cells (CD4⁺ T-cells) and cytotoxic T-cells (CTLs or CD8⁺ T-cells), to survey the compositions of cells by sampling an MHC-peptide complex. Helper T-cells interact with MHC II-peptide complexes, whereas cytotoxic T-cells interact with MHC I-peptide complexes. The interaction occurs via a T-cell receptor (TCR) expressed on the surface of a T-cell. Each T-cell expresses a unique TCR. Analogous to antibodies, TCR diversity is generated through rearrangement at the chromosomal TCR loci. As with antibody producing cells, encounter with a self-antigen (i.e., an antigen peptide generated from an endogenous protein present in the cell) during T-cell differentiation leads to the negative selection of that particular T-cell. Those T cells that are not eliminated during negative selection further develop into mature T cells. Eventually, interaction with a foreign antigen complexed with MHCs leads to T-cell activation, especially in the presence of a secondary stimulation.

### Survivin

Survivin protein is characterized by a conserved, approximately 70 amino acid domain, known as a BIR domain (or IAP domain). In human survivin, the BIR domain corresponds to the region spanning from Asp16 to Leu87. Cancer or tumor cells often overexpress the survivin protein, which is believed to block apoptosis of these cells and thus interfere with mechanisms that prevent the abnormal growth of cancer or tumor cells. Normal cells, on the other hand, express very little survivin. Therefore, survivin is an ideal tumor-specific or tumor-enriched antigen. It is therefore an object of the present invention to deliver an antigenic form of the survivin protein to antigen-presenting cells, so that an immune response to survivin-expressing tumor cells may be stimulated. Human and mammalian survivin proteins and genes are described in detail in U.S. Patent No. 6,245,523 or WO 2004/067023.

### Non-mammalian survivin

As shown in this patent application non-mammalian survivin genes and/or proteins can be used in vaccines for cancer and other diseases. For example, immunization of a mouse or a human with a nucleic acid encoding chicken survivin can generate CD8⁺ T cells that will attack cells expressing mouse or human survivin, respectively. This discovery indicates that chicken survivin protein is immunologically substantially similar to human survivin, suggesting that chicken survivin can be used as an antigenic form of the survivin protein to elicit immune response in a mammal.

Survivin proteins from mammalian and non-mammalian species are aligned in Figure 1. The survivin proteins included in the alignment are human survivin (Genbank Accession Number NM_001168 or 015392, SEQ ID NO:8), dog survivin (Genbank Accession Number NP_001003019, SEQ ID NO:9), pig survivin (Genbank Accession Number NP_999306, SEQ ID NO:39), cow survivin (Genbank Accession Number NP_001001855, SEQ ID NO:40), cat survivin (Genbank Accession Number NP_001009280, SEQ ID NO:41), mouse survivin (Genbank Accession Number NP_033819, SEQ ID NO:10), rat survivin (Genbank Accession Number AAF82586, SEQ ID NO:42), orangutan survivin (Genbank Accession Number CAH91231, SEQ ID NO:43), chicken survivin (Genbank Accession Number NP_001012318, SEQ ID NO:11, "wild-type"), Xenopus laevis SIX survivin (Genbank Accession Number AAO20085, SEQ ID NO:13), Xenopus laevis survivin homolog (Genbank Accession Number AAM76714, SEQ ID NO:46), Xenopus laevis survivin homolog2 (Genbank Accession Number AAH89271, SEQ ID NO:47), catfish survivin homolog (Genbank Accession Number CK419466, SEQ ID NO:52), zebrafish survivin homolog (Genbank Accession Number NP_919378, SEQ ID NO:48), zebrafish survivin homolog 2 (Genbank Accession Number NP_660196, SEQ ID NO:49), pufferfish survivin-like homolog (Genbank Accession Number CAG04432, SEQ ID NO:50), and pufferfish survivin-like homolog 2 (Genbank Accession Number CAG07433, SEQ ID NO:51). All of the sequences disclosed herein by Genbank Accession Numbers are incorporated by reference. The sequences included in Figure 1 are also listed in the sequence listing of the specification.

In addition, two chicken survivin variants are also included in the accompanying sequence listing, variant 1 (Genbank Accession Number NM_001012319,SEQ ID NO:44) which differs beginning at amino acid residue 116 from the wild-type chicken survivin sequence shown in SEQ ID NO:11 to give a different C terminus, and variant 2 (Genbank Accession Number NP_001012317, SEQ ID NO:45) which differs beginning at amino acid residue 38 from the wild-type chicken survivin sequence shown in SEQ ID NO:11.

The following additional sequences are included in the sequence listing: human survivin (Genbank Accession number N_001168.2, SEQ ID NO:64); *Homo sapiens* survivin (Genbank Accession number 015392, SEQ ID NO:65); *Canis familiaris* survivin (Genbank Accession number NM_001003019.1, SEQ ID NO:66); *Sus scrofa* survivin (Genbank accession number NP_999306.1, SEQ ID NO:67); *Bos taurus* survivin (Genbank accession number NP_001001855.2, SEQ ID NO:68); *Felis catus* survivin (Genbank accession number NP_001009280.1, SEQ ID NO:69); *Mus musculus* survivin (Genbank accession number NP_0338I9.1, SEQ ID NO:70); *Rattus norvegicus* survivin (Genbank accession number AAF82586.1, SEQ ID NO:71); *Pongo pygmaeus* survivin (Genbank accession number CAH91231.1, SEQ ID NO:72); *Gallus gallus* survivin (Genbank accession number NP_001012318.1, SEQ ID NO:73); *Xenopus laevis* SIX survivin (Genbank accession number AA020085.1, SEQ ID NO:74); *Xenopus laevis* survivin homolog (Genbank accession number AAM76714.1, SEQ ID NO:75); *Xenopus laevis* survivin homolog 2 (Genbank accession number AAH89271.1, SEQ ID NO:76); nucleic acid encoding *Ictalurus punctatus* survivin (Genbank accession number CK419466.1, SEQ ID NO: 77); *Danio rerio* survivin homolog (Genbank accession number NP_919378.1, SEQ ID NO:78); *Danio rerio* survivin homolog 2 (Genbank accession number NP_660196.1, SEQ ID NO:79); *Tetraodon nigroviridis* survivin-like homolog (Genbank accession number CAG04432.1, SEQ ID NO:80); *Tetraodon nigroviridis* survivin-like homolog 2 (Genbank accession number CAG07433.1, SEQ ID NO:81); *Drosophila melanogaster* survivin (Genbank accession number AAF55399.1, SEQ ID NO:82); and *Caenorhabditis elegans* survivin (Genbank accession number NP_505949.1, SEQ ID NO:83).

The invention contemplates using not only survivin sequences that are found in nature, such as survivin sequences disclosed in FIG. 1, but also other amino acid sequences that have, for example, at least 70% , at least 75%, at least 80%, at least 85%, at least 90%, or at least 95% amino acid identity with at least one of the sequences disclosed in FIG. 1.

As illustrated in Figure 1, whereas the mammalian survivin proteins in the alignment are more than 80 % identical to human survivin, the non-mammalian homologs of survivin in the alignment are less than 60 % identical to human survivin. Within the BIR domain (the underlined region in the alignment of Figure 1), however, non-mammalian survivin proteins are more than 60 % but less than 80 % identical to human survivin, while mammalian survivin proteins are more than 90 % identical to human survivin. The sequence pair percentage identities of BIR domains from survivin homologs are summarized in Figure 2. For example, chicken survivin is approximately 78 % identical to human survivin across the BIR domain.

Importantly, as shown in Figure 1, there is only one 9-mer peptide (18-26: STRAATFRN) (SEQ ID NO:7) in chicken survivin within the BIR domain containing more than 50 % amino acid variation relative to the corresponding human sequence (5 of 9 amino acids are different in this particular 9-mer peptide). Most 9-mer peptides within the BIR domain of chicken survivin contain two or fewer sequence changes (48 of 64 peptides). Without wishing to be bound by theory, it is contemplated that this degree of sequence conservation effectively makes chicken survivin immunologically substantially similar to human survivin.

In general, the percent identity between chicken survivin BIR domain and the mammalian survivin BIR domains shown in Figure 2 varies between approximately 72 % and approximately 78 %. Furthermore, about 75 % of chicken survivin-derived 9-mer peptides from the BIR domain contain two or fewer sequence changes compared to the corresponding mammalian 9-mer peptides. For example, chicken survivin is 75 % percent identical to mouse survivin across the BIR domain, which is consistent with the result that chicken survivin is immunologically substantially similar to mouse survivin, as described in Example 6 below.

The efficacy of a non-mammalian survivin composition, such as a chicken survivin composition, to induce an immune response in a mouse against a tumor expressing mouse survivin, as illustrated in the Examples to follow, underscores a principle of the invention, namely, that vaccination of a mammal with a non-mammalian survivin molecule is useful for inducing an immune response against mammalian survivin or against cells over-expressing mammalian survivin. Thus, chicken survivin is also useful for the generation of an effective immune response in humans against cells that over-express human survivin, such as many tumor cells, since the immunological response is sufficiently conserved between mammals.

In contrast, an alignment of BIR domains from invertebrates to the human survivin BIR domain shows that the *D. melanogaster* (AAF55399, SEQ ID NO:53) and *C. elegans* (NP_505949, SEQ ID NO:54) BIR domains are 50 % or less identical to the human survivin BIR domain sequence (Figure 3), although residues critical for Zn chelation are conserved (see asterisks in the alignment in Figure 3). For example, the fly survivin BIR domain is about 50 % identical to the human survivin BIR domain. Importantly, the fly BIR domain contains only three (3 of 64) 9-mer peptides that have two or fewer amino acid substitutions compared to the corresponding human peptides.

According to the invention, the term "non-mammalian survivin," as used herein, encompasses at least any survivin protein having a BIR domain (or an IAP domain) with at least more than 50% amino acid identity but less than 90% amino acid identity with a human survivin BIR domain, for example at least 55 %, 60%, 65%, 70%, 75%, 80% or 85% identity with a human survivin BIR domain.

To calculate a percent identity, the aligned amino acids of each sequence are compared sequentially. If the amino acids are non-identical, the pair-wise identity score is zero; otherwise the pair-wise identity score is 1.0. The raw identity score is the sum of the identical aligned amino acids. The raw score is then normalized by dividing it by the number of amino acids in the smaller of the candidate or reference sequences and multiplying the result by 100. The normalized raw score is the percent identity. Insertions and deletions are ignored for the purposes of calculating percent identity. Accordingly, gap penalties are not used in this calculation.

Methods to generate multiple sequence alignments are well known to practitioners of the art. To align survivin sequences, the Megalign 6.1 module of the DNASTAR Lasergene^{™} 6 software package was used, applying the ClustalV alignment algorithm, using default settings (Higgins and Sharp (1989) Comput Appl Biosci. 5:151-3). For the subalignment of the survivin BIR domains, the ClustalW ("slow-accurate") method was applied, using default settings (Thompson et al. (1994) Nucleic Acids Res. 22:4673-80).

It is contemplated that non-mammalian survivin may also contain more peptides with the potential of generating a T helper cell response than a mammalian survivin. Without wishing to be bound by theory, it is contemplated that an enhanced immunizing effect can be achieved by engagement of both cytotoxic T cells, through MHC I, and of T helper cells, through MHC II, in the immune response. Sequence differences between the endogenous human survivin and the non-mammalian survivin are likely to yield some peptides being presented to which the human immune system has not been tolerized. The presence of potential MHC II epitopes can be analyzed using publically available predictive algorithms, such as ProPred Analysis (www.imtech.res.in/raghava/propred; Singh et al., (2001) Bioinformatics 17:1236-1237).
Using such an algorithm, it is found that relative to mammalian survivin proteins such as human or dog survivin, non-mammalian survivin proteins, such as chicken survivin or frog SIX survivin are predicted to contain more peptides that bind and/or peptides with higher binding affinity to MHC II molecules (see Table 1).

**Table 1. Peptide binding prediction to human HLA-DR**

| | | | | |
|---|---|---|---|---|
| # | Peptide | #bind | score | |
| Human survivin (SEQ ID NO:8) | | | | |
| 10 | WQPFLKDHR | 2 | 59.1 | (residues 10-18 of SEQ ID NO:8) |
| 13 | FLKDHRIST | 9 | 272.5 | (residues 13-21 of SEQ ID NO:8) |
| 14 | LKDHRISTF | 3 | 68.2 | (residues 14-22 of SEQ ID NO:8) |
| 19 | ISTFKNWPF | 3 | 81.6 | (residues 19-27 of SEQ ID NO:8) |
| 22 | FKNWPFLEG | 4 | 95.9 | (residues 22-30 of SEQ ID NO:8) |
| 28 | LEGCACTPE | 1 | 21.3 | (residues 28-36 of SEQ ID NO:8) |
| 43 | FIHCPTENE | 4 | 114.6 | (residues 43-51 of SEQ ID NO:8) |
| 58 | FFCFKELEG | 3 | 80.6 | (residues 58-66 of SEQ ID NO:8) |
| 74 | IEEHKKHSS | 11 | 320.3 | (residues 74-82 of SEQ ID NO:8) |
| 86 | FLSVKKQFE | 5 | 142.5 | (residues 86-94 of SEQ ID NO:8) |
| 87 | LSVKKQFEE | 3 | 79.9 | (residues 87-95 of SEQ ID NO:8) |
| 96 | LTLGEFLKL | 5 | 148.5 | (residues 96-104 of SEQ ID NO:8) |
| 98 | LGEFLKLDR | 7 | 171.3 | (residues 98-106 of SEQ ID NO:8) |
| 101 | FLKLDRERA | 5 | 106.3 | (residues 101-109 of SEQ ID NO:8) |
| 102 | LKLDRERAK | 1 | 20.0 | (residues 102-110 of SEQ ID NO:8) |
| 113 | IAKETNNKK | 2 | 48.8 | (residues 113-121 of SEQ ID NO:8) |
| | | | | |
| # | Peptide | #bind | score | |
| Dog survivin (SEQ ID NO:9) | | | | |
| 10 | WQPFLKDHR | 2 | 59.1 | (residues 10-18 of SEQ ID NO:9) |
| 13 | FLKDHRIST | 9 | 272.5 | (residues 13-21 of SEQ ID NO:9) |
| 14 | LKDHRISTF | 3 | 68.2 | (residues 14-22 of SEQ ID NO:9) |
| 19 | ISTFKNWPF | 3 | 81.6 | (residues 19-27 of SEQ ID NO:9) |
| 22 | FKNWPFLEG | 4 | 95.9 | (residues 22-30 of SEQ ID NO:9) |
| 43 | FIHCPTENE | 4 | 114.6 | (residues 43-51 of SEQ ID NO:9) |
| 58 | FFCFKELEG | 3 | 80.6 | (residues 58-66 of SEQ ID NO:9) |
| | | | | |
| Dog survivin (SEQ ID NO:9) | | | | |
| 74 | IEEHKKHSS | 11 | 320.3 | (residues 74-82 of SEQ ID NO:9) |
| 86 | FLSVKKQFE | 5 | 142.5 | (residues 86-94 of SEQ ID NO:9) |
| 87 | LSVKKQFEE | 3 | 79.9 | (residues 87-95 of SEQ ID NO:9) |
| 96 | LTLGEFLKL | 5 | 148.5 | (residues 96-104 of SEQ ID NO:9) |
| 98 | LGEFLKLDR | 7 | 171.3 | (residues 98-106 of SEQ ID NO:9) |
| 101 | FLKLDRERA | 5 | 106.3 | (residues 101-109 of SEQ ID NO:9) |
| 102 | LKLDRERAK | 1 | 20.0 | (residues 102-110 of SEQ ID NO:9) |
| 113 | IAKETNNKK | 2 | 48.8 | (residues 113-121 of SEQ ID NO:9) |
| | | | | |
| # | Peptide | #bind | score | |
| Mouse survivin (SEQ ID NO:10) | | | | |
| 6 | LPQIWQLYL | 2 | 70.7 | (residues 6-14 of SEQ ID NO:10) |
| 10 | WQLYLKNYR | 26 | 897.3 | (residues 10-18 of SEQ ID NO:10) |
| 12 | LYLKNYRIA | 10 | 278.3 | (residues 12-20 of SEQ ID NO:10) |
| 13 | YLKNYRIAT | 8 | 202.3 | (residues 13-21 of SEQ ID NO:10) |
| 14 | LKNYRIATF | 19 | 627.3 | (residues 14-22 of SEQ ID NO:10) |
| 17 | YRIATFKNW | 5 | 139.8 | (residues 17-25 of SEQ ID NO:10) |
| 19 | IATFKNWPF | 4 | 111.6 | (residues 19-27 of SEQ ID NO:10) |
| 22 | FKNWPFLED | 2 | 55.9 | (residues 22-30 of SEQ ID NO:10) |
| 43 | FIHCPTENE | 4 | 114.6 | (residues 43-51 of SEQ ID NO:10) |
| 58 | FFCFKELEG | 3 | 80.6 | (residues 58-66 of SEQ ID NO:10) |
| 67 | WEPDDNPIE | 1 | 26.3 | (residues 67-75 of SEQ ID NO:10) |
| 86 | FLTVKKQME | 3 | 91.7 | (residues 86-94 of SEQ ID NO:10) |
| 87 | LTVKKQMEE | 4 | 135.9 | (residues 87-95 of SEQ ID NO:10) |
| 96 | LTVSEFLKL | 4 | 146.9 | (residues 96-104 of SEQ ID NO:10) |
| 101 | FLKLDRQRA | 13 | 305.6 | (residues 101-109 of SEQ ID NO:10) |
| 102 | LKLDRQRAK | 8 | 223.6 | (residues 102-110 of SEQ ID NO:10) |
| 113 | IAKETNNKQ | 3 | 90.8 | (residues 113-121 of SEQ ID NO:10) |
| chicken survivin (SEQ ID NO:11) | | | | |
| 1 | MAAYAEMLP | 2 | 42.9 | (residues 1-9 of SEQ ID NO:11) |
| 7 | MLPKEWLVY | 2 | 51.0 | (residues 7-15 of SEQ ID NO:11) |
| 12 | WLVYLVSTR | 11 | 315.7 | (residues 12-20 of SEQ ID NO:11) |
| 13 | LVYLVSTRA | 34 | 1188.0 | (residues 13-21 of SEQ ID NO:11) |
| 14 | VYLVSTRAA | 28 | 842.9 | (residues 14-22 of SEQ ID NO:11) |
| 15 | YLVSTRAAT | 3 | 70.8 | (residues 15-23 of SEQ ID NO:11) |
| 16 | LVSTRAATF | 8 | 234.7 | (residues 16-24 of SEQ ID NO:11) |
| 24 | FRNWPFTEG | 2 | 46.4 | (residues 24-32 of SEQ ID NO:11) |
| 45 | FVHCPSENS | 23 | 656.0 | (residues 45-53 of SEQ ID NO:11) |
| 56 | WQCFFCLK | 2 | 42.9 | (residues 56-64 of SEQ ID NO:11) |
| 57 | VQCFFCLKE | 14 | 428.5 | (residues 57-65 of SEQ ID NO:11) |
| 60 | FFCLKELEG | 3 | 81.0 | (residues 60-68 of SEQ ID NO:11) |
| | | | | |
| chicken survivin (SEQ ID NO:11) | | | | |
| 61 | FCLKELEGW | 2 | 41.5 | (residues 61-69 of SEQ ID NO:11) |
| 76 | LEEHKKHSA | 7 | 161.8 | (residues 76-84 of SEQ ID NO:11) |
| 91 | LQKDPSNLT | 8 | 226.6 | (residues 91-99of SEQ ID NO:11) |
| 98 | LTVQEFLKL | 2 | 48.3 | (residues 98-106 of SEQ ID NO:11) |
| 100 | VQEFLKLDK | 14 | 377.6 | (residues 100-108 of SEQ ID NO:11) |
| 103 | FLKLDKKRT | 9 | 236.2 | (residues 103-111 of SEQ ID NO:11) |
| 104 | LKLDKKRTK | 19 | 686.1 | (residues 104-112 of SEQ ID NO:11) |
| 114 | VIKKAISQK | 3 | 90.1 | (residues 114-122 of SEQ ID NO:11) |
| 115 | IKKAISQKE | 3 | 78.6 | (residues 115-123 of SEQ ID NO:11) |
| 129 | VAKGVRHAI | 2 | 46.9 | (residues 129-137 of SEQ ID NO:11) |
| # | Peptide | | #bind score | |
| ChickenSurvivin | | | | |
| (N97E,T99M,V100L,Q101G) (SEQ ID NO:12) | | | | |
| 1 | MAAYAEMLP | 2 | 42.9 | (residues 1-9 of SEQ ID NO:12) |
| 7 | MLPKEWLVY | 2 | 51.0 | (residues 7-15 of SEQ ID NO:12) |
| 12 | WLVYLVSTR | 11 | 315.7 | (residues 12-20 of SEQ ID NO:12) |
| 13 | LVYLVSTRA | 34 | 1188.0 | (residues 13-21 of SEQ ID NO:12) |
| 14 | VYLVSTRAA | 28 | 842.9 | (residues 14-22 of SEQ ID NO:12) |
| 15 | YLVSTRAAT | 3 | 70.8 | (residues 15-23 of SEQ ID NO:12) |
| 16 | LVSTRAATF | 8 | 234.7 | (residues 16-24 of SEQ ID NO:12) |
| 24 | FRNWPFTEG | 2 | 46.4 | (residues 24-32 of SEQ ID NO:12) |
| 45 | FVHCPSENS | 23 | 656.0 | (residues 45-53 of SEQ ID NO:12) |
| 56 | WQCFFCLK | 2 | 42.9 | (residues 56-64 of SEQ ID NO:12) |
| 57 | VQCFFCLKE | 14 | 428.5 | (residues 57-65 of SEQ ID NO:12) |
| 60 | FFCLKELEG | 3 | 81.0 | (residues 60-68 of SEQ ID NO:12) |
| 61 | FCLKELEGW | 2 | 41.5 | (residues 61-69 of SEQ ID NO:12) |
| 76 | LEEHKKHSA | 7 | 161.8 | (residues 76-84 of SEQ ID NO:12) |
| 91 | LQKDPSELM | 9 | 312.0 | (residues 91-99 of SEQ ID NO:12) |
| 98 | LMLGEFLKL | 5 | 201.1 | (residues 98-106 of SEQ ID NO:12) |
| 100 | LGEFLKLDK | 3 | 79.5 | (residues 100-108 of SEQ ID NO:12) |
| 103 | FLKLDKKRT | 9 | 236.2 | (residues 103-101 of SEQ ID NO:12) |
| 104 | LKLDKKRTK | 19 | 686.1 | (residues 104-112 of SEQ ID NO:12) |
| 114 | VIKKAISQK | 3 | 90.1 | (residues 114-122 of SEQ ID NO:12) |
| 115 | IKKAISQKE | 3 | 78.6 | (residues 115-123 of SEQ ID NO:12) |
| 129 | VAKGVRHAI | 2 | 46.9 | (residues 129-137 of SEQ ID NO:12) |
| Frog SIX survivin (SEQ ID NO:13) | | | | |
| 1 | MLSISPIVS | 35 | 1104.7 | (residues 1-9 of SEQ ID NO:13) |
| 2 | LSISPIVSL | 3 | 135.9 | (residues 2-10 of SEQ ID NO:13) |
| 4 | ISPIVSLRR | 6 | 143.4 | (residues 4-12 of SEQ ID NO:13) |
| 7 | IVSLRRCDN | 30 | 926.8 | (residues 7-15 of SEQ ID NO:13) |
| 8 | VSLRRCDNE | 1 | 22.1 | (residues 8-16 of SEQ ID NO:13) |
| 10 | LRRCDNEPS | 27 | 867.5 | (residues 10-18 of SEQ ID NO:13) |
| 23 | WRLYNLATR | 28 | 1040.3 | (residues 23-31 of SEQ ID NO:13) |
| 25 | LYNLATRLR | 22 | 716.5 | (residues 25-33 of SEQ ID NO:13) |
| Frog SIX survivin (SEQ ID NO:13) | | | | |
| 26 | YNLATRLRT | 21 | 663.2 | (residues 26-34 of SEQ ID NO:13) |
| 28 | LATRLRTFS | 2 | 59.1 | (residues 28-36 of SEQ ID NO:13) |
| 32 | LRTFSNWPF | 20 | 710.0 | (residues 32-40 of SEQ ID NO:13) |
| 56 | FVHCPTDNS | 21 | 683.3 | (residues 56-64 of SEQ ID NO:13) |
| 67 | WKCFFCLK | 2 | 42.9 | (residues 67-75 of SEQ ID NO:13) |
| 68 | VKCFFCLKE | 12 | 374.2 | (residues 68-76 of SEQ ID NO:13) |
| 71 | FFCLKELEG | 3 | 81.0 | (residues 71-79 of SEQ ID NO:13) |
| 72 | FCLKELEGW | 2 | 41.5 | (residues 72-80 of SEQ ID NO:13) |
| 98 | LFIALKKKA | 30 | 1015.6 | (residues 98-106 of SEQ ID NO:13) |
| 99 | FIALKKKAE | 9 | 325.6 | (residues 99-107 of SEQ ID NO:13) |
| 100 | IALKKKAEE | 13 | 464.8 | (residues 100-108 of SEQ ID NO:13) |
| 109 | LTLSEFLKL | 4 | 165.8 | (residues 109-117 of SEQ ID NO:13) |
| 111 | LSEFLKLDL | 3 | 81.6 | (residues 111-119 of SEQ ID NO:13) |
| 115 | LKLDLEHTK | 6 | 164.7 | (residues 115-123 of SEQ ID NO:13) |
| 124 | IKMQKQMNL | 28 | 729.2 | (residues 124-132 of SEQ ID NO:13) |
| 126 | MQKQMNLHI | 14 | 419.4 | (residues 126-134 of SEQ ID NO:13) |
| 130 | MNLHIERFQ | 12 | 410.6 | (residues 130-138 of SEQ ID NO:13) |
| 134 | IERFQAKAN | 1 | 24.4 | (residues 134-142 of SEQ ID NO:13) |
| 144 | VRGHLEKLD | 4 | 103.7 | (residues 144-152 of SEQ ID NO:13) |

Table 1 shows the start position (#) and sequence (Peptide) of each 9-mer peptide that binds to at least one HLA-DR allele. #bind refers to the number of alleles that the peptides binds to (out of 50), above an arbitrary binding threshold, in this case 20 %. The 20 % threshold refers to 20 % of a theoretical maximum binding score, as calculated by an algorithm as implemented in Propred. Score refers to a cumulative score across all HLA alleles to which that peptide binds. As a reference, the human myelin basic protein (MBP) peptide VVHFFKNIV (SEQ ID NO: 14), derived from a portion of the protein known to give rise to anti-MBP antibodies in humans (MBP(85-99)) binds to 29 HLA alleles, with a cumulative score of 1087.6.

### Modified survivin

In another aspect, the invention provides antigenic forms of the survivin protein by introducing modifications into mammalian survivin proteins. Specifically, the invention contemplates modified mammalian survivin peptides that are biologically inert but immunologically substantially similar to mammalian survivin. This aspect of the invention also addresses two particular problems with a naive approach of simply expressing a survivin protein in the cytoplasm of an antigen-presenting cell. The first problem is that biologically active survivin protein may disrupt the physiology of the antigen-presenting cell. The second problem is that when survivin or essentially any other protein is expressed in a cell, only a small fraction of the protein is degraded in such a way that antigenic peptides are presented through MHC Class I on the cell surface.

Thus, the invention contemplates modified mammalian survivin proteins, including mutant versions of human survivin. The invention also contemplates using not only survivin sequences that are found in nature, such as survivin sequences disclosed in FIG. 1, but also other amino acid sequences that have, for example, at least 70% , at least 75%, at least 80%, at least 85%, at least 90%, or at least 95% amino acid identity with at least one of the sequences disclosed in FIG. 1.

The structure of human survivin has been determined (Chantalat et al., (2000) Mol. Cell 6:183-189; Protein Data Bank (PDB) repository of protein structures Accession number: PDB ID 1E31; Verdecia et al., (2000) Nat. Struct. Bio. 7:602-608; Protein Data Bank (PDB) repository of protein structures Accession number: PDB ID IF3H.
Based on the 3-D structure, human survivin contains at least two domains: an N-terminal globular domain that includes a zinc-binding site, referred to as the BIR or IAP domain, and an extended C-terminal alpha-helix, referred to herein as the helical domain. In a preferred embodiment, the invention provides forms of survivin with a mutation in the N-terminal BIR domain and/or in the C-terminal helical domain. It is an insight of the invention that an optimal effect can be achieved by mutating both domains. Without wishing to be bound by theory, it is thought that the N-terminal BIR domain and the C-terminal helical domain each have a biological activity. For example, the BIR domain has some level of anti-apoptotic activity, even in the absence of the helical domain. In addition, the helical domain has a cytoskeletal-binding activity, and may have a distinct biological activity.

Specifically, in the BIR domain, for example, preferred mutations include, but are not limited to, amino acid substitutions at positions corresponding to Cys57, Cys60, His77, or Cys84 of human survivin. For example, a modified BIR domain can contain at least one of the following substitutions: Cys57Ser, Cys60Ser, His77Phe, and Cys84Ser. Substitutions of any of these amino acids to alanine or proline are also preferred. These mutations are believed to disrupt the zinc binding site in the BIR domain. Other mutations that have the effect of disrupting the zinc binding site may also be used. In addition, mutations at the position corresponding to human survivin Arg18, preferably to aspartic acid or glutamic acid, may be used to generate an inactive BIR domain. Modified BIR domains in which the zinc-binding amino acids are mutated at two, three, or four positions are particularly preferred.

It is generally useful to introduce proline residues anywhere in the helical domain in order to disrupt its function. In particular, proline substitutions can be introduced at positions corresponding to human survivin Ala128 or Ile135. Other amino acid substitutions that can disrupt the helical domain are also preferred.

A preferred modified mammalian survivin may contain a mutation at Arg18, Cys57, Cys60, His77, and/or Cys84 and a proline in the helical domain. Each of the aforementioned amino acids at positions Arg18, Cys57, Cys60, His77, Cys84, Ala128, and Ile135 are conserved between human and chicken survivin, and thus identical mutations may be introduced to generate a biologically inert chicken survivin. Similar mutations can also be introduced into the corresponding positions in other non-mammalian survivin proteins.

Any of the above mutations may be combined with a mutation at position corresponding to Leu98 of human survivin, such as, for example, Leu98Arg, Leu98Lys, and Leu98Ala. In chicken survivin, the position corresponding to human Leu98 is Va1100.

Other useful mutations may be identified by routine experimentation, as described in the Examples. For example, a mutation is introduced into a survivin protein and tested, for example, for lack of biological activity, and/or enhanced degradation, and/or ability to induce an immune response against tumor cells. Exemplary assays for these tests are described in the examples below and are known in the art.

Generally, a preferred modified survivin peptide contains three, four, five or more mutations. However, one utility of the modified survivin contemplated by the invention is to present survivin-derived T cell epitope peptides through MHC Class I molecules. Accordingly, it is generally preferred to mutate fewer than fifty (or fewer than thirty or fewer than twenty) amino acids to maintain immunologically substantial similarity with mammalian survivin.

### Antigen presentation

The invention provides guidelines for optimally introducing mutations into survivin or other protein sequences so that processing of MHC Class I epitopes is relatively unhindered.

It is possible that certain survivin mutations may lie within a peptide segment that is presented by an MHC Class I molecule and recognized by a particular T cell receptor. It is also possible that a mutation may alter the proteolytic processing of survivin into peptide epitopes such that an epitope is cleaved, or that an epitope is not cleaved but a novel epitope is preferentially created.To address the issue of antigen presentation, it is possible to use publicly available databases and information to identify candidate MHC Class I epitopes in a survivin protein, and to then determine if a mutation alters an epitope. For example, the SYFPEITHI algorithm can be used (www.unituebingen.de.uni.kxi; also see, Rammensee et al., (1999) Immunogenetics 50:213-219, the teachings of which are hereby incorporated by reference). Alternatively, the BIMAS algorithm can be used (bimas.dcrt.nih.gov/molbio/hla_bind/; also see, Parker et al., (1994) J. Immunol. 152:163-175, the teachings of which are hereby incorporated by reference).Since the HLA genes encoding the human MHC Class I proteins are highly polymorphic, with different MHC Class I proteins binding to different peptide motifs, a table of potential epitopes is created, having on one axis various MHC Class I molecules and on the other axis a survivin sequence. The entries in the table identify positions of an epitope for a given MHC Class I molecule. The effect of a candidate mutation is judged based on the effect on various epitopes. In the end a mutation or mutations are chosen based on the needs of a vaccine, taking into account, for example, the allelic frequency of different HLA alleles, or the group-specific frequency of HLA alleles. To address the issue of proteolytic processing, according to the invention it is possible to use publicly available databases and information to identify candidate proteasome cleavage sites, and to then determine if a mutation alters such a cleavage site. For example, the publically available database NetChop can be used (www.cbs.dtu.dk/services/NetChop/). It is generally preferable to avoid altering cleavage sites.

For illustrative purposes, Example 12 provides exemplary analysis in determining useful substitutions in survivin protein.

According to the invention, it is also useful to consider the "immunoproteasome." In antigen-presenting cells, the proteasome has a somewhat different composition of proteins and its proteolytic specificity is dominated by an enzyme that cleaves C-terminal to hydrophobic residues. As a result, the proteasome generates peptides that can bind to MHC Class I with a C-terminal hydrophobic anchor residue. If it is desirable to maintain the specificity of the immunoproteasome cleavage pattern, it is preferable to avoid mutation of such hydrophobic residues, particularly leucine, isoleucine, methionine, tyrosine, tryptophan, and phenylalanine. Likewise, it is also preferable to avoid mutating other residues to leucine, isoleucine, methionine, tyrosine, tryptophan, and phenylalanine. Thus, mutations at Arg18, Cys57, Cys60, His77, and Cys84 have the advantage of both destabilizing the structure of survivin and not eliminating immunoproteasome cleavage sites. In addition, it is preferable to avoid introducing mutations in the 7 to 9 amino acids immediately N-terminal to leucine, isoleucine, methionine, tyrosine, tryptophan, and phenylalanine in the protein sequence.

Thus, the invention provides a general method of identifying preferred mutation sites in a protein sequence to generate a protein with enhanced immunogenicity. Based on this method, a preferred mutation site in a protein sequence has the following properties. First, one or more mutations of the site destabilize the protein structure. Second, the destabilizing mutation does not introduce a leucine, isoleucine, methionine, tyrosine, tryptophan, or phenylalanine. Third, the normal amino acid at that position is not leucine, isoleucine, methionine, tyrosine, tryptophan, or phenylalanine. This method can be applied to a wide variety of proteins that can be used as antigens, such as, for example, tumor specific antigens, viral antigens, and other antigens. Among tumor-specific antigens, in addition to survivin, the method may be applied to epithelial cell adhesion molecule, oncogenes such as Ras, carcinoembryonic antigen, and others. Among viral antigens, the method may be applied to p24 of HIV, influenza hemagglutinin, and other viral proteins.

### Fusion proteins comprising non-mammalian and/or modified survivin

In order to promote the degradation of the survivin moiety and its processing into peptides that can be presented through MHC Class I molecules, it is desirable to fuse the non-mammalian survivin or the mutant survivin peptides described above to a suitable second protein. For example, ubiquitin is a particularly preferred fusion partner for this purpose. A ubiquitin-survivin fusion protein can be constructed in one of the following configurations: N terminus-ubiquitin-survivin-C terminus or N terminus-survivin-ubiquitin-C terminus. N terminus-ubiquitin-survivin-C terminus is the preferred orientation. In addition, it is desirable to mutate the survivin N-terminal methionine, preferably to arginine, aspartic acid, or glutamic acid. It is also desirable to introduce a lysine at a position corresponding to human survivin Ile19 or Val21 in a ubiquitin-survivin fusion protein.

It is also desirable to fuse the survivin of the present invention to an Fc moiety. A preferred Fc moiety contains antibody CH2 and CH3 domains, and optionally contains a hinge region. The Fc moiety can be fused to either the N-terminus or C-terminus of survivin. The protein sequence for mature human Fc-ChickenSurvivin is shown in SEQ ID NO:25. The protein sequence for mature murine Fc-ChickenSurvivin is shown in SEQ ID NO:27.

In addition, a fusion protein containing the survivin and Fc moieties may further contain other moieties, such as cytokines that stimulate the immune system as described in PCT Publication WO 01/07081. Without wishing to be bound by theory, the presence of the Fc moiety may enhance uptake of survivin-containing fusion proteins into antigen-presenting cells. It is generally preferable that the Fc moiety is derived from the organism being treated. For example, for treatment of a human, it is preferable to use a human Fc moiety.

The Fc-survivin fusion proteins or nucleic acids encoding the fusion proteins may preferably contain a signal sequence for secretion. Presence of the signal sequence facilitates expression of the fusion proteins in mammalian cell lines, such as NS/0 cells, and can permit secretion of the fusion-protein *in vivo* if a corresponding nucleic acid is administered to a patient. The Fc-survivin fusion protein-encoding sequences preferably start in a 5' to 3' direction with a "leader sequence" derived, for example, from an antibody light (L) chain, fused in frame with at least a portion of an immunoglobulin heavy chain or mutant form thereof, preferably from the Fcγ1 region of the human immunoglobulin γl gene. The Fcγ1 region of the immunoglobulin Fcγ1 gene preferably includes at least a portion of the hinge domain and a CH3 domain, and more preferably includes at least a hinge domain, a CH2 domain and a CH3 domain.

The portion of the DNA encoding the signal sequence preferably encodes a peptide segment which directs the secretion of the Fc fusion protein and thereafter is cleaved away from the remainder of the Fc fusion protein. The signal sequence of the invention is a polynucleotide which encodes an amino acid sequence which initiates transport of a protein across the membrane of the endoplasmic reticulum. Signal sequences which are useful in the invention include antibody light chain signal sequences, e.g., antibody 14.18 (Gillies et al. (1989) J. Immunol. Meth., 125:191), antibody heavy chain signal sequences, e.g., the MOPC141 antibody heavy chain signal sequence (Sakano et al. (1980) Nature, 286:5774), and any other signal sequences which are known in the art (see, for example, Watson (1984) Nucleic Acids Research, 12:5145).

Signal sequences have been well characterized in the art and are known typically to contain 16 to 30 amino acid residues, and may contain greater or fewer amino acid residues. A typical signal peptide consists of three regions: a basic N-terminal region, a central hydrophobic region, and a more polar C-terminal region. The central hydrophobic region contains 4 to 12 hydrophobic residues that anchor the signal peptide across the membrane lipid bilayer during transport of the nascent polypeptide. Following initiation, the signal peptide usually is cleaved within the lumen of the endoplasmic reticulum by cellular enzymes known as signal peptidases. Potential cleavage sites of the signal peptide generally follow the "(-3, -1) rule." Thus a typical signal peptide has small, neutral amino acid residues in positions -1 and -3 and lacks proline residues in this region. The signal peptidase will cleave such a signal peptide between the -1 and +1 amino acids. Thus, the signal sequence may be cleaved from the amino-terminus of the fusion protein during secretion. This results in the secretion of an Fc fusion protein. Signal peptide sequences useful in the practice of the invention are well known in the art. See, for example, von Heijne (1986) Nucleic Acids Res., 14:4683.

The terms "signal sequence," "signal peptide," "leader sequence," or "leader peptides" are used interchangeably herein.

The secreted fusion protein may generally have a correctly folded Fc domain, but with an incorrectly folded survivin moiety. Without wishing to be bound by theory, it is thought that when survivin is forced into the secretory pathway by the Fc moiety of an Fc-survivin fusion protein, survivin folding is compromised, particularly since survivin contains numerous cysteines that could engage in disulfide bonding. Correct folding of the survivin moiety is not necessary for the activity of the vaccines of the invention.

As an alternative to fusion of proteins by genetic engineering techniques, chemical conjugation using conventional chemical cross-linkers may be used to connect protein moieties.

### Nucleic acids

The invention also provides nucleic acids encoding the non-mammalian and/or modified survivin and fusion proteins including the survivin as described above. For example, the nucleotide sequence for human survivin is shown in SEQ ID NO:57, the nucleotide sequence for Xenopus SIX survivin is shown in SEQ ID NO:58, the nucleotide sequence for the Xenopus survivin homolog is shown in SEQ ID NO:59, the nucleotide sequence for zebrafish survivin homolog is shown in SEQ ID NO:60, and the nucleotide sequence for catfish survivin homolog is shown in SEQ ID NO:61. The nucleic acids are preferably linked to and interdigitated with elements that allow expression in eukaryotic cells, particularly, in mammalian cells, to form expression cassettes. Typically, an expression cassette encoding survivin includes at least one of the following regulatory elements such as promoters, enhancers, introns, terminators, a polyadenylation signals and others. Preferably, a mammalian promoter is included. As used herein, by "mammalian promoter" is meant a gene promoter that is derived from a promoter found in a mammal or in a virus that normally grows on a mammal. For example, preferred promoters include those that are present in viruses that grow in mammalian cells, such as in cytomegalovirus (CMV), Simian virus 40 (SV40), Epstein Barr virus (EBV), Rous Sarcoma virus (RSV), Moloney Monkey virus, Human Immunodeficiency virus (HIV), Mouse Mammary Tumor virus (MMTV), or those derived from mammalian genes, such as from mammalian actin, beta-globin, myosin, or optionally derived from mammalian genes preferentially expressed in antigen presenting cells, such as Fascin or other APC-specific genes known in the art. An example of a useful polyadenylation signal is a signal derived from an SV40 polyadenylation signal.

The expression elements may direct expression of the survivin proteins in all eukaryotic cells, or may have cell type-specific enhancers to direct expression, for example, in antigen-presenting cells. The coding portions of the expression cassettes are also preferably designed to have codons that are most commonly used in eukaryotic cells, especially in mammalian or human cells.

The expression cassettes of the invention can be used to produce proteins of the invention in cultured cells. Therefore, the expression cassettes can be linked to appropriate drug resistance genes, origins of DNA replication, and other elements to facilitate selection and replication of the expression cassettes. In one embodiment, the nucleic acid sequences of the expression cassette are incorporated in a plasmid capable of being replicated in a bacterium. Plasmids suitable for the invention may have markers for selection in bacterial cells, such as antibiotic resistance genes. Alternatively, suitable plasmids may lack antibiotic resistance markers but may optionally include a gene encoding a metabolic enzyme, a suppressor tRNA, or another such gene that would be normally found in the genome of a non-drug resistance bacterium. Thus, an exemplary embodiment of the invention is a plasmid containing a nucleic acid encoding a survivin peptide of the invention operatively associated with a mammalian promoter without an antibiotic resistance marker. Such a plasmid may be transformed into a bacterium, and the bacterium may then be used to vaccinate a patient without introducing an antibiotic resistance gene into the patient. Alternatively, such a plasmid may be introduced into a patient without a living carrier. For example, such a plasmid may be introduced as naked DNA or as DNA encapsulated within beads or coated on the surface of beads.

The expression cassettes of the invention can be incorporated into viral genomes, plasmids appropriate for naked DNA administration, or bacterial genomes. In the case of viral genomes, it is desirable to use double-stranded DNA viruses such as adenovirus and adeno-associated virus, and RNA viruses such as retroviruses. The expression cassettes are inserted into the genomes of such viruses by standard techniques. Thus, the invention provides both DNA and RNA forms of nucleic acids.

### Effector molecules

The present invention also provides embodiments that permit the non-mammalian peptide or the modified mammalian survivin peptide to function in concert with an effector molecule that contributes to the immune response. For example, such an effector molecule can be a cytokine moiety including, but not limited to, IL-2, IL-7, IL-12, IL-18, IL-21, IL-23, GM-CSF, or any other cytokine, particularly one capable of activating a Th1 immune response. Such an effector molecule can also be an inhibitor of a cytokine that represses the immune system, for example, a STAT3 inhibitor (e.g., a dominant negative STAT3 protein such as STAT3β). Cytokines or other effector molecules may also contain mutations. For example, a cytokine may contain a Ser substitution at a position corresponding to Cys125 of IL-2.

Thus, in preferred embodiments, the vaccine of the invention includes a nucleic acid encoding a non-mammalian or modified mammalian survivin peptide and a second peptide including an effector molecule described above that contributes to the immune response. The regions encoding the non-mammalian or modified mammalian survivin peptide and the effector molecule peptide can be in a single transcription unit, or in two separate transcription units. Alternatively, the vaccine of the invention includes a nucleic acid encoding a non-mammalian or modified mammalian survivin peptide and a separate nucleic acid encoding a second peptide including an effector molecule described above that contributes to the immune response.

In other embodiments, the vaccine of the invention includes a non-mammalian or modified mammalian survivin peptide and a second peptide including an effector molecule described above that contributes to the immune response. In one embodiment, the non-mammalian or modified mammalian survivin peptide is fused or conjugated to the peptide including an effector molecule. The survivin peptide and effector molecule fusion protein may be further fused or conjugated to an Fc moiety as described above.

### Administration

The vaccines of the invention are used to treat humans or mammals that display or are at risk of diseases associated with survivin overexpression. Such diseases include cancers or other tumors characterized by cells over-expressing survivin.

The vaccines produced according to the present invention can be administered to a mammalian host by any route. The injection of protein antigens commonly is used to elicit immune responses in mammals. However, the vaccines of the invention can also be delivered to APCs by nucleic acid, e.g., DNA, injection. A commonly used technique is to inject DNA expression vectors encoding an antigenic protein into muscle. It is believed that specialized APCs, for example, macrophages and dendritic cells, migrate to the site of injection or administration, pick up and present the antigen through a process known as the antigen presentation process.

Thus, as appropriate, administration can be oral or parenteral, including intravenous and intraperitoneal routes of administration. In addition, administration can be by periodic injections of a bolus of the vaccine or can be made more continuous by intravenous or intraperitoneal administration from a reservoir which is external (e.g., an i.v. bag). In certain embodiments, the vaccines of the instant invention can be pharmaceutical-grade. That is, certain embodiments comply with standards of purity and quality control required for administration to humans. Veterinary applications are also within the intended meaning as used herein.

The formulations, both for veterinary and for human medical use, of the vaccines according to the present invention typically include such vaccines in association with a pharmaceutically acceptable adjuvant, a carrier, and optionally other ingredient(s). The carrier(s) can be "acceptable" in the sense of being compatible with the other ingredients of the formulations and not deleterious to the recipient thereof. Pharmaceutically acceptable carriers, in this regard, are intended to include any and all solvents, dispersion media, coatings, antifungal agents, isotonic and absorption delaying agents, and the like, compatible with pharmaceutical administration. The use of such media and agents for pharmaceutically active substances is known in the art. Except insofar as any conventional medium or agent is incompatible with the active compound or its vector of administration, use thereof in the compositions is contemplated. Supplementary active compounds (identified according to the invention and/or known in the art) also can be incorporated into the compositions. The formulations can conveniently be presented in dosage unit form and can be prepared by any of the methods well known in the art of pharmacy/microbiology. In general, some formulations are prepared by bringing the vaccine into association with a liquid carrier or a finely divided solid carrier or both, and then, if necessary, shaping the product into the desired formulation.

A vaccine composition of the invention is formulated to be compatible with its intended route of administration. Examples of routes of administration include oral or parenteral, e.g., intravenous, intradermal, inhalation, transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide.

Useful solutions for oral or parenteral administration can be prepared by any of the methods well known in the pharmaceutical art, described, for example, in Remington's Pharmaceutical Sciences, (Gennaro, A., ed.), Mack Pub., 1990. Formulations for parenteral administration also can include glycocholate for buccal administration, methoxysalicylate for rectal administration, or citric acid for vaginal administration. The parenteral preparation can be enclosed in ampoules, disposable syringes or multiple dose vials made of glass or plastic. Suppositories for rectal administration also can be prepared by mixing the drug with a non-irritating excipient such as cocoa butter, other glycerides, or other compositions that are solid at room temperature and liquid at body temperatures. Formulations also can include, for example, polyalkylene glycols such as polyethylene glycol, oils of vegetable origin, hydrogenated naphthalenes, and the like. Formulations for direct administration can include glycerol and other compositions of high viscosity. Other potentially useful parenteral carriers for these vaccines include ethylene-vinyl acetate copolymer particles, osmotic pumps, implantable infusion systems, and liposomes. Formulations for inhalation administration can contain as excipients, for example, lactose, or can be aqueous solutions containing, for example, polyoxyethylene-9-lauryl ether, glycocholate and deoxycholate, or oily solutions for administration in the form of nasal drops, or as a gel to be applied intranasally. Retention enemas also can be used for rectal delivery.

Formulations of the present invention suitable for oral administration can be in the form of discrete units such as capsules, gelatin capsules, sachets, tablets, troches, or lozenges, each containing a predetermined amount of the drug; in the form of a powder or granules; in the form of a solution or a suspension in an aqueous liquid or non-aqueous liquid; or in the form of an oil-in-water emulsion or a water-in-oil emulsion. The vaccine can also be administered in the form of a bolus, electuary or paste. A tablet can be made by compressing or molding the drug optionally with one or more accessory ingredients. Compressed tablets can be prepared by compressing, in a suitable machine, the drug in a free-flowing form such as a powder or granules, optionally mixed by a binder, lubricant, inert diluent, surface active or dispersing agent. Molded tablets can be made by molding, in a suitable machine, a mixture of the powdered drug and suitable carrier moistened with an inert liquid diluent.

Oral compositions generally include an inert diluent or an edible carrier. For the purpose of oral vaccine administration, the active compound can be incorporated with excipients. Oral compositions prepared using a fluid carrier for use as a mouthwash include the compound in the fluid carrier and are applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose; a disintegrating agent such as alginic acid, Primogel, or com starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

Vaccine compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor ELTM (BASF, Parsippany, NJ) or phosphate buffered saline (PBS). In all cases, the composition can be sterile and can be fluid to the extent that easy syringability exists. It can be stable under the conditions of manufacture and storage and can be preserved against the action of contaminating microorganisms such as fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as manitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, methods of preparation include vacuum drying and freeze-drying which yields a powder of the active ingredient plus any additional desired ingredient from a previously sterile-filtered solution thereof.

Formulations suitable for intra-articular administration can be in the form of a sterile aqueous preparation of the vaccine which can be in microcrystalline form, for example, in the form of an aqueous microcrystalline suspension. Liposomal formulations or biodegradable polymer systems can also be used to present the vaccine for both intra-articular and ophthalmic administration.

Formulations suitable for topical administration include liquid or semi-liquid preparations such as liniments, lotions, gels, applicants, oil-in-water or water-in-oil emulsions such as creams, ointments or pasts; or solutions or suspensions such as drops. Formulations for topical administration to the skin surface can be prepared by dispersing the vaccine with a dermatologically acceptable carrier such as a lotion, cream, ointment or soap. In some embodiments, useful are carriers capable of forming a film or layer over the skin to localize application and inhibit removal. Where adhesion to a tissue surface is desired the composition can include the vaccine dispersed in a fibrinogen-thrombin composition or other bioadhesive. The vaccine then can be painted, sprayed or otherwise applied to the desired tissue surface. For topical administration to internal tissue surfaces, the agent can be dispersed in a liquid tissue adhesive or other substance known to enhance adsorption to a tissue surface. For example, hydroxypropylcellulose or fibrinogen/thrombin solutions can be used to advantage. Alternatively, tissue-coating solutions, such as pectin-containing formulations can be used.

For inhalation treatments, inhalation of powder (self-propelling or spray formulations) dispensed with a spray can, a nebulizer, or an atomizer can be used. Such formulations can be in the form of a finely comminuted powder for pulmonary administration from a powder inhalation device or self-propelling powder-dispensing formulations. In the case of self-propelling solution and spray formulations, the effect can be achieved either by choice of a valve having the desired spray characteristics (*i.e.*, being capable of producing a spray having the desired particle size) or by incorporating the active ingredient as a suspended powder in controlled particle size. For administration by inhalation, the vaccines also can be delivered in the form of an aerosol spray from a pressured container or dispenser which contains a suitable propellant, e.g., a gas such as carbon dioxide, or a nebulizer. Nasal drops also can be used.

Systemic administration also can be by transmucosal or transdermal means. For transmucosal or transdermal administration, penetrants appropriate to the barrier to be permeated are used in the formulation. Such penetrants generally are known in the art, and include, for example, for transmucosal administration, detergents, bile salts, and filsidic acid derivatives. Transmucosal administration can be accomplished through the use of nasal sprays or suppositories. For transdermal administration, the vaccines typically are formulated into ointments, salves, gels, or creams as generally known in the art.

In one embodiment, the vaccines are prepared with carriers that will protect against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials also can be obtained commercially from Alza Corporation and Nova Pharmaceuticals, Inc. Liposomal suspensions can also be used as pharmaceutically acceptable carriers. These can be prepared according to methods known to those skilled in the art, for example, as described in U.S. Pat. No. 4,522,811. Microsomes and microparticles also can be used.

Oral or parenteral compositions can be formulated in dosage unit form for ease of administration and uniformity of dosage. Dosage unit form refers to physically discrete units suited as unitary dosages for the subject to be treated; each unit containing a predetermined quantity of active compound calculated to produce the desired vaccine effect in association with the required pharmaceutical carrier. The specification for the dosage unit forms of the invention are dictated by and directly dependent on the unique characteristics of the active compound and the particular vaccine effect to be achieved, and the limitations inherent in the art of compounding such an active compound for the treatment of individuals.

Generally, the vaccines identified according to the invention can be formulated for parenteral or oral administration to humans or other mammals, for example, in therapeutically effective amounts, e.g., amounts which provide appropriate concentrations of the drug to target tissue for a time sufficient to induce the desired effect. Additionally, the vaccines of the present invention can be administered alone or in combination with other molecules known to have a beneficial effect on the particular disease or indication of interest. By way of example only, useful cofactors include symptom-alleviating cofactors, including antiseptics, antibiotics, antiviral and antifungal agents and analgesics and anesthetics.

The effective concentration of the vaccines identified according to the invention that is to be delivered in a vaccine composition will vary depending upon a number of factors, including the final desired dosage of the drug to be administered and the route of administration. The preferred dosage to be administered also is likely to depend on such variables as the type and extent of disease or indication to be treated, the overall health status of the particular patient, the relative biological efficacy of the vaccine delivered, the formulation of the vaccine, the presence and types of excipients in the formulation, and the route of administration. In some embodiments, the vaccines of this invention can be provided to an individual using typical dose units deduced from the earlier-described mammalian studies using non-human primates and rodents. As described above, a dosage unit refers to a unitary, *i.e.*, a single dose which is capable of being administered to a patient, and which can be readily handled and packed, remaining as a physically and biologically stable unit dose comprising either the vaccine as such or a mixture of it with solid or liquid pharmaceutical diluents or carriers.

### Delivery configuration of a survivin nucleic acid with a bead

In another embodiment, nucleic acids of the invention may be configured with a delivery vehicle such as a bead. For example, a nucleic acid of the invention may be coated onto cellulose beads according to the method as described in U.S. Application Publication No. 003-0166594, the teachings of which are hereby incorporated by reference. A nucleic acid of the invention may be incorporated within polymeric beads according to the method described in U.S. Patent No. 5,783,567.

In certain embodiments, organisms (e.g., bacteria, mammalian calls, and viral particles) are engineered to produce the survivin of the invention. These organisms can release the survivin for harvesting or can be introduced as vaccines directly to a patient.

### Delivery configuration of a survivin nucleic acid within a virus

Nucleic acids of the invention may be configured with a viral delivery vehicle. The viral delivery vehicle may optionally have an adjuvant effect. For example, suitable viruses include, but are not limited to adenovirus, adeno-associated virus, retrovirus, or vaccinia virus. It is often preferable to use a mutant or crippled form of a virus, such as a replication-incompetent virus. Nucleic acids of the invention are inserted into the viral genomes and are further incorporated into virus particles. The nucleic acids are packaged using helper virus systems that are well known in the art of gene therapy.

### Delivery configuration of a survivin nucleic acid within a bacterium

Nucleic acids of the invention may be configured for delivery within a microbial host, as, for example, described by Gentschev et al. (2000) J. Biotechnol., 83:19-26; or by Stocker BAD (2000) J. Biotechnol., 83:45-50.
Suitable bacteria for nucleic acid (*e.g*., DNA) delivery include, but are not limited to, Salmonella, Shigella, Listeria, or E. coli. It is often preferable to use a mutant, attenuated form of a bacterium, such as an auxotroph. For example, suitable Salmonella strains include, but are not limited to, Salmonella typhimurium aroA auxotroph SL7202 (see, *e.g*., Darji et al., (1997) Cell, 91:765-775), Salmonella typhimurium aroA, dam auxotroph RE88 (see, *e.g.*, Heithoff et al., (1999) Science, 284:967-70), Salmonella typhimurium msb, purI auxotroph VNP20009 (see, *e.g.*, Clairmont et al., (2000) J. Infect. Dis., 181(6):1996-2002; Low, et al., (2004) Methods Mol. Med., 90:47-60), or Salmonella typhimurium prototroph LT2 (ATCC #700720). An exemplary mode of DNA vaccination by Salmonella is illustrated in Figure 4.

### Combination therapy

The vaccine of the invention may be combined with other treatment modalities used in the care of the patient. In one embodiment of the treatment, the vaccine is used in concert with surgical resection of the tumor. In another set of embodiments of the treatment, the vaccine is used in combination with chemotherapy or radiation therapy which reduces tumor size, potentially damages the tumor vasculature, or renders the tumor susceptible to immune responses. Chemotherapy or radiation therapy agents particularly useful for combination therapy with the vaccine of the invention include, but are not limited to, DNA damaging agents such as cyclophosphamide; anti-metabolites such as gemcitibine; and agents disrupting cytoskeletal functions such as taxols. Such agents reduce tumor growth and may damage the tumor vasculature, which, without wishing to be bound by theory, is believed to make the tumor more susceptible to immune responses.

In combination therapy, chemotherapy or irradiation is typically followed by administration of the vaccine in such a way that the formation of an effective anti-tumor immune response is not compromised by potential residual effects of the prior treatment.

In a further embodiment of combination therapy, the vaccine treatment can be combined with immunocytokine treatments. Without wishing to be bound by theory, it is believed that a vaccine generates a more effective immune response, when a cytokine promoting the immune response is present in the tumor microenvironment. For example, particular useful immunocytokines are those that elicit Th1 response, such as IL-2 or IL-12. During a combination therapy, for example, a patient can first receive a vaccine of the invention to generate an immune response towards the tumor, then an immunocytokine that can target the tumor and support the immune response in the tumor. Preferred immunocytokines typically have, for example, an antibody moiety that recognizes a surface antigen characteristic of the tumor, such as EpCAM, or that recognizes a feature of the necrotic core of a tumor, such as DNA. Immunocytokines typically also have a cytokine moiety such as IL-2, IL-12, or others that preferentially direct a Th1 response. Immunocytokines suitable for the invention are described in U.S. Patent No. 5,650,150, the contents of which are hereby incorporated by reference.

### EXAMPLES

Practice of the invention will be more fully understood from the following examples, which are presented herein for illustrative purposes only, and should not be construed as limiting the invention in any way.

### Example 1. Cloning of chicken survivin, generation of chicken survivin variants, construction of plasmids that replicate in Salmonella and express chicken survivin in mammalian cells.

Methods to isolate desired DNA molecules are familiar to persons skilled in the art. For example, where the sequence of the desired DNA molecule is known, reverse transcription and polymerase chain reaction (RT-PCR) are commonly employed, or the DNA molecule may be obtained by chemical synthesis using a commercial supplier such as Blue Heron Biotechnology Inc. (Bothwell, WA). To obtain DNA encoding wild-type chicken survivin, RT-PCR was performed on polyA⁺ mRNA isolated from chicken liver (BD Biosciences cat # 636307), employing the Superscript One-Step RT-PCR for Long Templates Kit (Invitrogen, Carlsbad, CA) and a set of outside primers in a first round of reverse transcription and amplification, and the Supermix High Fidelity Kit (Invitrogen) and a set of inside primers for the second round of amplification. The outside primers were the sense primer 5' -GAAAAATGGCGGCCTATGC- 3' (SEQ ID NO:17) and antisense primer 5' -CACCGTAGACCCAGAGGAACC- 3 ' (SEQ ID NO: 18), and the inside primers were the sense primer 5 ' -CTCTAGAATGGCGGCCTATGCTG- 3' (SEQ ID NO: 19) incorporating an Xba I restriction site (underlined), and antisense primer 5 ' -CCTCGAGACCTAAGGGCCCATGTTCTC- 3' (SEQ ID NO:20), incorporating a Xho I restriction site (underlined), respectively. The amplified PCR product was separated by gel electrophoresis, isolated, cloned into a pCR2.1 vector (Invitrogen), and its sequence verified. The nucleotide and amino acid sequences of wild-type chicken survivin are shown in the sequence listing as SEQ ID NO:21 and SEQ ID NO:11, respectively.

The nucleic acid fragment encoding full-length wild-type chicken survivin was transferred to expression vectors appropriate for its expression in mammalian cells. For example, the Xba I /Xho I digested fragment of the wild-type chicken survivin coding sequence was inserted into the likewise digested plasmid pdCs (see Lo et al., (1998) Protein Engineering 11:495), placing the wild-type chicken survivin gene under the control of a CMV promoter.

As another example, a fragment encoding wild-type chicken survivin was inserted into the expression vector pdCs-huFc (see Lo et al., (1998) Protein Engineering 11:495), placing a full-length wild-type chicken survivin fragment lacking the start methionine downstream of the sequence encoding human Fc, generating a plasmid encoding a huFc-ChickenSurvivin fusion protein. Briefly, a triple ligation was performed with a PflMI / XhoI wild-type chicken survivin fragment (the PflMI site is at nucleotide 26 of wild-type chicken survivin), a SmaI / XhoI pdCs-huFc plasmid fragment (see Lo et al., (1998) Protein Engineering 11:495), and an adapter oligonucleotide duplex molecule that restores the 5' end of the wild-type chicken survivin sequence, omitting the start ATG codon. The duplex adapter molecule was formed by hybridization of complementary oligonucleotides, the sense 5' -GGGTGCAGCGGCCTATGCTGAAATGCTGCCCAAGGA- 3' (SEQ ID NO:22) and the antisense 5' -TTGGGCAGCATTTCAGCATAGGCCGCTGCACCC- 3' (SEQ ID NO:23) oligonucleotides. The generation of the correctly encoded fusion protein was verified by sequencing. The nucleotide sequence of mature human Fcγ1-ChickenSurvivin(ChickenSurvivin minus start Met) is shown in the sequence listing as SEQ ID NO:24.

To obtain an expression vector encoding muFc-ChickenSurvivin, the Sma I/XhoI digested wild-type chicken survivin fragment from pdCs-huFc-ChickenSurvivin was transferred into a likewise-treated pdCs-muFc derived expression vector, resulting in a chimeric sequence between muFc and wild-type chicken survivin, with chicken survivin placed in-frame, directly downstream of the sequence encoding a CH3 moiety of muFc. The muFc moiety encoded by this expression vector was of the IgG2a isotype. The nucleotide sequence of mature muFcγ2-Chicken Survivin(ChickenSurvivin minus start Met) is shown in SEQ ID NO:26. The amino acid sequence for mature mu-Fc chicken survivin is shown in SEQ ID NO:27.

The chicken survivin sequence encoding ChickenSurvivin(N97E, T99M, V100L, Q101G) was generated by a PCR approach incorporating mutagenic primers. Codon substitutions in the mutagenic sense primer (Mut1s) 5' - CCTCTGAACTGATGTTGGGGGAGTTCTTGAAGCTGGAT- 3' (SEQ ID NO:28) and antisense primer (Mut1a) 5 ' - AACTCCCCCAACATCAGTTCAGAGGGATCTTTCTG-3' (SEQ ID NO: 2 9 ) are shown underlined, and an A to G substitution that eliminated an EcoRI site is indicated in bold. Two overlapping PCR fragments were generated off of a wild-type chicken survivin DNA template, the upstream fragment using flanking primer Pr1s (5 ' -CCGCGGCCGCCCCCTTCACCATGGCGGCCTATGCTGAAATG-3') (SEQ ID NO:30) and Mut1a, and the downstream fragment using flanking primer Pr1a (5' - AGATCTGGATCCCTAAGGGCCCATGTTCTCTATC- 3') (SEQ ID NO:31) and Mut1s, respectively, for the amplifications. A PCR reaction was performed on the resulting PCR fragments, combined together with primers Pr1s and Pr1a, generating the full-length product, which was cloned into a pCR2.1 vector (Invitrogen), and its sequence was verified. The fragment encoding ChickenSurvivin(N97E, T99M, V100L, Q101G) was excised with Not I / Bam HI and ligated into a likewise digested pdCs expression vector. The protein sequence for ChickenSurvivin(N97E, T99M, V100L, Q101G) is shown as SEQ ID NO:12 and the amino acid sequence is shown as SEQ ID NO:62.

### Example 2. Synthesis of survivin genes from other non-mammals and construction of mammalian expression vectors.

According to the invention, survivin genes and proteins from other vertebrate non-mammals are used in vaccine compositions. For example, a survivin gene from a fish such as a pufferfish, shark, catfish or zebrafish, or from an amphibian such as the toad Xenopus, or from a reptile such as an alligator, crocodile, turtle, lizard or salamander, or from other birds in addition to chicken, are obtained by methods analogous to those described in Example 1 or by other methods known to those skilled in the art of molecular biology. In some cases, such as the survivin homologues of Xenopus, zebrafish, catfish and pufferfish, (the sequences of which are provided in the sequence listing of this application) survivin homologues have been described and are available through public databases such as PubMed. Where the gene sequence of a survivin homologue is known, the corresponding DNA can be obtained from a commercial DNA synthesis company, such as Blue Heron Biotechnology (Bothell, WA).

The survivin homologues from such fish, amphibians, reptiles, and birds are configured into vaccines compositions by methods analogous to those of Examples 3, 4 and 5. For example, survivin genes from Xenopus, zebrafish, catfish and pufferfish are placed into the plasmid pdCs after the addition of a 5' Xba I site and a 3' Xho I site at the ends of the coding sequence. This is done, for example, using PCR methods analogous to those of Example 1, or by total gene synthesis of the relevant coding sequence flanked by linkers. The pdCs-survivin plasmid is then placed into a Salmonella strain by the methods described in Example 3.

Alternatively, survivin genes are configured into plasmids expressing Fc-survivin fusion proteins as in Example 1, and the Fc-survivin fusion proteins are used as vaccines. For example, survivin genes from Xenopus, zebrafish, catfish or pufferfish are placed into the plasmid pdCs after the addition of a 5' Xma I site and a 3' Xho I site at the ends of the coding sequence. This is done, for example, using PCR methods analogous to those of Example 4, or by total gene synthesis of the relevant coding sequence flanked by linkers. The Fc-survivin plasmid is then placed into a mammalian cell line suitable for high-level expression, such as NS/0 cells, and the resulting secreted protein is collected and purified as in Example 4 and formulated with an adjuvant and used to vaccinate humans or animals as in Example 5.

### Example 3. Construction of Salmonella strains containing plasmids of the invention.

Salmonella strains carrying plasmids of the invention for use in vaccination were generated by a standard electroporation protocol outlined below, familiar to those skilled in the art. For example, the plasmid pdCs-ChickenSurvivin was transformed into a plasmid-free Salmonella strain such as the attenuated Salmonella typhimurium aroA strain SL7207. Other attenuated Salmonella strains also may be used, such as RE88, with the genotype aroA,dam-, VNP20009, with the genotype msb⁻, purI, or alternatively the prototrophic LT2 strain. Salmonella typhii strains, preferably attenuated, may also be used.

To prepare electrocompetent bacteria, a 50 ml log phase culture of Salmonella was harvested at an OD600 of at least 0.5, chilled on ice, and cells were sequentially washed in an equal volume and then in a half volume of ice-cold 1 mM HEPES, and repelleted. Cells were then resuspended in 1 ml cold 10% glycerol, 1 mM HEPES, pelleted and finally resuspended 0.5 ml cold 10% glycerol, 1 mM HEPES by gentle pipetting. Cells were kept on ice until further use, or aliquots were stored at - 80 °C.

For electroporation, 40 microliters of electrocompetent bacteria were added to a pre-chilled 1.5 ml microcentrifuge tube, combined with up to 200 ng of supercoiled plasmid pdCs-ChickenSurvivin in up to a 2 microliter volume, and mixed by tapping. The bacteria-DNA mixture was transferred to a pre-chilled 0.2 cm gap cuvette (Bio-Rad, Hercules, CA) and electroporated with an electrical pulse of 2500 V, 25 µF, 200 ohms, generally resulting in a time constant of 4.5 +/- 0.2, whereupon 1 ml of SOC medium was added. After a 1 hour incubation at 37 °C, 0.1 ml of the cells were plated on LB antibiotic selection plates (100 microgram / ml ampicillin).

Single colony isolates were obtained and cultured, and the plasmid DNA was isolated to confirm its identity by restriction analysis. A freshly grown validated culture was supplemented to 15 % glycerol, flash-frozen in 500 microliter aliquots and stored at - 80°C.

### Example 4. Transfection, expression, preliminary characterization, and purification of Fc-survivin fusion proteins

### A. Transfection and Expression of Fc-survivin Fusion Proteins.

For rapid analysis of protein expression, plasmids expressing, for example, muFc-ChickenSurvivin or muFc-muSurvivin are commonly introduced into a cell line such as human embryonic kidney HEK 293 cells (ATCC# CRL-1573) by transient transfection using lipofectamine (Invitrogen).

To obtain stably transfected clones which express Fc-survivin proteins, for example, the appropriate plasmid DNA was introduced into the mouse myeloma NS/0 cells by electroporation. NS/0 cells were grown in Dulbecco's modified Eagle's medium supplemented with 10% heat-inactivated fetal bovine serum, 2 mM glutamine and penicillin/streptomycin. About 5x106 cells were washed once with PBS and resuspended in 0.5 ml PBS. 10 µg of linearized plasmid DNA were then incubated with the cells in a Gene Pulser Cuvette (0.4 cm electrode gap, BioRad, Hercules, CA) on ice for 10 min. Electroporation was performed using a Gene Pulser (BioRad) with settings at 0.25 V and 500 mF. Cells were allowed to recover for 10 min on ice, after which they were resuspended in growth medium and plated onto two 96 well plates. Stably transfected clones were selected by their growth in the presence of 100 nM methotrexate (MTX), which was added to the growth medium two days post-transfection. The cells were fed every 3 days for two to three more times, and MTX-resistant clones appeared in 2 to 3 weeks. Supernatants from clones were assayed by anti-Fc ELISA to identify high producers. High producing clones were isolated and propagated in growth medium containing 100 nM MTX. Typically, H-SFM or CD medium (Invitrogen) growth medium was used.

Alternatively, clones stably expressing Fc-survivin fusion proteins are obtained in human embryonic kidney HEK 293 cells by methotrexate selection, by a method similar to the one described above. HEK 293 clones are maintained in DMEM supplemented with 10% FBS.

### B. Purification and analysis of Fc-survivin fusion proteins.

A standard purification of Fc-containing fusion proteins was performed based on the affinity of the Fc protein moiety for Protein A. Briefly, cells transfected with a plasmid encoding an Fc-survivin fusion protein were typically grown in roller bottles in H-SFM supplemented with 0.1 µM MTX, and the cell supernatant containing the fusion protein was loaded onto a pre-equilibrated (50 mM sodium phosphate, 150 mM NaCl, 0.01 % Tween 80 at neutral pH) Protein A Sepharose Fast Flow column. The column was washed extensively in this buffer, and bound protein was eluted at a low pH (pH 2.5) in same buffer as above and fractions were brought to pH 6.7 with 1M Tris base, pH 11.

The Protein A Sepharose - purified Fc-Survivin fusion proteins were analyzed by analytical size exclusion chromatography (SEC), and it was found that typically the majority of the material was in an aggregated state. This result was not unexpected since survivin is normally a cytoplasmic protein. Without wishing to be bound by theory, it is thought that when survivin is forced into the secretory pathway by the Fc moiety of an Fc -survivin fusion protein, survivin folding is compromised, particularly since survivin contains numerous cysteines that could engage in disulfide bonding. While this aggregation may interfere with biological activity, aggregation is not undesirable when the protein is to be used as a vaccine.

The integrity and purity of the fusion proteins were verified by reducing SDS-PAGE electrophoresis. The strongest band was seen at approximately 45 kDa, the expected size of the fusion protein. A multitude of secondary bands were present as well: further evidence that the protein had indeed aggregated.

### C. ELISA Procedures.

The concentration of Fc-containing protein products in the supernatants of MTX-resistant clones and other test samples were determined by anti-Fc ELISA. Standard procedures as described in detail below were essentially followed.

### i. Coating plates.

ELISA plates were coated with AffiniPure Goat anti-murine IgG (H+L) (Jackson Immuno Research Laboratories, West Grove, PA) at 5 µg/mL in PBS, and 100 µL/well in 96-well plates (Nunc-Immuno plate Maxisorp). Coated plates were covered and incubated at 4°C overnight. Plates then were washed 4 times with 0.05% Tween (Tween 20) in PBS, and blocked with 1% BSA/1% goat serum in PBS, 200 □1/well. After incubation with the blocking buffer at 37°C for 2 hrs, the plates were washed 4 times with 0.05% Tween in PBS and tapped dry on paper towels.

### ii. Incubation with test samples and secondary antibody

Test samples were diluted as appropriate in sample buffer (1% BSA/1% goat serum/0.05% Tween in PBS). A standard curve was prepared using a chimeric antibody (with a human Fc), the concentration of which was known. To prepare a standard curve, serial dilutions were made in the sample buffer to give a standard curve ranging from 125 ng/mL to 3.9 ng/mL. The diluted samples and standards were added to the plate, 100 µl/well and the plate incubated at 37°C for 2 hr. After incubation, the plate was washed 8 times with 0.05% Tween in PBS. To each well was then added 100 µl of the secondary antibody, the horseradish peroxidase-conjugated anti-human IgG (Jackson Immuno Research), diluted to approximately 1:120,000 in sample buffer. The precise dilution of the secondary antibody to be used varies for each lot. After incubation at 37°C for 2 hr, the plate was washed 8 times with 0.05% Tween in PBS.

### iii. Development

The substrate solution was added to the plate at 100 µL/well. The substrate solution was prepared by dissolving 30 mg of OPD (o-phenylenediamine dihydrochloride (OPD), (1 tablet) into 15 mL of 0.025 M Citric acid/0.05 M Na2HPO4 buffer, pH 5, which contained 0.03% of freshly added hydrogen peroxide. The color was allowed to develop for approximately 30 minutes at room temperature in the dark. The developing time is subject to change, depending on lot to lot variability of the coated plates, the secondary antibody, etc. The reaction was stopped by adding 4N sulfuric acid at 100 µL/well. The plate was read by a plate reader, which was set at both 490 and 650 nm and programmed to subtract the background OD. at 650 nm from the OD at 490 nm.

### Example 5. Methods for vaccine delivery and tumor challenge.

C57B1/6 and Balb/c mice (7-8 weeks old) were purchased from Jackson Laboratories (Bar Harbour, ME). The mice were maintained at EMD Lexigen Research Center Corp., Billerica, MA, and all animal experiments were performed in accordance with approved Animal Facilities Operating Procedures.

To prepare the Salmonella vaccine sample, a 5 ml culture of LB/AMP was inoculated with a single transformed bacterial colony, grown overnight at 37°C, centrifuged at 2000xg for 10 minutes, washed once in PBS and then resuspended in 2 ml PBS. The OD600 of a 1:10 dilution of the culture in PBS was measured, and the concentration of culture was adjusted to 10⁹ cells/ml with PBS, assuming that 1 OD600 unit corresponded to approximately 10⁹ bacteria/ml (as determined from plating a serial dilution of the culture onto LB/Ampicillin plates). Vaccination by oral gavage was performed with a 1 ml syringe fitted with a 20G x 1.5" disposable feeding needle that was threaded into the stomach. Mice were pretreated with a gavage of 0.1 ml of 10% sodium bicarbonate to neutralize the stomach acids, and five minutes later the mice were administered a 0.1 ml vaccine sample (108 bacteria).

The effect of the vaccine was measured on tumor cell lines that express mammalian survivin and present survivin-derived antigens on MHCs. The tumor cell lines used in the examples below were the murine lung carcinoma cell line D121, the murine lymphoma cell line A20, the murine breast tumor line 4T1, the murine Lewis Lung Carcinoma cell line LLC, the murine colon cancer cell line CT26, and the murine melanoma cell line B 16, but other cell lines expressing mammalian survivin and presenting-survivin derived antigens could be used. In some experiments, tumor cell lines were used that had been engineered to express human epithelial cell adhesion molecule (EpCAM), designated by "KSA" (for example LLC/KSA). Survivin expression is conveniently assessed by a Western blot, by standard methods. The rabbit polyclonal antibody to human and murine survivin (AHP604, Serotec, Raleigh, NC) was used at a 1:500 dilution.

Tumor cell lines used in the Examples below were maintained in the following media. D121 was grown in DMEM with 10% fetal bovine serum (FBS), supplemented with 1% pencillin/streptomycin (P/S), 1% L-glutamine, 1% Na pyruvate, and 1 % non-essential amino acids. A20 and 4T1 were grown in RPMI with 10% FBS, 1% P/S, 1% L-glutamine. LLC was grown in DMEM with 10% FBS, 1% P/S, 1% L-glutamine. CT26 was grown in DMEM with 10% FBS, 1 % P/S, 1% L-glutamine, and 1% vitamin B16 medium. CT26/KSA media also included 1% Na pyruvate and 1 % non-essential amino acids. The medium for cell lines additionally expressing EpCAM also contained 1 µg/ml G418.

To assess the vaccine effect on mouse survival or on the lung tumor burden of treated mice, the tumor cells were administered intravenously. The tumor cell lines were washed with PBS, trypsinized for 3 minutes, and the trypsin was neutralized with conditioned medium. The cells were pelleted and washed twice with PBS, or in the case of D121 cells, with PBS, 1% bovine serum albumin, 50 µM EDTA. A single cell suspension was obtained by applying the resuspended cells over multiple disposable 100 µM nylon mesh sieves (BD Falcon). Mice were injected intravenously into the tail vein with the cell suspension in a volume of 200 µl per mouse using a 27 gauge needle. Lungs were removed, weighed and scored by assessing percent area of lung covered by tumor to compare metastatic tumor burden.

### Example 6. Immunization with DNA encoding chicken survivin generates antibodies that cross-react with mammalian survivin.

To test whether vaccination with nucleic acids encoding chicken survivin can generate antibodies that cross-react with mammalian survivin, the following experiments were performed. An immunization protocol described by Davis ((1996) Adv. Drug Delivery Reviews 21:33) was used as a basis for the experiments. 7 - 8 week old female Balb/c mice (n=2 per treatment group) were immunized once (Day 1) or three times (Day 1, 21, and 46) with expression plasmids encoding wild-type chicken survivin (ChSur) or muFc-ChickenSurvivin (FcChSur) or with a control expression plasmid encoding muFc-carcinoembryonic antigen (CEA) (FcCEA). Mice were injected in the tibialis anterior muscle with 100 µl of a 10 µM solution of cardiotoxin, followed five days later by injection with 100 µg of plasmid DNA. On day 62, mice were bled and the serum was assayed for antibodies against murine survivin. Serially diluted serum was applied to an ELISA plate coated with muFc-muSurvivin fusion protein, the plate was washed, the samples were incubated with a peroxidase conjugated anti-mouse IgG2a antibody. The ELISA detected the presence of anti-survivin antibodies by a colorimetric assay compared to negative control naïve mice. The results shown in Table 2 indicate that the survivin DNA vaccines were effective in eliciting antibodies reactive to murine survivin while the control DNA vaccine was not.

**Table 2: Anti murine survivin antibody titers.**

| Serum dilution | 1 treatment (n =2) | | | 3 treatments (n=2) | | |
|---|---|---|---|---|---|---|
| | ChSur | FcChSur | FcCEA | ChSur | FcChSur | FcCEA |
| 50 x | 0.285 | 1.089 | n.a. | 2.176 | 2.495 | 0.046 |
| 200 x | 0.123 | 0.769 | n.a. | 1.177 | 1.276 | 0.042 |
| 800 x | 0.124 | 0.484 | n.a. | 0.417 | 0.454 | 0.044 |

The mouse anti-survivin antibodies are further tested for cross-reactivity towards human survivin in either a Western blot or an ELISA. It is expected that mouse survivin antibodies, obtained by vaccination of mice with a chicken survivin vaccine composition, also bind to human survivin protein.

### Example 7. Vaccination of a mammal with DNA encoding survivin elicits a T cell immune response to cancer cells.

To determine the ability of a Salmonella-based survivin vaccine to stimulate a T-cell response against cancer cells, the following experiments were performed. Balb/c mice were vaccinated with the SL7207 Salmonella strain carrying an AroA mutation (Medina et al., (1999) Infection and Immunity, pp. 1093-1099) and also carrying a mammalian expression vector encoding murine survivin (n= 2) or wild-type chicken survivin (n= 2) on day 0 and day 14, as described in Example 5. Naïve unimmunized mice (n=2) were used as the negative control in this experiment.

One month after the first vaccination, splenocytes from each group were extracted and pooled. CD8⁺ T cells were purified and used as responders in a murine IFNγ ELISpot assay, essentially as described below. In this assay, A20 and 4T1 tumor cell lines were used as the effector cells (Figure 6). The 4T1 cells had been engineered to express human epithelial cell adhesion molecule (EpCAM), but this was not considered to be relevant to the experiment. There were significantly more IFNγ responsive T cells from vaccinated mice than from the control animals. Vaccination with Salmonella containing a murine or chicken survivin expression plasmid correlated with an elevated number of IFNγ secreting precursor T cells in response to the different tumor cell lines.

The same vaccination protocol was also performed on C57B1/6 mice. CD8⁺ T cells from the spleens of these mice were incubated with Lewis Lung Carcinoma cells (LLC) or B16 melanoma cells in the murine IFNγ ELISpot assay. The LLC and B16 cells had been engineered to express human epithelial cell adhesion molecule (EpCAM), but this was not considered to be relevant to the experiment. Both vaccines resulted in an increase in the relative number of responsive T cells directed against peptides from the target antigen expressed in the context of MHC class I as compared to naïve C57B1/6 animals (Figure 5). There were significantly more IFNγ secreting cells produced in mice vaccinated with SL7207 chicken survivin than mice with SL7207 murine survivin against both LLC tumor cells and B16 cells.

Together, these results indicate that both vaccination approaches elicit an increase in the number of CD8⁺ T cell precursors reactive with cancer cells displaying mammalian survivin peptides in the context of MHC class I epitopes. These observations also suggest that immunization with chicken survivin may be more potent in breaking tolerance and generating an immune response directed against mammalian survivin-expressing target cells.

In this Example and in several of the following Examples, the Salmonella typhimurium strain SL7207 was used, but other strains of Salmonella could be used, such as Salmonella typhi. When treating humans with Salmonella typhi in humans, it is generally preferable to use auxotrophs that optionally carry additional attenuating mutations.

### Murine IFNγELISpot assay protocol:

The ELISpot assay was performed essentially as described by Power *et al.,* (Power et al., (1999) J. Immunol. Meth. 227:99-107). CD8⁺ T cells were isolated from splenocytes using a Miltenyi CD8⁺ T cell isolation kit (Miltenyi Biotech, Auburn, CA) and sorted on an Automacs magnetic bead sorter, per the manufacturer's instructions. The CD8⁺ T cells were maintained in RPMI supplemented with 0.7 ng/ml IL-2 (R&D Systems). Within 24 hours, viable cells were purified on a Lympholyte® gradient and resuspended to a final concentration of 10⁶ cells/ml. The CD8⁺ T cells were plated in serial dilutions starting at 10⁵ cells/well in a murine IFNγ ELISpot plate (BD Biosciences, San Jose, CA), precoated with 5 µg/ml of anti-murine IFNγ antibody. All tumor cell lines were added at a concentration of 5 x 10⁴ cells/well. After 18 - 24 hours, the cells were removed, the anti-IFNγ coated membranes were washed with 0.05% Tween in PBS, incubated with a secondary biotinylated antibody to IFNγ in PBS with 2% fetal bovine serum, and bound secondary antibody was visualized by exposure to streptavidin-conjugated HRP and AEC (3-amino-9-ethylcarbazole) acetate solution. The spots corresponding to IFNγ secreting CD8⁺ T cells were quantitated using the Zeiss KS ELISpot system (Muenchen-Hallergmoss, Germany).

### Example 8. In vitro immunization of human-derived immune cells with chicken survivin.

To confirm the ability of a non-mammalian survivin sequence, such as a chicken survivin sequence, to elicit an anti-survivin response in humans, an *in vitro* immunization assay is performed using patient peripheral blood cells (hu PBMCs). In essence, dendritic cells (DCs) and T-cells are purified from hu PBMCs, the DCs are loaded with an antigen of choice and incubated with T-cells, and the response of T-cells to exposure to survivin peptides, for example in the form of target cells expressing human survivin, is assayed, employing standard methods familiar to those skilled in the art.

For example, DCs (from huPBMCs of HLA-A2+ patients and derived using IL-4 and GM-CSF), are pulsed with a control survivin peptide sequence known to be an epitope for HLA-A2+ alleles, such as LMLGEFLKL (SEQ ID NO:6), or are pulsed with experimental peptides, such as (i) a 30-mer chicken survivin-derived peptide GCAFAALQKDPSELMLGEFLKLDRERAKNV (SEQ ID NO:32); (ii) a wild-type chicken survivin peptide; or (iii) a mutated chicken survivin peptide such as ChickenSurvivin(N97E,T99M,V100L,Q101G) (SEQ ID NO:12). Alternatively, DNA constructs expressing these sequences are introduced into DCs. After the incubation with the peptides or protein, or expression of the introduced DNA, DCs are irradiated and washed to remove excess exogenously added peptide, and are then mixed with syngeneic T-cells in the presence of IL-7 and IL-2. The T-cells are re-stimulated on a weekly basis by mixing the cultured T-cells with freshly treated and irradiated DCs.

In one assay, the presence of survivin-reactive T-cells is detected by an IFNγ ELISpot assay, essentially as described in Example 7. Alternatively, a conventional [⁵¹Cr] release assay is used to assess the functional activity of these cells, in which target tumor cells, presenting survivin peptides in the context of MHC class I molecules, are loaded with [⁵¹Cr] and incubated with these T-cells, and tumor cell lysis is quantitated by [⁵¹Cr] release. A control target cell line that does not express survivin is used as a negative control. It is expected that the experimental preparations are at least as effective as the control preparation in generating T-cells that are responsive to survivin, indicating that chicken survivin-derived sequences can serve as epitopes that direct a specific immune response to cells expressing human survivin.

### Example 9. Vaccination effects on lung cancers.

To determine if the immune response elicited by the vaccine could inhibit metastatic tumor growth when the second treatment was delayed until after tumor challenge, the following experiments were performed.

On Day 0, C57B1/6 mice (n=5 per treatment condition) were vaccinated orally with 100 µl of 10% sodium bicarbonate followed by 10⁸ SL7207 salmonella containing: a plasmid encoding a mutant chicken survivin (ChickenSurvivin((N97E, T99M, V100L, Q101G)); a plasmid encoding muFc-ChickenSurvivin; or a plasmid with two transcriptional units, one encoding ChickenSurvivin((N97E, T99M, V100L, Q101G) and one encoding the fusion protein muFc-muIL2 as an adjuvant. Control mice were treated with PBS.

Mice were orally pre-dosed with 100 µl of 10% sodium bicarbonate, and the SL7207 Salmonella were administered in PBS (prepared as described above). Ten days after the priming vaccination, the mice were injected intravenously with a single cell suspension of 1x10⁶ LCC/KSA cells in 200 µl of PBS, and boosted with a second oral vaccination, using the same concentration of Salmonella. The mice were sacrificed on Day 32.

Typical results are shown in the Table below (Table 3). The results indicate that vaccination with Salmonella carrying an expression vector for either chicken survivin construct give similar protection against metastatic lung cancer.

**Table 3. Lung weights and metastasis scores of mice with lung cancer treated with a survivin vaccine.**

| | Mean ± standard deviation (g) | Tumor Score* |
|---|---|---|
| PBS | 1.12 ± 0.25 | 3,3,3,3,3 |
| ChickenSurvivin(N97E,T99M,V100L,Q101G) | 0.23 ± 0.05 | 1,1,1,***1,1*** |
| ChickenSurvivin(N97E,T99M,V100L,Q101G) + muFc-muIL2 | 0.21 ± 0.13 | ***1***,1,2,1,1 |
| muFc-ChickenSurvivin | 0.24 ± 0.03 | 1,1,1,1,1 |
| | | |

In Table 3, (*) Tumor Burden was scored on a scale of 0-3 (0= absence of tumor in lungs, 1= <5% tumor burden, 2= 5-50% tumor burden, 3= >50 %of lungs covered with tumor.) Italicized and bolded tumor scores indicate mice with the finding of an extra-pulmonary tumor at base of tail.

### Example 10. Vaccination with SL7207 chicken survivin or murine survivin significantly delays mortality

To determine whether vaccination with Salmonella SL7207 carrying survivin expression plasmids could reduce tumor burden and increase survival, Balb/c mice (n=5 per treatment condition) were dosed twice by oral gavage as previously described, two weeks apart, with PBS, SL7207 Salmonella with a murine survivin expression plasmid, or SL7207 Salmonella with a wild-type chicken survivin expression plasmid. Two weeks after the second vaccination the mice were injected intravenously with 200 µl of a PBS suspension of 5 x10⁵ cells/ml CT26/KSA syngeneic cells (which also expressed the human EpCAM protein, which was not considered to be relevant to the experiment). The mice were monitored for survival. As illustrated in Figure 7, there was a longer survival profile in the mice treated with SL7207 chicken survivin compared to treatment with PBS or SL7207 murine survivin.

### Example 11. Prolonged survival of mammals with cancer metastases as a result of vaccination with Salmonella carrying survivin expression vectors.

To address whether mammals with pre-existing cancer could be vaccinated with Salmonella carrying survivin expression plasmids, the following experiment was performed. In this experiment, C57B1/6 mice were pretreated with 0.1 ml of 10 % sodium bicarbonate and then orally vaccinated with about 100 million SL7207 salmonella containing a vector for murine survivin (n=5) or ChickenSurvivin(N97E, T99M, V 100L, Q101G) (n=5) in PBS (prepared as described above) on day 0. Control mice were gavaged with sodium bicarbonate and PBS (n=5). Ten days after the priming vaccination, the mice were injected intravenously with 200 µl of a PBS suspension of 1 x10⁶ LLC/KSA cells (which also expressed the human EpCAM protein, which was not considered to be relevant to the experiment). All mice were boosted with a second oral vaccination administered 4 hours after tumor injection using the same concentration of Salmonella administered to prime the immune response.

Survival was followed over the course of a 12 week period after tumor challenge. All of the control mice were deceased within 35 days of tumor administration. Importantly, all of the mice vaccinated with one of the survivin constructs were still alive at day 35. All of the mice in the SL7207 ChickenSurvivin(N97E, T99M, V100L, Q101G) treatment group were deceased within 10 weeks of the LLC/KSA injection. There was one surviving animal in the group treated with SL7027 murine survivin after 3 months. There was no significant difference in survival between the two treatment groups (p= 0.243).

In Figure 8, survival curves for vaccination against a tumor challenge with LLC/KSA cells are shown.

### Example 12. Use of predictive algorithms to assess substitutions in survivin sequences.

Predictive algorithms can be used to evaluate whether survivin variants of the invention compromise potential antigenic epitopes, or conversely, whether subdominant antigenic epitopes potentially may be improved. For example, the huSurvivin variant huSur(R18E, H77A, C84A, A128P) (SEQ ID NO:84) is analyzed using a publically available database for proteasomal cleavage site prediction, for example NetChop (www.cbs.dtu.dk/NetChop), as well as one for epitope prediction for the major MHC I HLA supertypes, for example SYFPEITHI (www.syfpheithi.de). Using NetChop, it is found that the cleavage pattern is not drastically altered by the introduction of the substitutions R18E, H77A, C84A, A128P into human survivin ("S" indicates potential cleavage sites above a threshold of 0.8), as shown:

A previously identified antigenic epitope binding to the HLA-A0201 subtype is shown in bold and underlined, and NetChop does predict a C-terminal cleavage site for this epitope.

Using SYFPEITHI, the huSur(R18E, H77A, C84A, A128P) (SEQ ID NO:84) sequence is analyzed for epitopes that bind HLA supertypes A2, A3, and B7, which together cover about at least 85 % of the human population (see Sette et al., (1999) Immunogen. 50:201-212). When compared to wild-type huSurvivin, the top ranked epitopes are identical for HLA-A 0201 and HLA-A 03, but for HLA-B 0702, the variant survivin sequence yields a top-ranked epitope, due to a more favorable C-terminal anchoring residue achieved by the substitution H77A. Thus, the peptide EPDDDPIEEA (SEQ ID NO: 33) may be a better antigenic peptide than the peptide EPDDDPIEEH (SEQ ID NO: 34).

Further substitutions, as suggested from the NetChop analysis, such as P47L and Q56L, may be introduced into huSur(R18E, H77A, C84A, A128P), and the sequence can be re-analyzed by SYFPEITHI as above. The sequence for HumanSurvivin(R18E, H77A, C84A, A128P) is shown in SEQ ID NO:84. The sequence for HumanSurvivin(R18E, P47L, Q56L, H77A, C84A, A128P, I135P) is shown in SEQ ID NO:56. Specifically, the mutation P47L strengthens the anchor position 2 in the peptide CPTENEPDL (SEQ ID NO: 2), which is changed to CLTENEPDL (SEQ ID NO: 35).

Similarly, the substitution Q56L creates the peptide ALCFFCFKEL (SEQ ID NO: 36) with a preferred anchor residue at position 2 compared to AQCFFCFKEL (SEQ ID NO: 37) , which is predicted to be a rather poor binder to HLA-A 0201. When compared to wild-type huSurvivin, the Q56L variant survivin sequence yields a peptide that is predicted to be as strong an antigen as the validated huSurvivin antigenic peptide ELTLGEFLKL (SEQ ID NO: 38) (Andersen et al., (2001) Cancer Res. 61:869-872).

According to the invention, such an analysis can yield survivin sequences containing highly antigenic peptides to which CTLs are less likely to be tolerized, for instance, by promoting responses to sub-dominant epitopes.

### Example 13. Treatment of a human cancer patient with a vaccine containing a vector for expressing non-mammalian survivin.

According to the invention, a human with cancer is treated with a vaccine of the invention as follows. First, candidates for treatment are optionally categorized with a diagnostic agent that tests whether their cancer cells overexpress survivin. For example, a tumor sample may be analyzed by immunofluorescence with anti-survivin antibodies. Alternatively, a tumor sample from a candidate patient may be analyzed by hybridization to a gene chip that detects survivin mRNA levels, by reverse-transcriptase PCR, or by any other method that detects survivin mRNA or protein levels. Patients may also be categorized as likely to respond based on other diagnostic tests, such as tests for levels of MHC expression, levels of signal transduction molecules known to be involved in T cell-mediated killing, or based on levels of molecules that whose function is not understood but which are empirically known to correlate with responsiveness to immunotherapy.

It is particularly useful to choose human patients that have undergone a surgical resection of much of the tumor. This allows diagnostic analysis of the tumor as described above. In addition, without wishing to be bound by theory, it is advantageous to treat after surgical resection because a large, bulky tumor may simply titrate the immune system. In addition, tumors secrete diffusible immunosuppressive factors that achieve higher concentrations in large masses than in small metastases.

Patients that are chosen for treatment have melanoma, colon carcinoma, breast cancer, prostate cancer, lung cancer, kidney cancer, glioblastoma, head and neck cancer, and other cancers.

After appropriate diagnosis and selection of patients, a patient is treated with a Salmonella strain bearing a non-mammalian survivin expression vector (a vector for expressing non-mammalian survivin in a mammalian cell). For example, a patient may receive about 10⁹ to 10¹³ live Salmonella LT2 carrying a plasmid with chicken survivin expression driven from a mammalian promoter. Preferably, about 10¹⁰_10¹² bacteria are used, and more preferably about 2x10¹¹ Salmonella are administered. The Salmonella strain is preferably auxotrophic, and any of the strains mentioned in the Examples may be used.

Two alternative methods are used to address the issue of survival of the Salmonella bacteria in the acidic environment of the stomach. By the first method, a patient first ingests a safe and effective amount of sodium bicarbonate to neutralize the stomach acid. By the second method, Salmonella bacteria are formulated as a pill with an enteric coating that allows passage through the stomach and dissolution in the small intestine.

Side effects may include mild diarrhea when Salmonella typhimurium bacterial strains are used; Salmonella typhimurium is naturally a mouse pathogen and is not normally pathogenic to humans. It is preferable to treat such mild diarrhea symptomatically, but not to treat with antibiotics, as the latter treatment may destroy the vaccine effect.

### Example 14. Timing of vaccination relative to tumor challenge.

An experiment was performed to test the importance of the timing of vaccination with chicken survivin relative to the time of tumor challenge. Mice were dosed with Salmonella containing a plasmid encoding ChickenSurvivin(N97E, T99M, V100L, Q101G) by oral gavage. The dosing schedule is shown in Figure 9. As shown, oral gavage was performed on days 1 and 13; days 7 and 13; days 10 and 18; or days 13 and 21, relative to an intravenous challenge by LLC/KSA cells on day 10 and an evaluation of tumor lung burden on day 29. As shown in Figure 10, oral gavage with salmonella containing a plasmid encoding ChickenSurvivin(N97E, T99M, V100L, Q101G) was effective to reduce lung tumor burden regardless of the dosing schedule used. The vaccine effect was more pronounced when the priming vaccine dose was administered more than three days prior to tumor challenge.

The effects of the varying dosing schedules on lung metastases was also assessed. Lung metastases were scored as follows: a mouse with greater than 50% coverage of its lung surface with tumor was given a score of 3; a mouse with 5-50% of its lung surface covered with tumor was given a score of 2; a mouse with less than 5% lung surface covered with tumor was given a score of 1; and a mouse with no visible lung tumor colonies was given a score of zero. As shown in Figure 10, dosing schedules that included a first dose prior to tumor challenge tended to reduce the lung metastasis score compared with later dosing schedules or with mice that were not orally immunized with ChickenSurvivin(N97E, T99M, V100L, Q101G).

### Example 15. Combination therapy with survivin vaccine and chemotherapy.

The effects of combining vaccination against survivin and chemotherapy were also evaluated. The testing protocol is shown in Figure 11. As shown in the figure, mice receiving the complete treatment were primed with Salmonella bearing a plasmid encoding wild-type chicken survivin on day 1. LLC/KSA cells were introduced intravenously (challenge) on day 4. Cyclophosphamide (CTX) was administered intraperitoneally on day 11 and indomethacin was administered on days 12-15. On day 15, the mice were given a second oral gavage (boost) with Salmonella carrying the wild-type chicken survivin plasmid and lung tumor burden was evaluated on day 28.

Other mice received only portions of the treatment, receiving for example: only the prime, the challenge, and the boost (PCB); the challenge, the CTX and the indomethacin, but not the prime or the boost (C(CI)); the prime, the challenge, the CTX, and the indomethacin, but not the boost (PC(CI)); or the challenge, the CTX, the indomethacin, and the boost, but not the prime (C(CI)B).

As shown in Figure 12, mice receiving only the prime, challenge and boost showed a reduced tumor burden and reduced lung metastasis score compared to the negative control. CTX and indomethacin had similar effects on the lung metastasis score and greater overall reduction of tumor burden. The lowest tumor burdens and the lowest lung metastasis scores were observed in mice receiving at least the prime and the CTX and indomethacin, demonstrating that the vaccination treatment and the chemotherapy can cooperate to reduce tumor burdens and lung metastases.

### Example 16. Salmonella-based vaccination with DNA encoding non-mammalian survivin correlates with appearance of T cells with an activated-memory phenotype.

Peripheral blood lymphocytes were isolated from mice subjected to the PCB, CI, PC(CI), C(CI)B, or complete (PC(CI)B) treatments described in the previous example. The cells were analyzed by flow cytometry to determine whether the treatments had led to the appearance of activated memory T cells. The presence or absence of memory T cells was determined by monitoring for cells with high expression of CD44 and low expression of CD3 (CD44^{bright} CD3^{low}). As shown in Figure 13, mice treated with the prime-challenge-boost (PCB) protocol demonstrated T cells with a CD44^{bright} expression profile indicative of the appearance of activated memory T cells. Similar profiles were observed in each of the protocols that included immunization with Salmonella carrying a plasmid encoding chicken survivin, but not in the mice treated only with chemotherapy (and not in the negative controls).

### Example 17. Combination therapy with an immunocytokine and a vaccination with DNA encoding non-mammalian survivin.

To determine whether vaccination using a non-mammalian survivin can enhance the efficacy of an immunocytokine-based tumor treatment, mice were challenged on day 1 with LLC/KSA cells subcutaneously and treated according to the schedule shown in Figure 14. Mice receiving oral gavage with salmonella harboring a plasmid encoding ChickenSurvivin(N97E, T99M, V100L, Q101G) were dosed on days 4,11, 18, and 25. The immunocytokine selected for use in this experiment was a fusion of IL-2 with a deimmunized anti-EpCAM antibody, as described in US patent application publication 2003-0157054. Mice receiving the immunocytokine received 20 µg of the immunocytokine intravenously on days 8, 9 and 10.

The resulting tumor volumes over time are shown in Figure 15. Either vaccination or treatments with immunocytokine were sufficient to slow tumor growth over time. Mice receiving both the vaccine and the immunocytokine did even better, demonstrating further reductions in tumor growth rate compared to vaccination alone or to immunocytokine treatment alone.

### Example 18: Evaluation of biological activity of survivin constructs of the invention

Certain of the survivin variants of the invention are designed to be biologically inert. Thus, the proteins themselves do not exhibit anti-apoptotic activity when expressed in cells under conditions that normally induce apoptosis preventable by a wild-type survivin protein. Similarly, the proteins do not interfere with the activity of endogenous wild-type survivin to protect cells from the effect of apoptosis-inducing agents.

The biological inertness of a survivin protein can be tested using BaF3 lymphocyte precursor cells. BaF3 cells are dependent on IL-3 for growth and undergo apoptosis upon growth-factor withdrawal, which can be counteracted by the activity of survivin (see, for example, Ambrosini et al., (1997) Nat. Med. 3:917-921). BaF3 cells are transfected with plasmids encoding a survivin construct of the invention, a wild-type survivin protein or an empty insert, and additionally a marker protein such as GFP, so that cells expressing the exogenously introduced DNA can be monitored. After recovery from transfection, IL-3 is withdrawn from the medium and the BaF3 cells are monitored for apoptosis by, for example, assessing % viability on days 1, 2, 3, and 4, or by an assay indicative of apoptosis familiar to those skilled in the art, such as by nuclear morphology (DNA condensation and fragmentation). It is found that whereas BaF3 cells transfected with wild-type survivin remain largely viable over the 4 day period, BaF3 cells transfected with an empty plasmid or a survivin variant of the invention do not remain viable and largely display a nuclear morphology indicative of apoptosis.

The biological inertness of a survivin construct of the invention can also be tested using HeLa cells. HeLa cells express endogenous survivin at significant levels, and it is known that particular mutations, such as huSurvivin(C84A), act as a dominant negative form of survivin (DN Survivin) and induce apoptosis in these cells (see for example Li et al., (1999) Nat. Cell Biology 1:461-466). HeLa cells are transfected with plasmids encoding a DN Survivin or a survivin construct of the invention, in addition to a marker protein such as GFP, so that cells expressing the exogenously introduced DNA can be monitored. After recovery from transfection, the HeLa cells are grown for 48 hours, fixed, the nuclei are stained with DAPI, and the nuclear morphology of the cells is analyzed by immunofluorescence microscopy. It is found that whereas HeLa cells transfected with DN Survivin typically have hallmarks of undergoing apoptosis, such as condensed and fragmented nuclei, HeLa cells transfected with survivin constructs of the invention grow normally.

### Example 19: Evaluation of the stability of survivin constructs of the invention.

To assess stability of a survivin construct, it can be expressed from a vector that additionally expresses a marker protein such as GFP so that the amount of survivin protein expressed can be measured relative to a standard. Tissue culture cells, such as BHK cells, are transiently transfected and the expression level of the survivin construct is assayed in a Western blot, normalized to marker expression. If available anti-survivin antibodies do not recognize the protein, C-terminally tagged versions of these variants can be used. It is found that compared to wild-type survivin, certain preferred modified survivin constructs of the invention, such as those with multiple destabilizing mutations, are detected at much reduced levels.

In addition to assaying steady state levels, the rate of degradation can be evaluated in a time course experiment. Translation inhibitor cycloheximide is added to the cells at time 0 and cells are incubated for 4 hours. At 0, 2, 5, 10, 20, 60 and 240 minutes, samples are removed for Western blot analysis. To compare rates between modified survivin constructs and wild-type survivin, the amounts are normalized to the 0 time point. It is found that certain preferred modified survivin constructs of the invention are degraded more rapidly than wild-type survivin.

To assess whether degradation is proceeding by the proteasome mediated pathway, the cells are optionally incubated in the presence or absence of a proteasome inhibitor such as lactacystin (2 hrs, 100 µM), and again survivin levels are assayed by a Western blot. In the presence of lactacystin, it is found that survivin variants of the invention and wild-type huSurvivin are detected at similar levels.

## Claims

1. A vaccine that elicits an immune response to human survivin in a human comprising a peptide derived from a modified human survivin or a nucleic acid encoding said peptide, wherein said modified human survivin comprises a mutation within the BIR domain that abolishes substantially the biological activitiy assigned to the BIR domain, **characterized in that** said peptide comprises a modified helical domain and contains the following mutations of human surviving: R18E, P47L, Q56L, H77A, C84A, A128P and 1135P, thus forming the amino acid sequence as specified in SEQ ID NO 56.

2. A vaccine of claim, wherein the peptide is fused to an Fc moiety.

3. A vaccine of claim 1 or 2, wherein the vaccine further encodes or includes a peptide derived from an effector molecule.

4. A vaccine of claim 3, wherein the effector molecule is a cytokine selected from the group consisting of IL-2, IL-7, IL-12, IL-18, IL-21, IL-23 and GM-CSF.

5. A vaccine of any of the claims 1 - 4, wherein the nucleic acid encoding the peptide comprises a mammalian promoter.

6. A vaccine of claim 5, wherein the nucleic acid is a naked DNA.

7. A vaccine of any of the claims 1-6, wherein the nucleic acid is formulated with a reagent that enhances transfection of mammalian cells.

8. A vaccine of any of the claims 1 - 7, wherein the nucleic acid is part of a viral particle.

9. A vaccine of any of the claims 1 - 8 , wherein the vaccine comprises a bacterium comprising the nucleic acid.

10. A vaccine of any of the claims 1 - 9, additionally comprising an adjuvant.

11. A pharmaceutical composition comprising a vaccine as specified in any of the claims 1 - 10 optionally together with an acceptable carrier, diluent or excipient.

12. A vaccine or a pharmaceutical composition as specified in any of the claims 1 - 11 for the use for immunizing a patient against tumor cells overexpressing human survivin.

## Patentansprüche

1. Impfstoff, der eine Immunreaktion gegen Human-Survivin in einem Mensch auslöst, enthaltend ein Peptid, das von einem modifizierten Human-Survivin hergeleitet ist, oder eine Nukleinsäure, die das Peptid codiert, wobei das modifizierte Human-Survivin eine Mutation in der BIR-Domäne enthält, die die biologische Aktivität, die der BIR-Domäne zugeordnet wird, im Wesentlichen aufhebt, **dadurch gekennzeichnet, dass** das Peptid eine modifizierte helikale Domäne enthält, und die folgenden Mutationen von Human-Survivin enthält: R18E, P47L, Q56L, H77A, C84A, A128P und I135P, so dass somit die Aminosäuresequenz nach SEQ ID NO 56 gebildet wird.

2. Impfstoff nach Anspruch 1, wobei das Peptid an einen Fc-Teil fusioniert ist.

3. Impfstoff nach Anspruch 1 oder 2, wobei der Impfstoff zudem ein Peptid, das von einem Effektormolekül hergeleitet ist, codiert oder aufweist.

4. Impfstoff nach Anspruch 3, wobei das Effektormolekül ein Cytokin ist, das aus der aus IL-2, IL-7, IL-12, IL-18, IL-21, IL-23 und GM-CSF bestehenden Gruppe ausgewählt ist.

5. Impfstoff nach einem der Ansprüche 1 bis 4, wobei die Nukleinsäure, die das Peptid codiert, ein Säugetier-Promotor ist.

6. Impfstoff nach Anspruch 5, wobei die Nukleinsäure eine nackte DNA ist.

7. Impfstoff nach einem der Ansprüche 1 bis 6, wobei die Nukleinsäure mit einem Reagenz formuliert wird, das die Transfektion von Säugetierzellen steigert.

8. Impfstoff nach einem der Ansprüche 1 bis 7, wobei die Nukleinsäure Teil eines Viruspartikels ist.

9. Impfstoff nach einem der Ansprüche 1 bis 8, wobei der Impfstoff ein Bakterium enthält, das die Nukleinsäure aufweist.

10. Impfstoff nach einem der Ansprüche 1 bis 9, der zusätzlich einen Hilfsstoff enthält.

11. Pharmazeutische Zusammensetzung, umfassend einen Impfstoff nach einem der Ansprüche 1 bis 10, gegebenenfalls zusammen mit einem unbedenklichen Träger, Verdünnungsmittel oder Exzipienten.

12. Impfstoff oder pharmazeutische Zusammensetzung nach einem der Ansprüche 1 bis 11 zur Verwendung bei der Immunisierung eines Patienten gegen Tumorzellen, die Human-Survivin überexprimieren.

## Revendications

1. Vaccin qui provoque une réponse immune à une survivine humaine chez un être humain comprenant un peptide dérivé d'une survivine humaine modifiée ou d'un acide nucléique codant ledit peptide, dans lequel ladite survivine humaine modifiée comprend une mutation dans le domaine BIR qui abolit substantiellement l'activité biologique assignée au domaine BIR, **caractérisé en ce que** ledit peptide comprend un domaine en hélice modifié et contient les mutations de survivine humaine suivantes : R18E, P47L, Q5 6L, H77A, C84A, A128P et I135P, formant ainsi la séquence d'acides aminés telle que spécifiée dans SEQ ID NO 56.

2. Vaccin selon la revendication 1, dans lequel le peptide est fusionné sur une moitié Fc.

3. Vaccin selon la revendication 1 ou 2, dans lequel le vaccin code ou inclut en outre un peptide dérivé d'une molécule d'effecteur.

4. Vaccin selon la revendication 3, dans lequel la molécule d'effecteur est une cytokine choisie parmi le groupe constitué par IL-2, Il-7, Il-12, IL-18, IL-21, IL-23 et GM-CSF.

5. Vaccin selon l'une quelconque des revendications 1 à 4, dans lequel l'acide nucléique codant le peptide comprend un promoteur de mammifère.

6. Vaccin selon la revendication 5, dans lequel l'acide nucléique est un ADN nu.

7. Vaccin selon l'une quelconque des revendications 1 - 6, dans lequel l'acide nucléique est formulé avec un réactif qui améliore la transfection de cellules de mammifère.

8. Vaccin selon l'une quelconque des revendications 1 - 7, dans lequel l'acide nucléique est une partie d'une particule virale.

9. Vaccin selon l'une quelconque des revendications 1 - 8, dans lequel le vaccin comprend une bactérie comprenant l'acide nucléique.

10. Vaccin selon l'une quelconque des revendications 1 - 9, comprenant additionnellement un adjuvant.

11. Composition pharmaceutique comprenant un vaccin tel que spécifié selon l'une quelconque des revendications 1 -10, en option en association avec un vecteur, un diluant ou un excipient acceptable.

12. Vaccin ou composition pharmaceutique telle que spécifiée selon l'une quelconque des revendications 1 - 11 pour une utilisation pour immuniser un patient contre des cellules tumorales surexprimant une survivine humaine.
